# EUROPÄISCHE PATENTSCHRIFT

(11) **EP 1 888 509 B1**
(45) Veröffentlichungstag und Bekanntmachung des Hinweises auf die Patenterteilung: **15.05.2013**
(21) Anmeldenummer: 06725391.4
(22) Anmeldetag: 29.03.2006
(51) Int. Cl.: C07C 237/22, C07C 271/12, C07C 321/28, C07C 317/48, A01N 37/46, C07D 335/02, C07D 309/06, C07D 233/60, C07D 233/64, C07D 239/60, C07C 233/83

(54) **BENZOYLSUBSTITUIERTE SERIN-AMIDE**
BENZOYL-SUBSTITUTED SERINE AMIDES
SÉRINE-AMIDES SUBSTITUÉS PAR BENZOYLE

(30) Priorität: 25.05.2005 DE 102005024598
(43) Veröffentlichungstag der Anmeldung: 20.02.2008
(73) Patentinhaber: BASF SE, 67056 Ludwigshafen (DE)
(72) Erfinder: WITSCHEL, Matthias, 67098 Bad Dürkheim (DE); ZAGAR, Cyrill, Kowloon, Hong Kong (CN); HUPE, Eike, 67067 Ludwigshafen (DE); KÜHN, Toralf, 68199 Mannheim (DE); MOBERG, William Karl, 67454 Hassloch (DE); PARRA RAPADO, Liliana, 77654 Offenburg (DE); STELZER, Frank, 68309 Mannheim (DE); VESCOVI, Andrea, 68167 Mannheim (DE); REINHARD, Robert, 67117 Limburgerhof (DE); SIEVERNICH, Bernd, 67454 Hassloch (DE); GROSSMANN, Klaus, 67141 Neuhofen (DE); EHRHARDT, Thomas, 67346 Speyer (DE)
(86) Internationale Anmeldenummer: PCT/EP2006/061135
(87) Internationale Veröffentlichungsnummer: WO 2006/125688

(56) Entgegenhaltungen:
- WO-A-03/066576
- WO-A-2005/061443
- WO-A-2006/029828
- US-A- 4 944 796
- T. CAMPBELL BOURLAND ET AL.: "Vanadium-Catalyzed Selenide Oxidation with in situ [2,3] Sigmatropic Rearrangement (SOS Reaction): Scope and Asymmetric Applications" ORGANIC AND BIOMOLECULAR CHEMISTRY., Bd. 2, 2004, Seiten 1315-1329, XP002388277 GBROYAL SOCIETY OF CHEMISTRY, CAMBRIDGE.
- DANIEL J. BURDICK ET AL.: "N-Benzoyl Amino Acids as LFA-1/ICAM Inhibitors 1: Amino Acid Structure-Activity Relationship" BIOORGANIC & MEDICINAL CHEMISTRY LETTERS, Bd. 13, 2003, Seiten 1015-1018, XP002388278 GBOXFORD
- CHARLES F KREWSON ET AL: "Chemistry of some amino acid derivatives of 2,2-dichloropropionic and 2,3,6-trichlorobenzoic acids", WEEDS, WEED SCIENCE SOCIETY OF AMERICA, CHAMPAIGN, IL, US, vol. 8, no. 3, 1 July 1960 (1960-07-01), pages 407-412, XP008135866, ISSN: 0043-1745

## Beschreibung

Die vorliegende Erfindung betrifft benzoylsubstituierte Serin-Amide der Formel I 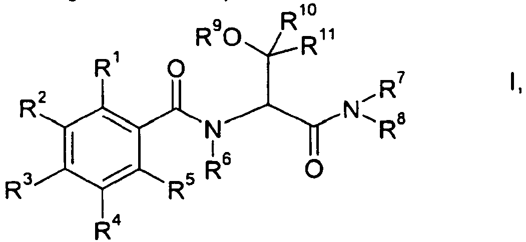 in der die Variablen die folgenden Bedeutungen haben:
- R¹: Halogen, Cyano, C₁-C₆-Alkyl, C₁-C₆-Halogenalkyl oder C₁-C₆-Halogenalkoxy;
- R², R³, R⁴, R⁵: Wasserstoff, Halogen, Cyano, C₁-C₆-Alkyl, C₁-C₆-Halogenalkyl, C₁-C₆-Alkoxy oder C₁-C₆-Halogenalkoxy;
- R⁶, R⁷: Wasserstoff, Hydroxy oder C₁-C₆-Alkoxy;
- R⁸: C₁-C₆-Alkyl, C₁-C₄-Cyanoalkyl oder C₁-C₆-Halogenalkyl;
- R⁹: Wasserstoff, C₁-C₆-Alkyl, C₃-C₆-Cycloalkyl, C₃-C₆-Alkenyl, C₃-C₆-Alkinyl, C₃-C₆-Halogenalkenyl, C₃-C₆-Halogenalkinyl, Formyl, C₁-C₆-Alkylcarbonyl, C₃-C₆-Cycloalkylcarbonyl, C₂-C₆-Alkenylcarbonyl, C₂-C₆-Alkinylcarbonyl, C₁-C₆-Alkoxycarbonyl, C₃-C₆-Alkenyloxycarbonyl, C₃-C₆-Alkinyloxycarbonyl, C₁-C₆-Alkylaminocarbonyl, C₃-C₆-Alkenylaminocarbonyl, C₃-C₆-Alkinylaminocarbonyl, C₁-C₆-Alkylsulfonylaminocarbonyl, Di-(C₁-C₆-alkyl)-aminocarbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-amino-carbonyl, N-(C₃-C₆-Alkenyl)-N-(C₁-C₆-alkoxy)-aminocarbonyl, N-(C₃-C₆-Alkinyl)-N-(C₁-C₆-alkoxy)-aminocarbonyl, Di-(C₁-C₆-alkyl)-aminothiocarbonyl, (C₁-C₆-Alkyl)cyanoimino, (Amino)cyanoimino, [(C₁-C₆-alkyl)amino]cyanoimino, [Di(C₁-C₆-alkyl)amino]cyanoimino, C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl, N-(Di-C₁-C₆-alkylamino)-imino-C₁-C₆-alkyl oder Tri-C₁-C₄-alkylsilyl,
wobei die genannten Alkyl-, Cycloalkyl- und Alkoxyreste partiell oder vollständig halogeniert sein können und/oder eine bis drei der folgenden Gruppen tragen können: Cyano, Hydroxy, C₃-C₆-Cycloalkyl, C₁-C₆-Alkoxy-C₁-C₄-alkyl, C₁-C₄-Alkoxy-C₁-C₄-alkoxy-C₁-C₄-alkyl, C₁-C₄-Alkoxy, C₁-C₄-Alkylthio, Di-(C₁-C₄-alkyl)-amino, C₁-C₄-Alkyl-C₁-C₆-alkoxycarbonylamino, C₁-C₄-Alkylcarbonyl, Hydroxycarbonyl, C₁-C₄-Alkoxycarbonyl, Aminocarbonyl, C₁-C₄-Alkylaminocarbonyl, Di(C₁-C₄-alkyl)aminocarbonyl oder C₁-C₄-Alkylcarbonyloxy;
Phenyl, Phenyl-C₁-C₆-alkyl, Phenylcarbonyl, Phenylcarbonyl-C₁-C₆-alkyl, Phenoxycarbonyl, Phenylaminocarbonyl, Phenylsulfonylaminocarbonyl, N-(C₁-C₆-Alkyl)-N-(phenyl)-aminocarbonyl, Phenyl-C₁-C₆-alkylcarbonyl,
wobei der Phenylrest partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy; oder
SO₂R¹²;
- R¹⁰: Wasserstoff oder C₁-C₆-Alkyl;
- R¹¹: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₁-C₆-Cyanoalkyl, C₂-C₆-Cyanoalkenyl, C₂₋C₆-Cyanoalkinyl, C₁-C₆-Hydroxyalkyl, C₂-C₆-Hydroxyalkenyl, C₂-C₆-Hydroxyalkinyl, C₃-C₆-Cycloalkyl, C₃-C₆-Cycloalkenyl, 3- bis 6-gliedriges Heterocyclyl, 3- bis 6-gliedriges Heterocyclyl-C₁-C₄-alkyl,
wobei die voranstehend genannten Cycloalkyl, Cycloalkenyl oder 3- bis 6-gliedrigen Heterocyclylreste partiell oder vollständig halogeniert sein können und/oder ein bis drei Reste aus der Gruppe Oxo, Cyano, Nitro, C₁-C₆-Alkyl, C₁-C₆-Halogenalkyl, Hydroxy, C₁-C₆-Alkoxy, C₁-C₆-Halogenalkoxy, Hydroxycarbonyl, C₁-C₆-Alkoxycarbonyl, Hydroxycarbonyl-C₁-C₆-alkoxy, C₁-C₆-Alkoxycarbonyl-C₁-C₆-alkoxy, Amino, C₁-C₆-Alkylamino, Di(C₁-C₆-alkyl)amino, C₁-C₆-Alkylsulfonylamino, C₁-C₆-Halogenalkylsulfonylamino, Aminocarbonylamino, (C₁-C₆-Alkylamino)carbonylamino, Di-(C₁-C₆-alkyl)-aminocarbonylamino, Aryl und Aryl(C₁-C₆-alkyl) tragen können;
C₁-C₆-Alkoxy-C₁-C₄-alkyl, C₂-C₆-Alkenyloxy-C₁-C₄-alkyl, C₂-C₆-Alkinyloxy-C₁-C₄-alkyl, C₁-C₆-Halogenalkoxy-C₁-C₄-alkyl, C₂-C₆-Halogenalkenyloxy-C₁-C₄-alkyl, C₂-C₆-Halogenalkinyloxy-C₁-C₄-alkyl, C₁-C₆-Alkoxy-C₁-C₄-alkoxy-C₁-C₄-alkyl, C₁-C₆-Alkylthio-C₁-C₄-alkyl, C₂-C₆-Alkenylthio-C₁-C₄-alkyl, C₂-C₆-Alkinylthio-C₁-C₄-alkyl, C₁-C₆-Halogenalkyl-C₁-C₄-thioalkyl, C₂-C₆-Halogenalkenyl-C₁-C₄-thioalkyl, C₂-C₆-Halogenalkinyl-C₁-C₄-thioalkyl, C₁-C₆-Alkylsulfinyl-C₁-C₄-alkyl, C₁-C₆-Halogenalkylsulfinyl-C₁-C₄-alkyl, C₁-C₆-Alkylsulfonyl-C₁-C₄-alkyl, C₁-C₆-Halogenalkylsulfonyl-C₁-C₄-alkyl, Amino-C₁-C₄-alkyl, C₁-C₆-Alkylamino-C₁-C₄-alkyl, Di(C₁-C₆-Alkyl)amino-C₁-C₄-alkyl, C₁-C₆-Alkylsulfonylamino-C₁-C₄-alkyl, Di(C₁-C₆-Alkylsulfonyl-(C₁-C₆-alkyl)-amino-C₁-C₄-alkyl, C₁-C₆-Alkylcarbonyl, Hydroxycarbonyl, C₁-C₆-Alkoxycarbonyl, Aminocarbonyl, C₁-C₆-Alkylaminocarbonyl, Di(C₁-C₆-alkyl)aminocarbonyl, C₁-C₆-Alkylcarbonyl-C₁-C₆-alkyl, Hydroxycarbonyl-C₁-C₄-alkyl, C₁-C₆-Alkoxycarbonyl-C₁-C₄-alkyl, C₁-C₆-Halogenalkoxycarbonyl-C₁-C₄-alkyl, C₁-C₆-Alkylcarbonyloxy-C₁-C₄-alkyl, Amino-carbonyl-C₁-C₄-alkyl, C₁-C₆-Alkylaminocarbonyl-C₁-C₄-alkyl, Di(C₁-C₆-Alkyl)aminocarbonyl-C₁-C₄-alkyl, Formylamino-C₁-C₄-alkyl, C₁-C₆-Alkoxycarbonylamino-C₁-C₄-alkyl, C₁-C₆-Alkylcarbonylamino-C₁-C₄-alkyl, C₁-C₆-Alkylcarbonyl-(C₁-C₆-alkylamino)-C₁-C₄-alkyl, [(C₁-C₆-Alkyl)aminocarbonyloxy]-C₁-C₄-alkyl, [Di(C₁-C₆-alkyl)aminocarbonyloxy]C₁-C₄-alkyl, {Di[di(C₁-C₆-alkyl)amino]carbonyloxy}C₁-C₄-alkyl, [(C₁-C₆-Alkyl)aminocarbonylamino]-C₁-C₄-alkyl, [Di(C₁-C₆-alkyl)aminocarbonylamino]-C₁-C₄-alkyl; Phenyl-C₁-C₄-alkyl, Phenyl-C₂-C₄-alkenyl, Phenyl-C₂-C₄-alkinyl, Phenyl-C₁-C₄-halogenalkyl, Phenyl-C₂-C₄-halogenalkenyl, Phenyl-C₂-C₄-halogenalkinyl, Phenyl-C₁-C₄-hydroxyalkyl, Phenyl-C₂-C₄-hydroxyalkenyl, Phenyl-C₂-C₄-hydroxyalkinyl, Phenylcarbonyl-C₁-C₄-alkyl, Phenylcarbonylamino-C₁-C₄-alkyl, Phenylcarbonyloxy-C₁-C₄-alkyl, Phenyloxycarbonyl-C₁-C₄-alkyl, Phenyloxy-C₁-C₄-alkyl, Phenylthio-C₁-C₄-alkyl, Phenylsulfinyl-C₁-C₄-alkyl, Phenylsulfonyl-C₁-C₄-alkyl,
Heteroaryl-C₁-C₄-alkyl, Heteroaryl-C₂-C₄-alkenyl, Heteroaryl-C₂-C₄-alkinyl, Heteroaryl-C₁-C₄-halogenalkyl, Heteroaryl-C₂-C₄-halogenalkenyl, Heteroaryl-C₂-C₄-halogenalkinyl, Heteroaryl-C₁-C₄-hydroxyalkyl, Heteroaryl-C₂-C₄-hydroxyalkenyl, Heteroaryl-C₂-C₄-hydroxyalkinyl, Heteroarylcarbonyl-C₁-C₄-alkyl, Heteroarylcarbonyloxy-C₁-C₄-alkyl, Heteroaryloxycarbonyl-C₁-C₄-alkyl, Heteroaryloxy-C₁-C₄-alkyl, Heteroarylthio-C₁-C₄-alkyl, Heteroarylsulfinyl-C₁-C₄-alkyl, Heteroarylsulfonyl-C₁-C₄-alkyl,
wobei die vorstehend genannten Phenyl- und Heteroarylreste partiell oder vollständig halogeniert sein können und/oder ein bis drei Reste aus der Gruppe Cyano, Nitro, C1₋C₆-Alkyl, C₁-C₆-Halogenalkyl, Hydroxy, C₁-C₆-Alkoxy, C₁-C₆-Halogenalkoxy, Hydroxycarbonyl, C₁-C₆-Alkoxycarbonyl, Hydroxycarbonyl-C₁-C₆-alkoxy, C₁-C₆-Alkoxycarbonyl-C₁-C₆-alkoxy, Amino, C₁-C₆-Alkylamino, Di(C₁-C₆-alkyl)amino, C₁-C₆-Alkylsulfonylamino, C₁-C₆-Halogenalkylsulfonylamino, (C₁-C₆-Alkylamino)carbonylamino, Di-(C₁-C₆-alkyl)-aminocarbonylamino, Aryl und Aryl(C₁-C₆-alkyl) tragen können;
- R¹²: C₁-C₆-Alkyl, C₁-C₆-Halogenalkyl oder Phenyl, wobei der Phenylrest partiell oder vollständig halogeniert sein kann und/oder eine bis drei der folgenden Gruppen tragen kann: C₁-C₆-Alkyl, C₁-C₆-Halogen-alkyl oder C₁-C₆-Alkoxy;
sowie deren landwirtschaftlich brauchbaren Salze.

Außerdem betrifft die Erfindung Verfahren und Zwischenprodukte zur Herstellung von Verbindungen der Formel I, Mittel welche diese enthalten sowie die Verwendung dieser Derivate oder diese enthaltende Mittel zur Schadpflanzenbekämpfung.

Fungizid wirksame thienylsubstituierte Aminosäurederivate, die in α-Position einen Alkylrest tragen, welcher gegebenenfalls durch Hydroxy oder Alkoxy substituiert sein kann, werden u.a. in EP 450 355 beschrieben.

WO 03/066576 offenbart als Herbizide Phenylalaninderivate, die in α-Position einen gegebenenfalls substituierten Benzylrest tragen.

Weiterhin sind aus der Literatur, beispielsweise aus US 5,346,907, WO 96/012499 sowie WO 021069905 Serinderivate mit pharmazeutischer Wirksamkeit bekannt, welche in α-Position unter anderem einen Alkylrest tragen können, der gegebenenfalls durch Hydroxy oder Alkoxy substituiert sein kann.

Die herbiziden Eigenschaften der bisher bekannten Verbindungen bzw. die Verträglichkeiten gegenüber Kulturpflanzen können jedoch nur bedingt befriedigen.

Es lag daher dieser Erfindung die Aufgabe zugrunde, neue, insbesondere herbizid wirksame, Verbindungen mit verbesserten Eigenschaften zu finden.

Demgemäß wurden die benzoylsubstituierten Serin-Amide der Formel I sowie deren herbizide Wirkung gefunden.

Ferner wurden herbizide Mittel gefunden, welche die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I enthalten je nach Substitutionsmuster zwei oder mehr Chiralitätszentren und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di-(2-hydroxyeth-1-yl)-ammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄alkyl)-sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die für die Substituenten R¹-R¹² oder als Reste an Phenyl-, Heterocyclyl, Aryl- Heteroaryl- oder Hetrocyclylringen genannten organischen Molekülteile stellen Sammelbegriffe für individuelle Aufzählungen der einzelnen Gruppenmitglieder dar. Sämtliche Kohlenwasserstoffketten, also alle Alkyl-, Alkylsilyl-, Alkenyl-, Alkinyl-, Cyanoalkyl-, Halogenalkyl-, Halogenalkenyl-, Halogenalkinyl-, Alkoxy-, Halogenalkoxy-, Alkoxyalkyl-, Alkoxyalkoxyalkyl, Alkylcarbonyl-, Alkenylcarbonyl-, Alkinylcarbonyl-, Alkoxycarbonyl-, Alkenyloxycarbonyl-, Alkinyloxycarbonyl-, Alkylamino-, Alkylsulfonylamino-, Halogenalkylsulfonylamino-, Alkylalkoxycarbonylamino-, Alkylaminocarbonyl-, Alkenylaminocarbonyl-, Alkinylaminocarbonyl-, Alkylsulfonylaminocarbonyl, Dialkylaminocarbonyl-, N-Alkenyl-N-alkylaminocarbonyl-, N-Alkinyl-N-alkylamino-carbonyl-, N-Alkoxy-N-alkylaminocarbonyl-, N-Alkenyl-N-alkoxyaminocarbonyl-, N-Alkinyl-N-alkoxyaminocarbonyl-, Dialkylaminothiocarbonyl-, Alkylcarbonylalkyl-, Alkoximinoalkyl-, N-(Alkylamino)-iminoalkyl, N-(Dialkylamino)-iminoalkyl, Alkylcyanoimino-, Alkylaminocyanoimino-, Dialkylaminocyanoimino-, Formylaminoalkyl-, Alkoxycarbonylaminoalkyl-, (Alkylamino)carbonyloxyalkyl-, (Alkylamino)carbonylaminoalkyl-, (Dialkylamino)carbonylaminoalkyl-, Phenylcarbonylaminoalkyl, Phenylalkyl-, Phenylcarbonylalkyl-, N-Alkyl-N-phenylaminocarbonyl-, Phenylalkylcarbonyl-, Arylalkyl-, Heterocyclylalkyl, Heterocyclylcarbonylalkyl-, N-Alkyl-N-heterocyclylaminocarbonyl-, Heterocyclylalkylcarbonyl-, Alkylthio- und Alkylcarbonyloxy-Teile können geradkettig oder verzweigt sein.

Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl sowie die Alkylteile von Tri-C₁-C₄-alkylsilyl, C₁-C₄-Alkylcarbonyloxy, C₁-C₄-alkyl-C₁-C₄-alkoxycarbonylamino, C₁-C₆₋Alkyliminooxy-C₁-C₄-alkyl, C₁-C₄-Alkoxy-C₁-C₄-alkoxy-C₁-C₄-alkyl, C₁-C₆-Alkoxy-C₁-C₄-alkyl, C₂-C₆-Alkenyloxy-C₁-C₄-alkyl, C₂-C₆-Alkinyloxy-C₁-C₄-alkyl, C₁-C₆-Halogenalkoxy-C₁-C₄-alkyl, C₂-C₆-Halogenalkenyloxy-C₁-C₄-alkyl, C₂-C₆-Halogenalkinyloxy-C₁-C₄-alkyl, C₁-C₆-Alkoxy-C₁-C₄-alkoxy-C₁-C₄-alkyl, C₁-C₆-Alkylthio-C₁-C₄-alkyl, C₂-C₆-Alkenylthio-C₁-C₄-alkyl, C₂-C₆-Alkinylthio-C₁-C₄-alkyl, C₁-C₆-Alkylsulfinyl-C₁-C₄-alkyl, C₁-C₆-Halogenalkylsulfinyl-C₁-C₄-alkyl, C₁-C₆-Alkylsulfonyl-C₁-C₄-alkyl, C₁-C₆-Halogenalkylsulfonyl-C₁-C₄-alkyl, Amino-C₁-C₄-alkyl, C₁-C₆-Alkylamino-C₁-C₄-alkyl, Di(C₁-C₆-Alkyl)amino-C₁-C₄-alkyl, Formylamino-C₁-C₄-alkyl, C₁-C₆-Alkoxycarbonylamino-C₁-C₄-alkyl, C₁-C₆-Alkylsulfonylamino-C₁-C₄-alkyl, C₁-C₆-Alkylsulfonyl-(C₁-C₆-alkylamino)-C₁-C₄-alkyl, Hydroxycarbonyl-C₁-C₄-alkyl, C₁-C₆-Alkoxycarbonyl-C₁-C₄-alkyl, C₁-C₆-Halogenalkoxycarbonyl-C₁-C₄-alkyl, C₁-C₆-Alkylcarbonyloxy-C₁-C₄-alkyl, Aminocamonyl-C₁-C₄-alkyl, C₁-C₆-Alkylaminocarbonyl-C₁-C₄-alkyl, Di(C₁-C₆-Alkyl)aminocarbonyl-C₁-C₄-alkyl, [(C₁-C₆-alkyl)aminocarbonylamino]-C₁-C₄-alkyl, [Di(C₁-C₆-alkyl)aminocarbonylamino]-C₁-C₄-alkyl, C₁-C₆-Alkylcarbonylamino-C₁-C₄-alkyl, C₁-C₆-Alkylcarbonyl-(C₁-C₆-alkylamino)-C₁-C₄-alkyl, [(C₁-C₆-Alkyl)aminocarbonyloxy]C₁-C₄-alkyl, [Di(C₁-C₆-alkyl)aminocarbonyloxy]C₁-C₄-alkyl, {Di[di(C₁-C₆-alkyl)amino]carbonyloxy}C₁-C₄-alkyl, Heterocyclyl-C₁-C₄-alkyl, Phenyl-C₁-C₄-alkyl, Phenylcarbonylamino-C₁-C₄-alkyl, Phenyl-C₁-C₄-alkyl, Phenylcarbonyl-C₁-C₄-alkyl, Heteroarylcarbonyl-C₁-C₄-alkyl, Heteroarylcarbonyloxy-C₁-C₄-alkyl, Heteroaryloxycarbonyl-C₁-C₄-alkyl, Heteroaryloxy-C₁-C₄-alkyl, Heteroarylthio-C₁-C₄-alkyl, Heteroarylsulfinyl-C₁-C₄-alkyl, Heteroarylsulfonyl-C₁-C₄-alkyl, und Aryl(C₁-C₄-alkyl): z.B. Methyl, Ethyl, n-Propyl, 1-Methylethyl, n-Butyl, 1-Methylpropyl, 2-Methylpropyl und 1,1-Dimethylethyl;
- C₁-C₆-Alkyl sowie die Alkylteile von C₁-C₆-Cyanoalkyl, C₁-C₆-Alkoxycarbonyl-C₁-C₆-alkyl, C₁-C₆-Alkylsulfonylamino, C₁-C₆-Alkylsulfonylaminocarbonyl, N-(C₃-C₆-Alkenyl)-N-(C₁-C₆-alkyl)-aminocarbonyl, (C₃-C₆-Alkinyl)-N-(C₁-C₆-alkyl)-aminocarbonyl, N-(C₁-C₆-Alkoxy)-N-(C₁-C₆-alkyl)-aminocarbonyl, C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl, N-(Di-C₁-C₆-alkyl-amino)-imino-C₁-C₆-alkyl, (C₁-C₆-Alkyl)-cyanoimino, Phenyl-C₁-C₆-alkyl, Phenylcarbonyl-C₁-C₆-alkyl, N-(C₁-C₆-Alkyl)-N-phenylaminocarbonyl, Heterocyclyl-C₁-C₆-alkyl, Hetrocyclyl-carbonyl-C₁-C₆-alkyl und N-(C₁-C₆-Alkyl)-N-heterocyclylaminocarbonyl: C₁-C₄-Alkyl, wie voranstehend genannt, sowie z.B. n-Pentyl, 1-Methyl-butyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethyl-butyl, 2-Ethylbutyl, 1,1,2-Trimethylpropyl, 1-Ethyl-1-methylpropyl und 1-Ethyl-3-methylpropyl;
- C₁-C₄-Alkylcarbonyl: z.B. Methylcarbonyl, Ethylcarbonyl, Propylcarbonyl, 1-Methylethylcarbonyl, Butylcarbonyl, 1-Methylpropylcarbonyl, 2-Methylpropylcarbonyl oder 1,1-Dimethylethylcarbonyl;
- C₁-C₆-Alkylcarbonyl sowie die Alkylcarbonylreste von C₁-C₆-Alkylcarbonyl-C₁-C₆alkyl, C₁-C₄-Alkylcarbonyloxy-C₁-C₆-alkyl, C₁-C₆-Alkylcarbonylamino-C₁-C₄-alkyl, Phenyl-C₁-C₆-alkylcarbonyl und Heterocyclyl-C₁-C₆-alkylcarbonyl, C₁-C₆-Alkylcarbonyl-(C₁-C₆-alkylamino)-C₁-C₄-alkyl: C₁-C₄-Alkylcarbonyl, wie voranstehend genannt, sowie z.B. Pentylcarbonyl, 1-Methylbutylcarbonyl, 2-Methylbutylcarbonyl, 3-Methylbutylcarbonyl, 2,2-Dimethylpropylcarbonyl, 1-Ethylpropylcarbonyl, Hexylcarbonyl, 1,1-Dimethylpropylcarbonyl, 1,2-Dimethylpropylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl oder 1-Ethyl-2-methylpropylcarbonyl;
- C₃-C₆-Cycloalkyl sowie die Cycloalkylteile von C₃-C₆-Cycloalkylcarbonyl: monocyclischer, gesättigter Kohlenwasserstoff mit 3 bis 6 Ringgliedern, wie Cyclopropyl, Cyclobutyl, Cyclopentyl und Cyclohexyl;
- C₃-C₆-Cycloalkenyl: z.B. 1-Cyclopropenyl, 2-Cyclopropenyl, 1-Cyclobutenyl, 2-Cyclobutenyl, 1-Cyclopentenyl, 2-Cyclopentenyl, 1,3-Cyclopentadienyl, 1,4-Cyclopentadienyl, 2,4-Cyclopentadienyl, 1-Cyclohexenyl, 2-Cyclohexenyl, 3-Cyclohexenyl, 1,3-Cyclohexadienyl, 1,4-Cyclohexadienyl, 2,5-Cyclohexadienyl;
- C₃-C₆-Alkenyl sowie die Alkenylteile von C₃-C₆-Alkenyloxycarbonyl, C₃-C₆-Alkenyl-aminocarbonyl, N-(C₃-C₆-Alkenyl)-N-(C₁-C₆-alkyl)aminocarbonyl und N-(C₃-C₆-Alkenyl)-N-(C₁-C₆-alkoxy)aminocarbonyl: z.B. 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
- C₂-C₆-Alkenyl sowie die Alkenylteile von C₂-C₆-Alkenylcarbonyl, C₂-C₆-Alkenyloxy-C₁-C₄-alkyl, C₂-C₆-Alkenylthio-C₁-C₄-alkyl, Phenyl-C₂-C₄-alkenyl, Heteroary-C₂-C₄-alkenyl: C₃-C₆-Alkenyl wie voranstehend genannt sowie Ethenyl;
- C₃-C₆-Alkinyl sowie die Alkinylteile von C₃-C₆-Alkinyloxycarbonyl, C₃-C₆-Alkinylaminocarbonyl, N-(C₃-C₆-Alkinyl)-N-(C₁-C₆-alkyl)-aminocarbonyl, N-(C₃-C₆-Alkinyl)-N-(C₁-C₆-alkoxyaminocarbonyl: z.B. 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
- C₂-C₆-Alkinyl sowie die Alkinylteile von C₂-C₆-Alkinylcarbonyl, C₂-C₂-Alkinyloxy-C₁-C₄-alkyl, C₂-C₆-Alkinylthio-C₁-C₄-alkyl, Phenyl-C₂-C₄-alkinyl, Heteroaryl-C₂-C₄-alkinyl: C₃-C₆-Alkinyl wie voranstehend genannt sowie Ethinyl;
- C₁-C₄-Cyanoalkyl: z.B. Cyanomethyl, 1-Cyanoeth-1-yl, 2-Cyanoeth-1-yl, 1-Cyano-prop-1-yl, 2-Cyanoprop-1-yl, 3-Cyanoprop-1-yl, 1-Cyanoprop-2-yl, 2-Cyanoprop-2-yl, 1-Cyanobut-1-yl, 2-Cyanobut-1-yl, 3-Cyanobut-1-yl, 4-Cyanobut-1-yl, 1-Cyano-but-2-yl, 2-Cyanobut-2-yl, 1-Cyanobut-3-yl, 2-Cyanobut-3-yl, 1-Cyano-2-methyl-prop-3-yl, 2-Cyano-2-methyl-prop-3-yl, 3-Cyano-2-methyl-prop-3-yl und 2-Cyanomethyl-prop-2-yl;
- C₁-C₄-Hydroxyalkyl sowie die C₁-C₄-Hydroxyalkyl-Teile von Phenyl-C₁-C₄-hydroxyalkyl, Heteroaryl-C₁-C₄-hydroxyalkyl: z.B. Hydroxymethyl, 1-Hydroxyeth-1-yl, 2-Hydroxyeth-1-yl, 1-Hydroxyprop-1-yl, 2-Hydroxyprop-1-yl, 3-Hydroxyprop-1-yl, 1-Hydroxyprop-2-yl, 2-Hydroxyprop-2-yl, 1-Hydroxybut-1-yl, 2-Hydroxybut-1-yl, 3-Hydroxybut-1-yl, 4-Hydroxybut-1-yl, 1-Hydroxybut-2-yl, 2-Hydroxybut-2-yl, 1-Hydroxybut-3-yl, 2-Hydroxybut-3-yl, 1-Hydroxy -2-methyl-prop-3-yl, 2-Hydroxy -2-methyl-prop-3-yl, 3-Hydroxy -2-methyl-prop-3-yl und 2-Hydroxymethyl-prop-2-yl, 1,2-Diydroxyethyl, 1,2-Dihydroxyprop-3-yl, 2,3-Dihydroxyprop-3-yl, 1,2-Dihydroxy-prop-2-yl, 1,2-Diydroxybut-4-yl, 2,3-Diydroxybut-4-yl, 3,4-Diydroxybut-4-yl, 1,2-Diydroxybut-2-yl, 1,2-Diydroxybut-3-yl, 2,3-Diydroxybut-3-yl, 1,2-Dihydroxy-2-methyl-prop-3-yl, 2,3-Dihydroxy-2-methyl-prop-3-yl;
- C₁-C₆-Hydroxyalkyl: C₁-C₄-Hydroxyalkyl wie voranstehend genannt, sowie z.B. 1-Hydroxy-pent-5-yl, 2-Hydroxy-pent-5-yl, 3-Hydroxy-pent-5-yl, 4-Hydroxy-pent-5-yl, 5-Hydroxy-pent-5-yl, 1-Hydroxypent-4-yl, 2-Hydroxypen-4-tyl, 3-Hydroxypent-4-yl, 4-Hydroxypent-4-yl, 1-Hydroxy-pent-3-yl, 2-Hydroxy-pent-3-yl, 3-Hydroxy-pent-3-yl, 1-Hydroxy-2-methyl-but-3-yl, 2-Hydroxy-2-methyl-but-3-yl, 3-Hydroxy-2-methyl-but-3-yl, 1-Hydroxy-2-methyl-but-4-yl, 2-Hydroxy-2-methyl-but-4-yl, 3-Hydroxy-2-methyl-but-4-yl, 4-Hydroxy-2-methyl-but-4-yl, 1-Hydroxy-3-methyl-but-4-yl, 2-Hydroxy-3-methyl-but-4-yl, 3-Hydroxy-3-methyl-but-4-yl, 4-Hydroxy-3-methyl-but-4-yl, 1-Hydroxy-hex-6-yl, 2-Hydroxy-hex-6-yl, 3-Hydroxy-hex-6-yl, 4-Hydroxy-hex-6-yl, 5-Hydroxy-hex-6-yl, 6-Hydroxy-hex-6-yl, 1-Hydroxy-2-methyl-pent-5-yl, 2-Hydroxy-2-methyl-pent-5-yl, 3-Hydroxy-2-methyl-pent-5-yl, 4-Hydroxy-2-methyl-pent-5-yl, 5-Hydroxy-2-methyl-pent-5-yl, 1-Hydroxy-3-methyl-pent-5-yl, 2-Hydroxy-3-methyl-pent-5-yl, 3-Hydroxy-3-methyl-pent-5-yl, 4-Hydroxy-3-methyl-pent-5-yl, 5-Hydroxy-3-methyl-pent-5-yl, 1-Hydroxy-4-methyl-pent-5-yl, 2-Hydroxy-4-methyl-pent-5-yl, 3-Hydroxy-4-methyl-pent-5-yl, 4-Hydroxy-4-methyl-pent-5-yl, 5-Hydroxy-4-methyl-pent-5-yl, 1-Hydroxy-5-methyl-pent-5-yl, 2-Hydroxy-5-methyl-pent-5-yl, 3-Hydroxy-5-methyl-pent-5-yl, 4-Hydroxy-5-methyl-pent-5-yl, 5-Hydroxy-5-methyl-pent-5-yl, 1-Hydroxy-2,3-dimethyl-but-4-yl, 2-Hydroxy-2,3-dimethyl-but-4-yl, 3-Hydroxy-2,3-dimethyl-but-4-yl, 4-Hydroxy-2,3-dimethyl-but-4-yl, 1,2-Dihydroxy-pent-5-yl, 2,3-Dihydroxy-pent-5-yl, 3,4-Dihydroxy-pent-5-yl, 4,5-Dihydroxy-pent-5-yl, 1,2-Diydroxypent-4-yl, 2,3-Diydroxypent-4-yl, 3,4-Diydroxypent-4-yl, 4,5-Diydroxypent-4-yl, 1,2-Dihydroxy-pent-3-yl, 2,3-Dihydroxy-pent-3-yl, 1,2-Dihydroxy-2-methyl-but-3-yl, 2,3-Dihydroxy-2-methyl-but-3-yl, 3,4-Dihydroxy-2-methyl-but-3-yl, 2-Hydroxy-2-hdroxymethyl-but-3-yl-, 1,2-Dihydroxy-2-methyl-but-4-yl, 2,3-Dihydroxy-2-methyl-but-4-yl, 3,4-Dihydroxy-2-methyl-but-4-yl, 1,2-Dihydroxy-3-methyl-but-4-yl, 2,3-Dihydroxy-3-methyl-but-4-yl, 3,4-Dihydroxy-3-methyl-but-4-yl, 3-Hydroxy-3-hydroxymethyl-but-4-yl, 1,2-Diydroxy-hex-6-yl, 2,3-Diydroxy-hex-6-yl, 3,4-Diydroxy-hex-6-yl, 4,5-Diydroxy-hex-6-yl, 5,6-Diydroxy-hex-6-yl, 1,2-Dihydroxy-2-methyl-pent-5-yl, 2,3-Dihydroxy-2-methyl-pent-5-yl, 3,4-Dihydroxy-2-methyl-pent-5-yl, 4,5-Dihydroxy-2-methyl-pent-5-yl, 2-Hydroxy-2-hdroxymethyl-pent-5-yl, 1,2-Dihydroxy-3-methyl-pent-5-yl, 2,3-Dihydroxy-3-methyl-pent-5-yl, 3,4-Dihydroxy-3-methyl-pent-5-yl, 4,5-Dihydroxy-3-methyl-pent-5-yl, 3-Hydroxy-3-hdroxymethyl-pent-5-yl, 1,2-Dihydroxy-4-methyl-pent-5-yl, 2,3-Dihydroxy-4-methyl-pent-5-yl, 3,4-Dihydroxy-4-methyl-pent-5-yl, 4,5-Dihydroxy-4-methyl-pent-5-yl, 4-Hydroxy-4-hdroxymethyl-pent-5-yl, 1,2-Dihydroxy-5-methyl-pent-5-yl, 2,3-Dihydroxy-5-methyl-pent-5-yl, 3,4-Dihydroxy-5-methyl-pent-5-yl, 4,5-Dihydroxy-5-methyl-pent-5-yl, 5-Hydroxy-5-hdroxymethyl-pent-5-yl, 1,2-Diydroxy-2,3-dimethyl-but-4-yl, 2,3-Diydroxy-2,3-dimethyl-but-4-yl, 3,4-Dihydroxy-2,3-dimethyl-but-4-yl, 2-Hydroxy-2-hydroxymethyl-3-methyl-but-4-yl, 3-Hydroxy-3-hydroxymethyl-2-methyl-but-4-yl;
- C₁-C₄-Halogenalkyl sowie die Halogenalkylteile von Phenyl-C₁-C₄-halogenalkyl, Heteroaryl-C₁-C₄-halogenalkyl: ein C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, Brommethyl, I-odmethyl, 2-Fluorethyl, 2-Chlorethyl, 2-Bromethyl, 2-lodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl, Nonafluorbutyl, 1,1,2,2-Tetrafluorethyl und 1-Trifluormethyl-1,1,2,2-tetrafluorethyl;
- C₁-C₆-Halogenalkyl sowie die Halogenalkylteile von C₁-C₆-Halogenalkylsulfonyl-amino, C₁-C₆-Halogenalkyl-C₁-C₄-thioalkyl,: C₁-C₄-Halogenalkyl wie voranstehend genannt, sowie z.B. 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl und Tridecafluorhexyl;
- C₃-C₆-Halogenalkenyl: ein C₃-C₆-Alkenylrest, wie voranstehend genannt, der partiell oder vollständig durch Fluor, Chlor, Brom und/oder lod substituiert ist, z.B. 2-Chlor-prop-2-en-1-yl, 3-Chlorprop-2-en-1-yl, 2,3-Dichlorprop-2-en-1-yl, 3,3-Dichlorprop-2-en-1-yl, 2,3,3-Trichlor-2-en-1-yl, 2,3-Dichlorbut-2-en-1-yl, 2-Bromprop-2-en-1-yl, 3-Bromprop-2-en-1-yl, 2,3-Dibromprop-2-en-1-yl, 3,3-Dibromprop-2-en-1-yl, 2,3,3-Tribrom-2-en-1-yl oder 2,3-Dibrombut-2-en-1-yl;
- C₂-C₆-Halogenalkenyl sowie die C₂-C₆-Halogenalkenyl-Teile von C₂-C₆-Halogenalkenyloxy-C₁-C₄-alkyl, C₂-C₆-Halogenalkenyl-C₁-C₄-thioalkyl, Phenyl-C₂-C₄-halogenalkenyl, Heteroaryl-C₂-C₄-halogenalkenyl,: ein C₂-C₆-Alkenylrest, wie voranstehend genannt, der partiell oder vollständig durch Fluor, Chlor, Brom und/oder Iod substituiert ist, z.B. 2-Chlorvinyl, 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromvinyl, 2-Bromallyl, 3-Bromallyl, 2,3-Dibromallyl, 3,3-Dibromallyl, 2,3,3-Tribromallyl oder 2,3-Dibrombut-2-enyl;
- C₂-C₆-Cyanoalkenyl: z.B. 2-Cyanovinyl, 2-Cyanoallyl, 3-Cyanoallyl, 2,3-Dicyanoallyl, 3,3-Dicyanoallyl, 2,3,3-Tricyanoallyl, 2,3-Dicyanobut-2-enyl;
- C₂-C₆-Hydroxyalkenyl sowie die Hydroxy-Teile von Phenyl-C₁-C₄-hydroxyalkenyl, Heteroaryl-C₁-C₄-hydroxyalkenyl: z.B. 2-Hydroxyvinyl, 2-Hydroxyallyl, 3-Hydroxyallyl, 2,3-Dihydroxyallyl, 3,3-Dihydroxyallyl, 2,3,3-Trihydroxyallyl, 2,3-Dihydroxybut-2-enyl;
- C₃-C₆-Halogenalkinyl: ein C₃-C₆-Alkinylrest, wie voranstehend genannt, der partiell oder vollständig durch Fluor, Chlor, Brom und/oder Iod substituiert ist, z.B. 1,1-Difluor-prop-2-in-1-yl, 3-lod-prop-2-in-1-yl, 4-Fluorbut-2-in-1-yl, 4-Chlorbut-2in-1-yl, 1,1-Difluorbut-2-in-1-yl, 4-Iodbut-3-in-1-yl, 5-Fluorpent-3-in-1-yl, 5-lodpent-4-in-1-yl, 6-Fluorhex-4-in-1-yl oder 6-Iodhex-5-in-1-yl;
- C₂-C₆-Halogenalkinyl sowie die C₂-C₆-Halogenalkinyl-Teile von C₂-C₆-Halogenalkinyloxy-C₁-C₄-alkyl, C₂-C₆-Halogenalkinyl-C₁-C₄-thioalkyl, Phenyl-C₂-C₄-halogenalkinyl, Heteroaryl-C₂-C₄-halogenalkinyl: ein C₂-C₆-Alkinylrest, wie voranstehend genannt, der partiell oder vollständig durch Fluor, Chlor, Brom und/oder Iod substituiert ist, z.B. 1,1-Difluor-prop-2-in-1-yl, 3-lod-prop-2-in-1-yl, 4-Fluorbut-2-in-1-yl, 4-Chlorbut-2-in-1-yl, 1,1-Difluorbut-2-in-1-yl, 4-lodbut-3-in-1-yl, 5-Fluorpent-3-in-1-yl, 5-lodpent-4-in-1-yl, 6-Fluorhex-4-in-1-yl oder 6-lodhex-5-in-1-yl;
- C₂-C₆-Cyanoalkinyl: z.B. 1,1-Dicyano-prop-2-in-1-yl, 3-Cyano -prop-2-in-1-yl, 4-Cyano-but-2-in-1-yl, 1,1-Dicyanobut-2-in-1-yl, 4-Cyanobut-3-in-1-yl, 5-Cyanopent-3-in-1-yl, 5-Cyanopent-4-in-1-yl, 6-Cyanohex-4-in-1-yl oder 6-Cyanohex-5-in-1-yl;
- C₂-C₆-Hydroxyalkinyl sowie die Hydroxy-Teile von Phenyl-C₂-C₄-hydroxyalkinyl: z.B. 1,1-Dihydroxy-prop-2-in-1-yl, 3-Hydroxy-prop-2-in-1-yl, 4-Hydroxy-but-2-in-1-yl, 1,1-Dihydroxybut-2-in-1-yl, 4-Hydroxybut-3-in-1-yl, 5-Hydroxypent-3-in-1-yl, 5-Hydroxypent-4-in-1-yl, 6-Hydroxyhex-4-in-1-yl oder 6-Hydroxyhex-5-in-1-yl;
- C₁-C₆-Alkylsulfinyl (C₁-C₆-Alkyl-S(=O)-) sowie die C₁-C₆-Alkylsulfinyl-Teile von C₁-C₆-Alkylsulfinyl-C₁-C₄-alkyl: z.B. Methylsulfinyl, Ethylsulfinyl, Propylsulfinyl, 1-Methylethylsulfinyl, Butylsulfinyl, 1-Methylpropylsulfinyl, 2-Methylpropyl-sulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Dimethylpropylsulfinyl, 1-Ethylpropyl-sulfinyl, 1,1-Dimethylpropylsulfinyl, 1,2-Dimethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl;
- C₁-C₆-Halogenalkylsulfinyl sowie die C₁-C₆-Halogenalkylsulfinyl-Teile von C₁-C₆-Halogenalkylsulfinyl-C₁-C₄-alkyl: C₁-C₆-Alkylsulfinylrest wie voranstehend genannt, der partiell oder vollständig durch Fluor, Chlor, Brom und/oder Iod substituiert ist, also z.B. Fluormethylsulfinyl, Difluormethylsulfinyl, Trifluormethylsulfinyl, Chlordifluormethylsulfinyl, Bromdifluormethylsulfinyl, 2-Fluorethylsulfinyl, 2-Chlorethylsulfinyl, 2-Bromethylsulfinyl, 2-lodethylsulfinyl, 2,2-Difluorethylsulfinyl, 2,2,2-Trifluor-ethylsulfinyl, 2,2,2-Trichlorethylsulfinyl, 2-Chlor-2-fluorethylsulfinyl, 2-Chlor-2,2-difluorethylsulfinyl, 2,2-Dichlor-2-fluorethylsulfinyl, Pentafluorethylsulfinyl, 2-Fluorpropylsulfinyl, 3-Fluorpropylsulfinyl, 2-Chlorpropylsulfinyl, 3-Chlorpropyl-sulfinyl, 2-Brompropylsulfinyl, 3-Brompropylsulfinyl, 2,2-Difluorpropylsulfinyl, 2,3-Difluorpropylsulfinyl, 2,3-Dichlorpropylsulfinyl, 3,3,3-Trifluorpropylsulfinyl, 3,3,3-Trichlorpropylsulfinyl, 2,2,3,3,3-Pentafluorpropylsulfinyl, Heptafluorpropylsulfinyl, 1-(Fluormethyl)-2-fluorethylsulfinyl, 1-(Chlormethyl)-2-chlorethylsulfinyl, 1-(Brommethyl)-2-bromethylsulfinyl, 4-Fluorbutylsulfinyl, 4-Chlorbutylsulfinyl, 4-Brombutylsulfinyl, Nonafluorbutylsulfinyl, 5-Fluorpentylsulfinyl, 5-Chlorpentyl-sulfinyl, 5-Brompentylsulfinyl, 5-Iodpentylsulfinyl, Undecafluorpentylsulfinyl, 6-Fluorhexylsulfinyl, 6-Chlorhexylsulfinyl, 6-Bromhexylsulfinyl, 6-Iodhexylsulfinyl und Dodecafluorhexylsulfinyl;
- C₁-C₆-Alkylsulfonyl (C₁-C₆-Alkyl-S(O)₂-) sowie die C₁-C₆-Alkylsulfonyl-Teile von C₁-C₆-Alkylsulfonyl-C₁-C₄-alkyl, C₁-C₆-Alkylsulfonylamino, C₁-C₆-Alkylsulfonylamino-C₁-C₄-alkyl, C₁-C₆-Alkylsulfonyl-(C₁-C₆-alkylamino)-C₁-C₄-alkyl: z.B. Methylsulfonyl, Ethylsulfonyl, Propylsulfonyl, 1-Methylethylsulfonyl, Butylsulfonyl, 1-Methylpropylsulfonyl, 2-Methyl-propylsulfonyl, 1,1-Dimethylethylsulfonyl, Pentylsulfonyl, 1-Methylbutylsulfonyl, 2-Methylbutylsulfonyl, 3-Methylbutylsulfonyl, 1,1-Dimethylpropylsulfonyl, 1,2-Di-methylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;
- C₁-C₆-Halogenalkylsulfonyl sowie die C₁-C₆-Halogenalkylsulfonyl-Teile von C₁-C₆-Halogenalkylsulfonyl-C₁-C₄-alkyl, C₁-C₆-Halogenalkylsulfonylamino: einen C₁-C₆-Alkylsulfonylrest wie voranstehend genannt, der partiell oder vollständig durch Fluor, Chlor, Brom und/oder Iod substituiert ist, also z.B. Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-lodethylsulfonyl, 2,2-Difluorethyl-sulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, Pentafluorethylsulfonyl, 2-Fluorpropylsulfonyl, 3-Fluorpropylsulfonyl, 2-Chlor-propylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluor-propylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl, Nonafluorbutylsulfonyl, 5-Fluorpentylsulfonyl, 5-Chlorpentylsulfonyl, 5-Brompentylsulfonyl, 5-lod-pentylsulfonyl, 6-Fluorhexylsulfonyl, 6-Bromhexylsulfonyl, 6-Iodhexylsulfonyl und Dodecafluorhexylsulfonyl;
   C₁-C₄-Alkoxy sowie die Alkoxyteile von Hydroxycarbonyl-C₁-C₄-alkoxy, C₁-C₄-Alkoxycarbonyl-C₁-C₄-alkoxy, C₁-C₄-Alkoxy-C₁-C₄-alkoxy-C₁-C₄-alkyl und C₁-C₄-alkyl-C₁-C₄-alkoxycarbonylamino: z.B. Methoxy, Ethoxy, Propoxy, 1-Methyl-ethoxy, Butoxy, 1-Methylpropoxy, 2-Methylpropoxy und 1,1-Dimethylethoxy;
- C₁-C₆-Alkoxy sowie die Alkoxyteile von Hydroxycarbonyl-C₁-C₆-alkoxy, C₁-C₆-Alkoxycarbonyl-C₁-C₆-alkoxy, N-(C₁-C₆-Alkoxy)-N-(C₁-C₆-alkyl)-aminocarbonyl, N-(C₃-C₆-Alkenyl)-N-(C₁-C₆-alkoxy)-aminocarbonyl, N-(C₃-C₆-Alkinyl)-N-(C₁-C₆-alkoxy)-aminocarbonyl und C₁-C₆-Alkoxyimino-C₁-C₆-alkyl: C₁-C₄-Alkoxy wie voranstehend genannt, sowie z.B. Pentoxy, 1-Methyl-butoxy, 2-Methylbutoxy, 3-Methoxylbutoxy, 1,1-Dimethyl-propoxy, 1,2-Dimethyl-propoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Di-methylbutoxy,1,2-Dimethyl-butoxy, 1,3-Dimethylbutoxy, 2,2-Dimethylbutoxy, 2,3-Dimethylbutoxy, 3,3-Dimethyl-butoxy, 1-Ethylbutoxy, 2-Ethylbutoxy, 1,1,2-Tri-methylpropoxy, 1,2,2-Trimethyl-propoxy, 1-Ethyl-1-methylpropoxy und 1-Ethyl-2-methylpropoxy;
- C₁-C₄-Halogenalkoxy: ein C₁-C₄-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Brommethoxy, 2-lodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₁-C₆-Halogenalkoxy sowie die C₁-C₆-Halogenalkoxy-Teile von C₁-C₆-Halogenalkoxy-C₁-C₄-alkyl, C₁-C₆-Halogenalkoxycarbonyl-C₁-C₄-alkyl: C₁-C₄-Halogenalkoxy wie voranstehend genannt, sowie z.B. 5-Fluorpentoxy, 5-Chlorpentoxy, 5-Brompentoxy, 5-lodpentoxy, Undecafluorpentoxy, 6-Fluorhexoxy, 6-Chlorhexoxy, 6-Bromhexoxy, 6-Iodhexoxy und Dodecafluorhexoxy;
- C₁-C₆-Alkoxy-C₁-C₄-alky sowie die C₁-C₆-Alkoxy-C₁-C₄-alkyl-Teile von C₁-C₆-Alkoxy-C₁-C₄-alkoxy-C₁-C₄-alkyl I: durch C₁-C₆-Alkoxy wie vorstehend genannt substituiertes C₁-C₄-Alkyl, also z.B. für Methoxymethyl, Ethoxymethyl, Propoxymethyl, (1-Methylethoxy)methyl, Butoxymethyl, (1-Methylpropoxy)methyl, (2-Methyl-propoxy)methyl, (1,1-Dimethylethoxy)methyl, 2-(Methoxy)ethyl, 2-(Ethoxy)ethyl, 2-(Propoxy)ethyl, 2-(1-Methylethoxy)ethyl, 2-(Butoxy)ethyl, 2-(1-Methylpropoxy)-ethyl, 2-(2-Methylpropoxy)ethyl, 2-(1,1-Dimethylethoxy)ethyl, 2-(Methoxy)-propyl, 2-(Ethoxy)propyl, 2-(Propoxy)propyl, 2-(1-Methylethoxy)propyl, 2-(Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)-propyl, 3-(Propoxy)propyl, 3-(1-Methylethoxy)-propyl, 3-(Butoxy)propyl, 3-(1-Methylpropoxy)propyl, 3-(2-Methylpropoxy)propyl, 3-(1,1-Dimethylethoxy)propyl, 2-(Methoxy)-butyl, 2-(Ethoxy)butyl, 2-(Propoxy)-butyl, 2-(1-Methylethoxy)butyl, 2-(Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)-butyl, 3-(Propoxy)butyl, 3-(1-Methylethoxy)butyl, 3-(Butoxy)-butyl, 3-(1-Methyl-propoxy)-butyl, 3-(2-Methylpropoxy)butyl, 3-(1,1-Dimethylethoxy)butyl, 4-(Methoxy)-butyl, 4-(Ethoxy)butyl, 4-(Propoxy)butyl, 4-(1-Methylethoxy)butyl, 4-(Butoxy)butyl, 4-(1-Methylpropoxy)butyl, 4-(2-Methylpropoxy)butyl und 4-(1,1-Dimethylethoxy)-butyl;
- C₁-C₄-Alkoxycarbonyl sowie die Alkoxycarbonylteile von C₁-C₄-Alkoxycarbonyl-C₁-C₄-alkoxy, C₁-C₄-Alkoxy-C₁-C₄-alkoxycarbonyl und Di-(C₁-C₄-alkyl)-amino-C₁-C₄-alkoxycarbonyl: z.B. Methoxycarbonyl, Ethoxycarbonyl, Propoxycarbonyl, 1-Methylethoxycarbonyl, Butoxycarbonyl, 1-Methylpropoxycarbonyl, 2-Methylpropoxycarbonyl oder 1, 1-Dimethylethoxycarbonyl;
- C₁-C₆-Alkoxycarbonyl sowie die Alkoxycarbonylteile von C₁-C₆-Alkoxycarbonyl-C₁-C₆-alkoxy und C₁-C₆-Alkoxycarbonylamino-C₁-C₄-alkyl: C₁-C₄-Alkoxycarbonyl, wie voranstehend genannt, sowie z.B. Pentoxycarbonyl, 1-Methylbutoxycarbonyl, 2-Methylbutoxycarbonyl, 3-Methyl-butoxycarbonyl, 2,2-Dimethylpropoxycarbonyl, 1-Ethylpropoxycarbonyl, Hexoxy-carbonyl, 1,1-Dimethylpropoxycarbonyl, 1,2-Dimethylpropoxycarbonyl, 1-Methyl-pentoxycarbonyl, 2-Methylpentoxycarbonyl, 3-Methylpentoxycarbonyl, 4-Methyl-pentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, 1,1,2-Trimethylpropoxycarbonyl, 1,2,2-Trimethylpropoxycarbonyl, 1-Ethyl-1-methyl-propoxycarbonyl oder 1-Ethyl-2-methyl-propoxycarbonyl;
- C₁-C₄-Alkylthio sowie die C₁-C₄-Alkylthio-Teile von C₁-C₆-Halogenalkyl-C₁-C₄-thioalkyl, C₂-C₆-Halogenalkenyl-C₁-C₄-thioalkyl, C₂-C₆-Halogenalkinyl-C₁-C₄-thioalkyl: z.B. Methylthio, Ethylthio, Propylthio, 1-Methylethylthio, Butylthio, 1-Methylpropylthio, 2-Methylpropylthio und 1,1-Dimethylethylthio;
- C₁-C₆-Alkylthio sowie die C₁-C₆-Alkylthio-Teile von C₁-C₆-Alkylthio-C₁-C₄-alkyl: C₁-C₄-Alkylthio wie voranstehend genannt, sowie z.B. Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutyl-thio, 2,3-Dimethylbutylthio, 3,3-Dimethylbutylthio, 1-Ethylbutylthio, 2-Ethylbutyl-thio, 1,1,2-Trimethylpropylthio, 1,2,2-Trimethylpropylthio, 1-Ethyl-1-methylpropyl-thio und 1-Ethyl-2-methylpropylthio;
- C₁-C₆-Alkylamino sowie die C₁-C₆-Alkylaminoreste von N(C₁-C₆-Alkylamino)imino-C₁-C₆-alkyl, C₁-C₆-Alkylamino-C₁-C₄-alkyl, C₁-C₆-Alkylsulfonyl-(C₁-C₆-alkylamino)-C₁-C₄-alkyl, C₁-C₆-Alkylcarbonyl-(C₁-C₆-alkylamino)-Cₗ-C₄-alkyl und [(C₁-C₆-Alkyl)amino]cyanoimino: z.B. Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylarnino, 1-Methylpentylamino, 2-Methylpentylamino, 3-Methyl-pentylamino, 4-Methylpentylamino, 1,1-Dimethylbutylamino, 1,2-Dimethylbutyl-arnino, 1,3-Dimethylbutyfamino, 2,2-Dimethylbutylamino; 2,3-Dimethylbutylamino, 3,3-Dimethylbutylamino, 1-Ethylbutylamino, 2-Ethylbutylamino, 1,1,2-Trimethyl-propylamino, 1,2,2-Trimethyl-propylamino, 1-Ethyl-1-methylpropylamino oder 1-Ethyl-2-methylpropylamino;
- Di(C₁-C₄-alkyl)amino: z.B. N,N-Dimethylamino, N,N-Diethylamino, N,N-Dipropylamino, N,N-Di-(1-methylethyl)-amino, N,N-Dibutylamino, N,N-Di-(1-methylpropyl)amino, N,N-Di-(2-methylpropyl)-amino, N,N-Di-(1,1-dimethylethyl)-amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethyl-ethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)-amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl-)amino, N-(1-Methylethyl)-N-(1-methylpropyl)amino, N-(1-Methylethyl)-N-(2-methyl-propyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethyl-ethyl)amino, N-(1-Methylpropyl)-N-(2-methylpropyl)amino, N-(1,1-Dimethylethyl)-N-(1-methylpropyl)amino und N-(1,1-Dimethylethyl)-N-(2-methylpropyl)amino;
- Di(C₁-C₆-alkyl)amino sowie die Dialkylaminoreste von N-(Di-C₁-C₆-alkylamino)-imino-C₁-C₆-alkyl, Di(C₁-C₆-alkyl)amino-C₁-C₄-alkyl, [Di(C₁-C₆-alkyl)aminocabonyloxy]-C₁-C₄-alkyl, {Di[di(C₁-C₆-alkyl)amino]cabonyloxy}-C₁-C₄-alkyl und [Di(C₁-C₆-alkyl)amino]cyanoimino: Di(C₁-C₄-alkyl)amino wie voranstehend genannt sowie: z.B. N,N-Dipentylamino, N,N-Dihexylamino, N-Methyl-N-pentylamino, N-Ethyl-N-pentylamino, N-Methyl-N-hexylamino und N-Ethyl-N-hexylamino;(C₁-C₄-Alkylamino)carbonyl: z.B. Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl oder 1,1-Dimethylethylaminocarbonyl;
- (C₁-C₄-Alkylamino)carbonyl sowie die (C₁-C₄-Alkylamino)carbonyl-Teile von (C₁-C₄-Alkylamino)carbonylamino: z.B. Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl oder 1,1-Dimethylethylaminocarbonyl;
- Di(C₁-C₄-alkyl)aminocarbonyl sowie die Di(C₁-C₄-alkyl)aminocarbonyl-Teile von Di(C₁-C₄-alkyl)aminocarbonylamino: z.B. N,N-Dimethylaminocarbonyl, N,N-Diethylaminocarbonyl, N,N-Di-(1-methylethyl)aminocarbonyl, N,N-Dipropylaminocarbonyl, N,N-Dibutylaminocarbonyl, N,N-Di-(1-methylpropyl)aminocarbonyl, N,N-Di-(2-methylpropyl)aminocarbonyl, N,N=Di-(1,1-dimethylethyl)aminocarbonyl, N-Ethyl-N-methylaminocarbonyl, N-Methyl-N-propylaminocarbonyl, N-Methyl-N-(1-methyl-ethyl)aminocarbonyl, N-Butyl-N-methylaminocarbonyl, N-Methyl-N-(1-methylpropyl)aminocarbonyl, N-Methyl-N-(2-methylpropyl)aminocarbonyl, N-(1,1-Dimethylethyl)-N-methylaminocarbonyl, N-Ethyl-N-propylaminocarbonyl, N-Ethyl-N-(1-methylethyl)aminocarbonyl, N-Butyl-N-ethylaminocarbonyl, N-Ethyl-N-(1-methylpropyl)aminocarbonyl, N-Ethyl-N-(2-methylpropyl)aminocarbonyl, N-Ethyl-N-(1,1-dimethylethyl)aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)aminocarbonyl, N-(1-Methylethyl)-N-(1-methylpropyl)-aminocarbonyl, N-(1-Methylethyl)-N-(2-methylpropyl)aminocarbonyl, N-(1,1-Dimethylethyl)-N-(1-methylethyl)aminocarbonyl, N-Butyl-N-(1-methylpropyl)-aminocarbonyl, N-Butyl-N-(2-methylpropyl)aminocarbonyl, N-Butyl-N-(1,1-dimethylethyl)aminocarbonyl, N-(1-Methylpropyl)-N-(2-methylpropyl)aminocarbonyl, N-(1,1-Dimethylethyl)-N-(1-methylpropyl)aminocarbonyl oder N-(1,1-Dimethylethyl)-N-(2-methylpropyl)aminocarbonyl;
- (C₁-C₆-Alkylamino)carbonyl sowie die (C₁-C₆-Alkylamino)carbonyl-Teile von (C₁-C₆-Alkylamino)carbonylamino, (C₁-C₆-Alkylamino)carbonyloxy-C₁-C₄-alkyl, C₁-C₆-Alkylaminocarbonyl-C₁-C₄-alkyl und [(C₁-C₆-Alkyl)aminocarbonylamino]-C₁-C₄-alkyl: (C₁-C₄-Alkylamino)carbonyl, wie voranstehend genannt, sowie z.B. Pentylaminocarbonyl, 1-Methylbutylaminocarbonyl, 2-Methyl-butylaminocarbonyl, 3-Methylbutylaminocarbonyl, 2,2-Dimethylpropylamino-carbonyl, 1-Ethylpropylaminocarbonyl, Hexylaminocarbonyl, 1,1-Dimethylpropyl-aminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylamino-carbonyl, 1,1-Dimethylbutylaminocarbonyl, 1,2-Dimethylbutylaminocarbonyl, 1,3-Dimethylbutyl-aminocarbonyl, 2,2-Dimethylbutylaminocarbonyl, 2,3-Dimethylbutyl-aminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methylpropylaminocarbonyl oder 1-Ethyl-2-methylpropylaminocarbonyl;
- Di(C₁-C₆-alkyl)aminocarbonyl sowie die Di(C₁-C₆-alkyl)aminocarbonyl-Teile von Di(C₁-C₆-alkyl)aminocarbonylamino, Di(C₁-C₆-alkyl)aminocarbonyl-C₁-C₄-alkyl und [Di(C₁-C₆-alkyl)aminocarbonylamino]-C₁-C₄-alkyl: Di(C₁-C₄-alkyl)aminocarbonyl, wie voranstehend genannt, sowie z.B. N-Methyl-N-pentylaminocarbonyl, N-Methyl-N-(1-methylbutyl)-aminocarbonyl, N-Methyl-N-(2-methylbutyl)-aminocarbonyl, N-Methyl-N-(3-methylbutyl)-aminocarbonyl, N-Methyl-N-(2,2-dimethylpropyl)-aminocarbonyl, N-Methyl-N-(1-ethylpropyl)-aminocarbonyl, N-Methyl-N-hexylaminocarbonyl, N-Methyl-N-(1,1-dimethylpropyl)-aminocarbonyl, N-Methyl-N-(1,2-dimethylpropyl)-aminocarbonyl, N-Methyl-N-(1-methylpentyl)-aminocarbonyl, N-Methyl-N-(2-methy)pentyl)-aminocarbonyl, N-Methyl-N-(3-methylpentyl)-aminocarbonyl, N-Methyl-N-(4-methylpentyl)-aminocarbonyl, N-Methyl-N-(1,1-dimethylbutyl)-aminocarbonyl, N-Methyl-N-(1,2-dimethylbutyl)-aminocarbonyl, N-Methyl-N-(1,3-dimethylbutyl)-aminocarbonyl, N-Methyl-N-(2,2-dimethylbutyl)-aminocarbonyl, N-Methyl-N-(2,3-dimethylbutyl)- amino-carbonyl, N-Methyl-N-(3,3-dimethylbutyl)-aminocarbonyl, N-Methyl-N-(1-ethyl-butyl)-aminocarbonyl, N-Methyl-N-(2-ethylbutyl)-aminocarbonyl, N-Methyl-N-(1,1,2-trimethylpropyl)-aminocarbonyl, N-Methyl-N-(1,2,2-trimethylpropyl)-amino-carbonyl, N-Methyl-N-(1-ethyl-1-methylpropyl)-aminocarbonyl, N-Methyl-N-(1-ethyl-2-methylpropyl)-aminocarbonyl, N-Ethyl-N-pentylaminocarbonyl, N-Ethyl-N-(1-methylbutyl)-aminocarbonyl, N-Ethyl-N-(2-methylbutyl)-aminocarbonyl, N-Ethyl-N-(3-methylbutyl)-aminocarbonyl, N-Ethyl-N-(2,2-dimethylpropyl)-aminocarbonyl, N-Ethyl-N-(1-ethylpropyl)-aminocarbonyl, N-Ethyl-N-hexylaminocarbonyl, N-Ethyl-N-(1,1-dimethylpropyl)-aminocarbonyl, N-Ethyl-N-(1,2-dimethylpropyl)-aminocarbonyl, N-Ethyl-N-(1-methylpentyl)-aminocarbonyl, N-Ethyl-N-(2-methylpentyl)-aminocarbonyl, N-Ethyl-N-(3-methylpentyl)-aminocarbonyl, N-Ethyl-N-(4-methylpentyl)-aminocarbonyl, N-Ethyl-N-(1,1-dimethylbutyl)-aminocarbonyl, N-Ethyl-N-(1,2-dimethylbutyl)-aminocarbonyl, N-Ethyl-N-(1,3-dimethylbutyl)-aminocarbonyl, N-Ethyl-N-(2,2-dimethylbutyl)- amino-carbonyl, N-Ethyl-N-(2,3-dimethylbutyl)-aminocarbonyl, N-Ethyl-N-(3,3-dimethylbutyl)-aminocarbonyl, N-Ethyl-N-(1-ethylbutyl)-amino-carbonyl, N-Ethyl-N-(2-ethylbutyl)-aminocarbonyl, N-Ethyl-N-(1,1,2-trimethyl-propyl)-aminocarbonyl, N-Ethyl-N-(1,2,2-trimethylpropyl)-aminocarbonyl, N-Ethyl-N-(1-ethyl-1-methylpropyl)-aminocarbonyl, N-Ethyl-N-(1-ethyl-2-methylpropyl)-aminocarbonyl, N-Propyl-N-pentylaminocarbonyl, N-Butyl-N-pentylaminocarbonyl, N,N-Dipentylaminocarbonyl, N-Propyl-N-hexyl-aminocarbonyl, N-Butyl-N-hexylaminocarbonyl, N-Pentyl-N-hexylaminocarbonyl oder N,N-Dihexylamino-carbonyl;
- Di(C₁-C₆-alkyl)aminothiocarbonyl: z.B. N,N-Dimethylaminothiocarbonyl, N,N-Diethylaminothiocarbonyl, N,N-Di-(1-methylethyl)aminothiocarbonyl, N,N-Dipropylaminothiocarbonyl, N,N-Dibutylaminothiocarbonyl, N,N-Di-(1-methylpropyl)-aminothiocarbonyl, N,N-Di-(2-methylpropyl)-aminothiocarbonyl, N,N-Di-(1,1-dimethylethyl)-aminothiocarbonyl, N-Ethyl-N-methylaminothiocarbonyl, N-Methyl-N-propylaminothiocarbonyl, N-Methyl-N-(1-methylethyl)-aminothiocarbonyl, N-Butyl-N-methylaminothiocarbonyl, N-Methyl-N-(1-methylpropyl)-aminothiocarbonyl, N-Methyl-N-(2-methylpropyl)aminothiocarbonyl, N-(1,1-Dimethylethyl)-N-methylaminothiocarbonyl, N-Ethyl-N-propylaminothiocarbonyl, N-Ethyl-N-(1-methylethyl)-aminothiocarbonyl, N-Butyl-N-ethylaminothiocarbonyl, N-Ethyl-N-(1-methylpropyl)-aminothiocarbonyl, N-Ethyl-N-(2-methylpropyl)-aminothiocarbonyl, N-Ethyl-N-(1,1-dimethylethyl)-aminothiocarbonyl, N-(1-Methylethyl)-N-propylaminothiocarbonyl, N-Butyl-N-propylaminothiocarbonyl, N-(1-Methylpropyl)-N-propylaminothiocarbonyl, N-(2-Methylpropyl)-N-propylamino-thiocarbonyl, N-(1,1-Dimethylethyl)-N-propylaminothiocarbonyl, N-Butyl-N-(1-methylethyl)-aminothiocarbonyl, N-(1-Methylethyl)-N-(1-methylpropyl)-aminothiocarbonyl, N-(1-Methylethyl)-N-(2-methylpropyl)-aminothiocarbonyl, N-(1,1-Dimethylethyl)-N-(1-methylethyl)-aminothiocarbonyl, N-Butyl-N-(1-methylpropyl)-aminothiocarbonyl, N-Butyl-N-(2-methylpropyl)-aminothiocarbonyl, N-Butyl-N-(1,1-dimethylethyl)-aminothiocarbonyl, N-(1-Methylpropyl)-N-(2-methylpropyl)-aminothiocarbonyl, N-(1,1-Dimethylethyl)-N-(1-methylpropyl)-aminothiocarbonyl, N-(1,1-Dimethylethyl)-N-(2-methylpropyl)-aminothiocarbonyl, N-Methyl-N-pentylaminothiocarbonyl, N-Methyl-N-(1-methylbutyl)-aminothio-carbonyl, N-Methyl-N-(2-methylbutyl)-aminothiocarbonyl, N-Methyl-N-(3-methylbutylraminothiocarbonyl, N-Methyl-N-(2,2-dimethylpropyl)-aminothio-carbonyl, N-Methy4-N-(1-ethylpropyl)-aminothiocarbonyl, N-Methyl-N-hexyl-aminothiocarbonyl, N-Methyl-N- (1,1-dimethylpropyl)-aminothiocarbonyl, N-Methy)-N-(1,2-dimethylpropyl)-aminothiocarbonyl, N-Methyl-N-(1-methylpentyl)-aminothiocarbonyl, N-Methyl-N-(2-methylpentyl)-aminothiocarbonyl, N-Methyl-N-(3-methylpentyl)-aminothiocarbonyl, N-Methyl-N-(4-methylpentyl)-aminothio-carbonyl, N-Methyl-N-(1,1-dimethylbutyl)-aminothiocarbonyl, N-Methyl-N-(1,2-dimethylbutyl)-aminothiocarbonyl, N-Methyl-N-(1,3-dimethylbutyl)-aminothiocarbonyl, N-Methyl-N-(2,2-dimethylbutyl)- aminothiocarbonyl, N-Methyl-N-(2,3-dimethylbutyl)-aminothiocarbonyl, N-Methyl-N-(3,3-dimethylbutyl)-aminothiocarbonyl, N-Methyl-N-(1-ethylbutyl)-aminothiocarbonyl, N-Methyl-N-(2-ethylbutyl)-aminothiocarbonyl, N-Methyl-N-ethyl-N-(1,1,2-trimethylpropyl)-aminothiocarbonyl, N-Methyl-N-(1,2,2-trimethylpropyl)-aminothiocarbonyl, N-Methyl-N-(1-ethyl-1-methylpropyl)-aminothiocarbonyl, N-Methyl-N-(1-ethyl-2-methylpropyl)-aminothiocarbonyl, N-Ethyl-N-pentyl-aminothiocarbonyl, N-Ethyl-N-(1-methylbutyl)-aminothiocarbonyl, N-Ethyl-N-(2-methylbutyl)-aminothiocarbonyl, N-Ethyl-N-(3-methylbutyl)- aminothiocarbonyl, N-Ethyl-N-(2,2-dimethylpropyl)-aminothiocarbonyl, N-Ethyl-N-(1-ethylpropyl)-aminothiocarbonyl, N-Ethyl-N-hexylaminothiocarbonyl, N-Ethyl-N-(1,1-dimethyl-propyl)-aminothiocarbonyl, N-Ethyl-N-(1,2-dimethylpropyl)- aminothiocarbonyl, N-Ethyl-N-(1-methylpentyl)-aminothiocarbonyl, N-Ethyl-N-(2-methylpentyl)-aminothiocarbonyl, N-Ethyl-N-(3-methylpentyl)-aminothiocarbonyl, N-Ethyl-N-(4-methylpentyl)-aminothiocarbonyl, N-Ethyl-N-(1,1-dimethylbutyl)-amino-thiocarbonyl, N-Ethyl-N-(1,2-dimethylbutyl)- aminothiocarbonyl, N-Ethyl-N-(1,3-dimethylbutyl)-aminothiocarbonyl, N-Ethyl-N-(2,2-dimethylbutyl)-aminothiocarbonyl, N-Ethyl-N-(2,3-dimethylbutyl)-aminothiocarbonyl, N-Ethyl-N-(3,3-dimethylbutyl)-aminothiocarbonyl, N-Ethyl-N-(1-ethylbutyl)-aminothiocarbonyl, N-Ethyl-N-(2-ethylbutyl)-aminothiocarbonyl, N-Ethyl-N-(1,1,2-trimethylpropyl)-aminothiocarbonyl, N-Ethyl-N-(1,2,2-trimethylpropyl)-aminothiocarbonyl, N-Ethyl-N-(1-ethyl-1-methylpropyl)-aminothiocarbonyl, N-Ethyl-N-(1-ethyl-2-methylpropyl)-aminothiocarbonyl, N-Propyl-N-pentylaminothiocarbonyl, N-Butyl-N-pentylaminothiocarbonyl, N,N-Dipentylaminothiocarbonyl, N-Propyl-N-hexyl-aminothiocarbonyl, N-Butyl-N-hexylaminothiocarbonyl, N-Pentyl-N-hexyl-aminothiocarbonyl oder N,N-Dihexylaminothiocarbonyl;
- drei- bis sechsgliedriges Heterocyclyl sowie die drei- bis sechsgliedrigen Heterocyclyl-Teile von drei- bis sechsgliedriges Heterocyclyl-C₁-C₄-alkyl: monocyclische, gesättigte oder partiell ungesättigte Kohlenwasserstoffe mit drei bis sechs Ringgliedem wie voranstehend genannt, welche neben Kohlenstoffatomen ein bis vier Stickstoffatome, oder ein bis drei Stickstoffatome und ein Sauerstoff- oder Schwefelatom, oder ein bis drei Sauerstoffatome, oder ein bis drei Schwefelatome enthalten können und welche über ein C-Atom oder ein N-Atom verknüpft sein können,
   z.B. 2-Oxrianyl, 2-Oxetanyl, 3-Oxetanyl, 2-Aziridinyl, 3-Thiethanyl, 1-Azetidinyl, 2-Azetidinyl,
   z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thia-diazolidin-2-yl, 1,3,4-Triazolidin-2-yl, 1,2,3,4-Tetrazolidin-5-yl;
   z.B. 1-Pyrrolidinyl, 2-Isothiazolidinyl, 2-Isothiazolidinyl, 1-Pyrazolidinyl, 3-Oxazolidinyl, 3-Thiazolidinyl, 1-Imidazolidinyl, 1,2,4-Triazotidin-1-yl, 1,2,4-Oxadiazolidin-2-yl, 1,2,4-Oxadiazolidin-4-yl, 1,2,4-Thiadiazolidin-2-yl, 1,2,4-Thiadiazolidin-4-yl, 1,2,3,4-Tetrazolidin-1-yl,
   z.B. 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 4,5-Dihydropyrrol-2-yl, 4,5-Dihydropyrrol-3-yl, 2,5-Dihydropyrrol-2-yl, 2,5-Dihydropyrrol-3-yl, 4,5-Dihydroisoxazol-3-yl, 2,5-Dihydroisoxazol-3-yl, 2,3-Di-hydroisoxazol-3-yl, 4,5-Dihydroisoxazol-4-yl, 2,5-Dihydroisoxazol-4-yl, 2,3-Dihydroisoxazol-4-yl, 4,5-Dihydroisoxazol-5-yl, 2,5-Dihydroisoxazol-5-yl, 2,3-Dihydroisoxazol-5-yl, 4,5-Dihydroisothiazol-3-yl, 2,5-Dihydroisothiazol-3-yl, 2,3-Dihydroisothiazol-3-yl, 4,5-Dihydroisothiazol-4-yl, 2,5-Dihydroisothiazol-4-yl, 2,3-Dihydro-isothiazol-4-yl, 4,5-Dihydroisothiazol-5-yl, 2,5-Dihydroisothiazol-5-yl, 2,3-Dihydroisothiazol -5-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-3-yl, 3,4-Dihydropyrazol-4-yl, 3,4-Dihydropyrazol-5-yl, 4,5-Dihydropyrazol-3-yl, 4,5-Dihydropyrazol-4-yl, 4,5-Dihydropyrazol-5-yl, 2,3-Dihydroimidazol-2-yl, 2,3-Dihydroimidazol-3-yl ,2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2,3-Dihydrooxazol-3-yl, 2,3-Dihydrooxazol-4-yl, 2,3-Dihydrooxazol-5-yl, 3,4-Dihydrooxazol-3-yl, 3,4-Dihydrooxazol-4-yl, 3,4-Dihydrooxazol-5-yl, 2,3-Dihydrothiazol-3-yl, 2,3-Dihydrothiazol-4-yl, 2,3-Dihydrothiazol-5-yl, 3,4-Dihydrothiazol-3-yl, 3,4-Dihydrothiazol-4-yl, 3,4-Dihydrothiazol-5-yl, 3,4-Dihydrothiazol-2-yl, 3,4-Dihydrothiazol-3-yl, 3,4-Dihydrothiazol-4-yl,
   z.B. 4,5-Dihydropyrrol-1-yl, 2,5-Dihydropyrrol-1-yl, 4,5-Dihydroisoxazol-2-yl, 2,3-Dihydroisoxazol-1-yl, 4,5-Dihydroisothiazol-1-yl, 2,3-Dihydroisothiazol-1-yl, 2,3-Dihydropyrazol-1-yl, 4,5-Dihydropyrazol-1-yl, 3,4-Dihydropyrazol-1-yl, 2,3-Dihydroimidazol-1-yl, 4,5-Dihydroimidazol-1-yl, 2,5-Dihydroimidazol-1-yl, 2,3-Dihydrooxazol-2-yl, 3,4-Dihydrooxazol-2-yl, 2,3-Dihydrothiazol-2-yl, 3,4-Dihydrothiazol-2-yl;
   z.B. 2-Piperidinyl, 3-Piperidinyl, 4-Piperidinyl, 1,3-Dioxan-2-yl, 1,3-Dioxan-4-yl, 1,3-Dioxan-5-yl, 1,4-Dioxan-2-yl, 1,3-Dithian-2-yl, 1,3-Dithian-3-yl, 1,3-Dithian-4-yl, 1,4-Dithian-2-yl, 1,3-Dithian-5-yl, 2-Tetrahydropyranyl, 3-Tetrahydropyranyl, 4-Tetrahydropyranyl, 2-Tetrahydrothiopyranyl, 3-Tetrahydrothiopyranyl, 4-Tetrahydrothiopyranyl 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydrotriazin-2-yl, 1,2,4-Hexahydrotriazin-3-yl, Tetrahydro-1,3-oxazin-2-yl, Tetrahydro-1,3-oxazin-6-yl, 2-Morpholinyl, 3-Morpholinyl, 1,3,5-Trioxan-2-yl;
   z.B. 1-Piperidinyl, 1-Hexahydropyridazinyl, 1-Hexahydropyrimidinyl, 1-Piperazinyl, 1,3,5-Hexahydrotriazin-1-yl, 1,2,4-Hexahydrotriazin-1-yl, Tetrahydro-1,3-oxazin-1-yl, 1-Morpholinyl;
   z.B. 2H-Pyran-2-yl, 2H-Pyran-3-yl, 2H-Pyran-4-yl, 2H-Pyran-5-yl, 2H-Pyran-6-yl, 3,6-Dihydro-2H-pyran-2-yl, 3,6-Dihydro-2H-pyran-3-yl, 3,6-Dihydro-2H-pyran-4-yl, 3,6-Dihydro-2H-pyran-5-yl, 3,6-Dihydro-2H-pyran-6-yl, 3,4-Dihydro-2H-pyran-3-yl, 3,4-Dihydro-2H-pyran-4-yl, 3,4-Dihydro-2H-pyran-6-yl, 2H-Thiopyran-2-yl, 2H-Thiopyran-3-yl, 2H-Thiopyran-4-yl, 2H-Thiopyran-5-yl, 2H-Thiopyran-6-yl, 5,6-Dihydro-4H-1,3-oxazin-2-yl
- Aryl sowie der Arylteil von Aryl(C₁-C₄-alkyl): ein- bis dreikemiger aromatischer Carbocyclus mit 6 bis 14 Ringgliedern, wie z.B. Phenyl, Naphthyl und Anthracenyl;
- Heteroaryl sowie die Heteroarylreste in Heteroaryl-C₁-C₄-alkyl, Heteroaryl-C₁-C₄alkyl, Heteroaryl-C₂-C₄-alkenyl, Heteroaryl-C₂-C₄-alkinyl, Heteroaryl-C₁-C₄halogenalkyl, Heteroaryl-C₂-C₄-halogenalkenyl, Heteroaryl-C₂-C₄-halogenalkinyl, Heteroaryl-C₁-C₄-hydroxyalkyl, Heteroaryl-C₂-C₄-hydroxyalkenyl, Heteroaryl-C₂-C₄hydroxyalkinyl, Heteroarylcarbonyl-C₁-C₄-alkyl, Heteroarylcarbonyloxy-C₁-C₄-alkyl, Heteroaryloxycarbonyl-C₁-C₄-alkyl, Heteroaryloxy-C₁-C₄-alkyl, Heteroarylthio-C₁-C₄alkyl, Heteroarylsulfinyl-C₁-C₄-alkyl, Heteroarylsulfonyl-C₁-C₄-alkyl : mono- oder bicyclisches aromatisches Heteroaryl mit 5 bis 10 Ringgliedern, welches neben Kohlenstoffatomen 1 bis 4 Stickstoffatome, oder 1 bis 3 Stickstoffatome und ein Sauerstoff- oder ein Schwefelatom, oder ein Sauerstoff- oder ein Schwefelatom enthält, z.B.
   Monocyclen wie Furyl (z.B. 2-Furyl, 3-Furyl), Thienyl (z.B. 2-Thienyl, 3-Thienyl), Pyrrolyl (z.B. Pyrrol-2-yl, Pyrrol-3-yl), Pyrazolyl (z.B. Pyrazol-3-yl, Pyrazol-4-yl), Isoxazolyl (z.B. Isoxazol-3-yl, Isoxazol-4-yl, Isoxazol-5-yl), Isothiazolyl (z.B. Isothiazol-3-yl, Isothiazol-4-yl, Isothiazol-5-yl), Imidazolyl (z.B. Imidazol-2-yl, Imidazol-4-yl), Oxazolyl (z.B. Oxazol-2-yl, Oxazol-4-yl, Oxazol-5-yl), Thiazolyl (z.B. Thiazol-2-yl, Thiazol-4-yl, Thiazol-5-yl), Oxadiazolyl (z.B. 1,2,3-Oxadiazol-4-yl, 1,2,3-Oxadiazol-5-yl, 1,2,4-Oxadiazol-3-yl, 1,2,4,-Oxadiazol-5-yl, 1,3,4-Oxadiazol-2-yl), Thiadiazolyl (z.B. 1,2,3-Thiadiazol-4-yl, 1,2,3-Thiadiazol-5-yl, 1,2,4-Thiadiazol-3-yl, 1,2,4-Thiadiazol-5-yl, 1,3,4-Thiadiazolyl-2-yl), Triazolyl (z.B. 1,2,3-Triazol-4-yl, 1,2,4-Triazol-3-yl), Tetrazol-5-yl, Pyridyl (z.B. Pyridin-2-yl, Pyridin-3-yl, Pyridin-4-yl), Pyrazinyl (z.B. Pyridazin-3-yl, Pyridazin-4-yl), Pyrimidinyl (z.B. Pyrimidin-2-yl, Pyrimidin-4-yl, Pyrimidin-5-yl), Pyrazin-2-yl, Triazinyl (z.B. 1,3,5-Triazin-2-yl, 1,2,4-Triazin-3-yl, 1,2,4-Triazin-5-yl, 1,2,4-Triazin-6-yl), Tetrazinyl (z.B. 1,2,4,5-Tetrazin-3-yl); sowie
   Bicyclen wie die benzanellierten Derivate der vorgenannten Monocyclen, z.B. Chinolinyl, Isochinolinyl, Indolyl, Benzthienyl, Benzofuranyl, Benzoxazolyl, Benzthiazolyl, Benzisothiazolyl, Benzimidazolyl, Benzopyrazolyl, Benzthiadiazolyl, Benzotriazolyl.

Alle Phenyl- und Arylringe bzw. Heterocyclyl- und Heteroarylreste sowie alle Phenylkomponenten in Phenyl-C₁-C₆-alkyl, Phenylcarbonyl, Phenylcarbonyl-C₁-C₆-alkyl, Phenylcarbonylamino-C₁-C₄-alkyl, Phenoxycarbonyl, Phenylaminocarbonyl, Phenylsulfonylaminocarbonyl, N-(C₁-C₆-Alkyl)-N-phenylaminocarbonyl und Phenyl-C₁-C₆-alkylcarbonyl, alle Arylkomponenten in Aryl(C₁-C₄-alkyl), alle Heteroarylkomponenten in mono- oder bicyclischem Heteroaryl und alle Heterocyclylkomponenten in Heterocyclyl, Heterocyclyl-C₁-C₆-alkyl, Heterocyclylcarbonyl, Heterocyclylcarbonyl-C₁-C₆-alkyl, Heterocyclyloxycarbonyl, Heterocyclylaminocarbonyl, Heterocyclylsulfonylaminocarbonyl, N-(C₁-C₆-Alkyl)-N-heterocyclylaminocarbonyl und Heterocyclyl-C₁-C₆-alkylcarbonyl sind, soweit nicht anders angegeben, vorzugsweise unsubstituiert oder tragen ein bis drei Halogenatome und/oder eine Nitrogruppe, einen Cyanorest und/oder einen oder zwei Methyl-, Trifluormethyl-, Methoxy- oder Trifluormethoxysubstituenten.

In einer besonderen Ausführungsform haben die Variablen der benzoylsubstituierten Serin-Amide der Formel I folgende Bedeutungen, wobei diese für sich allein betrachtet als auch in Kombination miteinander besondere Ausgestaltungen der Verbindungen der Formel I darstellen:

Bevorzugt sind die benzoylsubstituierten Serin-Amide der Formel I, in der
- R¹: Halogen, C₁-C₄-Alky) oder C₁-C₆-Halogenalkyl; besonders bevorzugt Halogen oder C₁-C₆-Halogenalkyl; insbesondere bevorzugt Halogen oder C₁-C₄-Halogenalkyl; außerordentlich bevorzugt Fluor, Chlor oder CF₃;
bedeutet.

Ebenso bevorzugt sind die benzoylsubstituierten Serin-Amide der Formel I, in der
R² und R³ unabhängig voneinander
Wasserstoff, Halogen, C₁-C₄-Alkyl oder C₁-C₆-Halogenalkyl;
sehr bevorzugt Wasserstoff, Halogen oder C₁-C₈-Halogenalkyl;
besonders bevorzugt Wasserstoff, Halogen oder C₁-C₄-Halogenalkyl;
insbesondere bevorzugt Wasserstoff, Fluor, Chlor oder CF₃;
außerordentlich bevorzugt Wasserstoff, Fluor oder Chlor;
sehr außerordentlich bevorzugt Wasserstoff oder Fluor;
bedeuten.

Ebenso bevorzugt sind die benzoylsubstituierten Serin-Amide der Formel I, in der
- R⁴: Wasserstoff, Halogen, C₁-C₄-Alkyl oder C₁-C₄-Halogenalkyl; besonders bevorzugt Wasserstoff, Halogen oder C₁-C₄-Alkyl; insbesondere bevorzugt Wasserstoff oder Halogen; außerordentlich bevorzugt Wasserstoff;
bedeutet.

Ebenso bevorzugt sind die benzoylsubstituierten Serin-Amide der Formel I, in der
- R⁵: Wasserstoff, Halogen, C₁-C₄-Alkyl oder C₁-C₄-Halogenalkyl; besonders bevorzugt Wasserstoff, Halogen oder C₁-C₄-Alkyl: insbesondere bevorzugt Wasserstoff oder Halogen; außerordentlich bevorzugt Wasserstoff;
bedeutet.

Ebenso bevorzugt sind die benzoylsubstituierten Serin-Amide der Formel 1, in der
- R6: Wasserstoff;
bedeutet.

Ebenso bevorzugt sind die benzoylsubstituierten Serin-Amide der Formel I, in der
- R⁷: Wasserstoff oder Hydroxy;
besonders bevorzugt Wasserstoff;
bedeutet.

Ebenso bevorzugt sind die benzoylsubstituierten Serin-Amide der Formel I, in der
- R⁶: Wasserstoff; und
- R⁷: Wasserstoff oder Hydroxy; besonders bevorzugt Wasserstoff;
bedeuten.

Ebenso bevorzugt sind die benzoylsubstituierten Serin-Amide der Formel I, in der
- R⁸: C₁-C₆-Alkyl oder C₁-C₆-Halogenalkyl; besonders bevorzugt C₁-C₆-Alkyl; insbesondere bevorzugt C₁-C₄-Alkyl; außerordentlich bevorzugt CH₃;
bedeutet.

Ebenso bevorzugt sind die benzoylsubstituierten Serin-Amide der Formel I, in der
- R⁹: Wasserstoff, C₁-C₆-Alkyl, C₃-C₆-Alkenyl, C₃-C₆-Alkinyl, Formyl, C₁-C₆-Alkylcarbonyl, C₂-C₆-Alkenylcarbonyl, C₃-C₆-Cycloalkylcarbonyl, C₁-C₆-Alkoxycarbonyl, C₁-C₆-Alkylaminocarbonyl, C₁-C₆-Alkylsulfonylaminocarbonyl, Di-(C₁-C₆-alkyl)-aminocarbonyl, N-(C₁-C₆-Alkoxy)-N-(C₁-C₆-alkyl)-aminocarbonyl, Di-(C₁-C₆-alkyl)-aminothicarbonyl, C₁-C₆-Alkoxyimino-C₁-C₆-alkyl,
wobei die genannten Alkyl, Cycloalkyl- und Alkoxyreste partiell oder vollständig halogeniert sein können und/oder eine bis drei der folgenden Gruppen tragen können: Cyano, Hydroxy, C₃-C₆-Cycloalkyl, C₁-C₄-Alkoxy, C₁-C₄-Alkylthio, Di-(C₁-C₄-alkyl)-amino, C₁-C₄-Alkylcarbonyl, Hydroxycarbonyl, C₁-C₄-Alkoxycarbonyl, Aminocarbonyl, C₁-C₄-Alkylaminocarbonyl, Di-(C₁-C₄-alkyl)-aminocarbonyl, oder C₁-C₄-Alkylcarbonyloxy;
Phenyl, Phenyl-C₁-C₆-alkyl, Phenylcarbonyl, Phenylcarbonyl-C₁-C₆-alkyl, Phenylsulfonylaminocarbonyl oder Phenyl-C₁-C₆-alkylcarbonyl,
wobei der Phenylring partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy; oder
SO₂R¹²;
besonders bevorzugt Wasserstoff, C₁-C₆-Alkyl, C₃-C₆-Alkenyl, C₃-C₆-Alkinyl, Formyl, C₁-C₆-Alkylcarbonyl, C₂-C₆-Alkenylcarbonyl, C₁-C₆-Alkoxycarbonyl, C₁-C₆-Alkylsulfonylaminocarbonyl, Di-(C₁-C₆-alkyl)-aminocarbonyl, N-(C₁-C₆-Alkoxy)-N-(C₁-C₆-alkyl)-aminocarbonyl oder Di-(C₁-C₆-alkyl)-aminothiocarbonyl, wobei die genannten Alkyl- oder Alkoxyreste partiell oder vollständig halogeniert sein können und/oder eine bis drei der folgenden Gruppen tragen können: Cyano, C₁-C₄-Alkoxy, C₁-C₄-Alkoxycarbonyl, C₁-C₄-Alkylaminocarbonyl, Di-(C₁-C₄-alkyl)-aminacarbonyl oder C₁-C₄-Alkylcarbonyloxy;
Phenyl-C₁-C₆-alkyl, Phenylcarbonyl, Phenylcarbonyl-C₁-C₆-alkyl, Phenylsulfonylaminocarbonyl oder Phenyl-C₁-C₆-alkylcarbonyl,
wobei der Phenylring partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenoxy; oder SO₂R¹²;
insbesondere bevorzugt Wasserstoff, C₁-C₆-Alkyl, C₃-C₆-Alkenyl, C₃-C₆-Alkinyl, Formyl, C₁-C₆-Alkyl-carbonyl, C₂-C₆-Alkenylcarbonyl, C₁-C₆-Alkoxycarbonyl, Di-(C₁-C₆-alkyl)-amino-carbonyl, N-(C₁-C₆-Alkoxy)-N-(C₁-C₆-alkyl)-aminocarbonyl, Di-(C₁-C₆-alkyl)-aminothiocarbonyl, Phenyl-C₁-C₆-alkyl, Phenylcarbonyl, Phenylcarbonyl-C₁-C₆-alkyl oder Phenyl-C₁-C₆-alkylcarbonyl wobei der Phenylring partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogen-alkoxy; oder
SO₂R¹²;
bedeutet.

Ebenso bevorzugt sind die benzoylsubstituierten Serin-Amide der Formel I, in der
- R⁹: Wasserstoff, C₁-C₆-Alkyl, C₃-C₆-Alkenyl, C₃-C₆-Alkinyl, Formyl, C₁-C₆-Alkylcarbonyl, C₂-C₆-Alkenylcarbonyl, C₃-C₆-Cycloalkylcarbonyl, C₁-C₆-Alkoxycarbonyl, C₁-C₆-Alkylaminocarbonyl, Di-(C₁-C₆-alkyl)-aminocarbonyl, N-(C₁-C₆-Alkoxy)-N-(C₁-C₆-alkyl)-aminocarbonyl, Di-(C₁-C₆-alkyl)-aminothiocarbonyl, C₁-C₆-Alkoxyimino-C₁-C₆-alkyl,
wobei die genannten Alkyl-, Cycloalkyl- oder Alkoxyreste partiell oder vollständig halogeniert sein können und/oder eine bis drei der folgenden Gruppen tragen können: Cyano, Hydroxy, C₃-C₆-Cycloalkyl, C₁-C₄-Alkoxy, C₁-C₄-Alkylthio, Di-(C₁-C₄-alkyl)-amino, C₁-C₄-Alkylcarbonyl, Hydroxycarbonyl, C₁-C₄-Alkoxycarbonyl, Aminocarbonyl, C₁-C₄-Alkylaminocarbonyl, Di-(C₁-C₄-alkyl)-aminocarbonyl oder C₁-C₄-Alkylcarbonyloxy, oder
SO₂R¹²;
bedeutet.

Ebenso bevorzugt sind die benzoylsubstituierten Serin-Amide der Formel I, in der
- R⁹: Wasserstoff, C₁-C₆-Alkyl, C₃-C₆-Alkenyl, C₃-C₆-Alkinyl, Formyl, C₁-C₆-Alkylcarbonyl, C₁-C₆-Alkoxycarbonyl, C₁-C₆-Alkylaminocarbonyl, Di-(C₁-C₆-alkyl)-aminocarbonyl, N-(C₁-C₆-Alkoxy)-N-(C₁-C₆-alkyl)-amino-carbonyl,
wobei-die genannten Alkyl-,und Alkoxyreste partiell oder vollständig halogeniert sein können und/oder eine bis drei der folgenden Gruppen tragen können: Cyano, C₁-C₄-Alkoxy, C₁-C₄-Alkylaminocarbonyl oder Di-(C₁-C₄-alkyl)-aminocarbonyl;
Phenyl-C₁-C₆-alkyl, Phenylcarbonyl, Phenylcarbonyl-C₁-C₆-alkyl, Phenylaminocarbonyl oder N-(C₁-C₆-Alkyl)-N-(phenyl)-aminocarbonyl,
wobei der Phenylring partiell oder vollständig halogeniert sein kann und/oder eine bis drei der folgenden Gruppen tragen kann: Cyano, C₁-C₄-Alkyl oder C₁-C₄-Halogenalkyl; oder
SO₂R¹²;
besonders bevorzugt Wasserstoff, Formyl, C₁-C₄-Alkylcarbonyl, C₁-C₄-Alkylaminocarbonyl, Di-(C₁-C₄-alkyl)-aminocarbonyl, Phenylaminocarbonyl, N-(C₁-C₄-Alkyl)-N-(phenyl)-aminocarbonyl, SO₂CH₃, SO₂CF₃ oder SO₂(C₆H₅);
bedeutet.

Ebenso bevorzugt sind die benzoylsubstituierten Serin-Amide der Formel I, in der
- R¹⁰: Wasserstoff oder C₁-C₄-Alkyl; bevorzugt Wasserstoff oder CH₃; insbesondere bevorzugt Wasserstoff;
bedeutet.

Ebenso bevorzugt sind die benzoylsubstituierten Serin-Amide der Formel I, in der
- R¹¹: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₁-C₆-Cyanoalkyl, C₁-C₆-Hydroxyalkyl, C₂-C₆-Hydroxyalkenyl, C₂-C₆-Hydroxyalkinyl, C₃-C₆-Cycloalkyl, C₃-C₆-Cycloalkenyl, 3- bis 6-gliedriges Heterocyclyl-C₁-C₄-alkyl,
wobei die voranstehend genannten Cycloalkyl, Cycloalkenyl oder 3- bis 6-gliedrigen Heterocyclylreste partiell oder vollständig halogeniert sein können und/oder einen bis drei Reste aus der Gruppe Oxo, C₁-C₆-Alkyl, C₁-C₆-Halogenalkyl, Hydroxycarbonyl und C₁-C₆-Alkoxycarbonyl tragen können,
C₁-C₆-Alkoxy-C₁-C₄-alkyl, C₁-C₆-Halogenalkoxy-C₁-C₄-alkyl, C₁-C₆-Alkoxy-C₁-C₄-alkoxy-C₁-C₄-alkyl, C₁-C₆-Alkylthio-C₁-C₄-alkyl, C₁-C₆-Alkylsulfonylamino-C₁-C₄-alkyl, Hydroxycarbonyl, C₁-C₆-Alkoxycarbonyl, Hydroxycarbonyl-C₁-C₄-alkyl, C₁-C₆-Alkoxycarbonyl-C₁-C₄-alkyl, C₁-C₆-Halogenalkoxycarbonyl-C₁-C₄-alkyl, C₁-C₆-Alkylcarbonyloxy-C₁-C₄-alkyl, C₁-C₆-Alkylcarbonylamino-C₁-C₄-alkyl, Di(C₁-C₆-Alkyl)carbonylamino-C₁-C₄-alkyl, Di(C₁-C₆-alkyl)aminocarbonylamino-C₁-C₄-alkyl, [(C₁-C₆-alkyl)aminocarbonyl]amino-C₁-C₄-alkyl, [Di(C₁-C₆-alkyl)aminocarbonyl-oxy]C₁-C₄-alkyl, {Di[di(C₁-C₆-alkyl)amino]carbonyloxy}C₁-C₄-alkyl, Formylamino-C₁-C₄-alkyl,
Phenyl-C₁-C₄-alkyl, Phenyl-C₂-C₄-alkenyl, Phenyl-C₂-C₄-alkinyl, Phenyl-C₁-C₄-halogenalkyl, Phenyl-C₂-C₄-halogenalkenyl, Phenyl-C₁-C₄-hydroxyalkyl, Pheny-loxy-C₁-C₄-alkyl, Phenylthio-C₁-C₄-alkyl, Phenylsulfinyl-C₁-C₄-alkyl, Phenylsulfonyl-C₁-C₄-alkyl, Heteroaryl-C₁-C₄-alkyl, Heteroaryl-C₁-C₄-hydroxyalkyl, Heteroaryloxy-C₁-C₄-alkyl, Heteroarylthio-C₁-C₄-alkyl, Heteroarylsulfinyl-C₁-C₄-alkyl oder Heteroarylsulfonyl-C₁-C₄-alkyl,
wobei die vorstehend genannten Phenyl- und Heteroarylreste partiell oder vollständig halogeniert sein können und/oder ein bis drei Reste aus der Gruppe Cyano, Nitro, C₁-C₆-Alkyl, C₁-C₆-Halogenalkyl, Hydroxy, C₁-C₆-Alkoxy, C₁-C₆-Halogenalkoxy, Hydroxycarbonyl, C₁-C₆-Alkoxycarbonyl, Hydroxycarbonyl-C₁-C₆-alkoxy, C₁-C₆-Alkylsulfonylamino und C₁-C₆-Halogenalkylsulfonylamino tragen können;
besonders bevorzugt C₁-C₆-Alkyl, C₂-C₆-Alkenyl, C₂-C₆-Alkinyl, C₁-C₆-Halogenalkyl, C₂-C₆-Halogenalkenyl, C₁-C₆-Hydroxyalkyl, C₁-C₆-Alkoxy-C₁-C₄-alkyl, C₁-C₆-Halogenalkoxy-C₁-C₄-alkyl, Hydroxycarbonyl-C₁-C₄-alkyl, C₁-C₆-Alkoxycarbonyl-C₁-C₄-alkyl, C₁-C₆-Alkylcarbonyloxy-C₁-C₄-alkyl, C₁-C₆-Alkylcarbonylamino-C₁-C₄-alkyl, [Di(C₁-C₆-alkyl)aminocarbonyloxy]C₁-C₄-alkyl, {Di[di(C₁-C₆-alkyl)amino]carbonyloxy}C₁-C₄-alkyl, Formylamino-C₁-C₄-alkyl; Phenyl-C₁-C₄-alkyl, Phenyl-C₂-C₄-alkenyl, Phenyl-C₂-C₄-alkinyl, Phenyl-C₁-C₄-halogenalkyl, Phenyl-C₂-C₄-halogenalkenyl, Phenyl-C₁-C₄-hydroxyalkyl, Phenyloxy-C₁-C₄-alkyl, Phenylthio-C₁-C₄-alkyl, Phenylsulfinyl-C₁-C₄-alkyl oder Phenylsulfonyl-C₁-C₄-alkyl,
wobei die vorstehend genannten Phenylreste partiell oder vollständig halogeniert sein können und/oder ein bis drei Reste aus der Gruppe C₁-C₆-Alkyl, C₁-C₆-HalogenalkylC₁-C₆-Alkoxy, Hydroxycarbonyl, C₁-C₆-Alkoxycarbonyl, C₁-C₆-Alkylsulfonylamino und C₁-C₆-Halogenalkylsulfonylamino tragen können;
insbesondere bevorzugt C₁-C₆-Alkyl, C₂-C₆-AJkenyl, C₂-C₆-Alkinyl, C₁-C₆-Halogenalkyl, C₂-C₆-Halogenalkenyl, C₁-C₆-Hydroxyalkyl, Hydroxycarbonyl-C₁-C₄-alkyl, C₁-C₆-Alkoxycarbonyl-C₁-C₄-alkyl, [Di(C₁-C₆-alkyl)aminocarbonyloxy]C₁-C₄-alkyl, {Di[di(C₁-C₆-alkyl)amino]carbonyloxy}C₁-C₄-alkyl, Formylamino-C₁-C₄-alkyl; Phenyl-C₁-C₄-alkyl, Phenyl-C₂-C₄-alkenyl, Phenyl-C₁-C₄-hydroxyalkyl oder Phenylthio-C₁-C₄-alkyl;
außerordentlich bevorzugt C₁-C₆-Alkyl, C₂-C₆-Alkenyl, C₁-C₆-Halogenalkyl, C₂-C₆-Halogenalkenyl, C₁-C₆-Hydroxyalkyl, Hydroxycarbonyl-C₁-C₄-alkyl, Formylamino-C₁-C₄-alkyl, Phenyl-C₁-C₄-alkyl oder Phenyl-C₁-C₄-hydroxyalkyl;
bedeutet.

Ebenso bevorzugt sind die benzoylsubstituierten Serin-Amide der Formel I, in der
- R¹¹: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₁-C₆-Cyanoalkyl, C₁-C₆-Hydroxyalkyl, C₂-C₆-Hydroxyalkenyl, C₂-C₆-Hydroxyalkinyl, C₃-C₆-Cycloalkyl, C₃-C₆-Cycloalkenyl, 3- bis 6-gliedriges Heterocyclyl,
wobei die voranstehend genannten Cycloalkyl, Cycloalkenyl oder 3- bis 6-gliedrigen Heterocyclylreste partiell oder vollständig halogeniert sein können und/oder einen bis drei Rest aus der Gruppe Oxo, C₁-C₆-Alkyl, C₁-C₆-Halogenalkyl, Hydroxycarbonyl und C₁-C₆-Alkoxycarbonyl tragen können,
C₁-C₆-Alkoxy-C₁-C₄-alkyl, C₁-C₆-Halogenalkoxy-C₁-C₄-alkyl, C₁-C₆-Alkoxy-C₁-C₄-alkoxy-C₁-C₄-alkyl, C₁-C₆-Alkylthio-C₁-C₄-alkyl, C₁-C₆-Alkylsulfonylamino-C₁-C₄-alkyl, Hydroxycarbonyl, C₁-C₆-Alkoxycarbonyl, Hydroxycarbonyl-C₁-C₄-alkyl, C₁-C₆-Alkoxycarbonyl-C₁-C₄-alkyl, C₁-C₆-Halogenalkoxycarbonyl-C₁-C₄-alkyl, C₁-C₆-Alkylcarbonyloxy-C₁-C₄-alkyl, C₁-C₆-Alkylcarbonylamino-C₁-C₄-alkyl, Di(C₁-C₆-Alkyl)carbonylamino-C₁-C₄-alkyl, Di(C₁-C₆-alkyl)aminocarbonylamino-C₁-C₄-alkyl, [(C₁-C₆-alkyl)aminocarbonyl]amino-C₁-C₄-alkyl, [Di(C₁-C₆-alkyl)aminocarbonyloxy]C₁-C₄-alkyl, Formylamino-C₁-C₄-alkyl, Phenyl-C₁-C₄-alkyl, Phenyl-C₂-C₄-alkenyl, Phenyl-C₂-C₄-alkinyl, Phenyl-C₁-C₄-halogenalkyl, Phenyl-C₂-C₄-halogenalkenyl, Phenyl-C₁-C₄-hydroxyalkyl, Phenyloxy-C₁-C₄-alkyl, Phenylthio-C₁-C₄-alkyl, Phenylsulfinyl-C₁-C₄-alkyl, Phenylsulfonyl-C₁-C₄-alkyl,
Heteroaryl-C₁-C₄-alkyl, Heteroaryl-C₁-C₄-hydroxyalkyl, Heteroaryloxy-C₁-C₄-alkyl, Heteroarylthio-C₁-C₄-alkyl, Heteroarylsulfinyl-C₁-C₄-alkyl oder Heteroarylsulfonyl-C₁-C₄-alkyl,
wobei die vorstehend genannten Phenyl- und Heteroarylreste partiell oder vollständig halogeniert sein können und/oder ein bis drei Reste aus der Gruppe Cyano, Nitro, C₁-C₆-Alkyl, C₁-C₆-Halogenalkyl, Hydroxy, C₁-C₆-Alkoxy, C₁-C₆-Halogenalkoxy, Hydroxycarbonyl, C₁-C₆-Alkoxycarbonyl, Hydroxycarbonyl-C₁-C₆-alkoxy, C₁-C₆-Alkylsulfonylamino und C₁-C₆-Halogenalkylsulfonylamino tragen können;
besonders bevorzugt C₁-C₆-Alkyl, C₂-C₆-Alkenyl, C₂-C₆-Alkinyl, C₁-C₆-Halogenalkyl, C₂-C₆-Halogenalkenyl, C₁-C₆-Hydroxyalkyl, 3- bis 6-gliedriges Heterocyclyl, C₁-C₆-Alkoxy-C₁-C₄-alkyl, C₁-C₆-Alkoxy-C₁-C₄-alkoxy-C₁-C₄-alkyl, C₁-C₆-Halogenalkoxy-C₁-C₄-alkyl, Hydroxycarbonyl-C₁-C₄-alkyl, C₁-C₆-Alkoxycarbonyl-C₁-C₄-alkyl, C₁-C₆-Alkylcarbonyloxy-C₁-C₄-alkyl, C₁-C₆-Alkylcarbonylamino-C₁-C₄-alkyl, [Di(C₁-C₆-alkyl)aminocarbonyloxy]C₁-C₄-alkyl, Formylamino-C₁-C₄-alkyl;
Phenyl-C₁-C₄-alkyl, Phenyl-C₂-C₄-alkenyl, Phenyl-C₂-C₄-alkinyl, Phenyl-C₁-C₄-halogenalkyl, Phenyl-C₂-C₄-halogenalkenyl, Phenyl-C₁-C₄-hydroxyalkyl, Phenyloxy-C₁-C₄-alkyl, Phenylthio-C₁-C₄-alkyl, Phenylsulfinyl-C₁-C₄-alkyl oder Phenylsulfonyl-C₁-C₄-alkyl,
wobei die vorstehend genannten Phenylreste partiell oder vollständig halogeniert sein können und/oder ein bis drei Reste aus der Gruppe C₁-C₆-Alkyl, C₁-C₆-HalogenalkylC₁-C₆-Alkoxy, Hydroxycarbonyl, C₁-C₆-Alkoxycarbonyl, C₁-C₆-Alkylsulfonylamino und C₁-C₆-Halogenalkylsulfonylamino tragen können;
insbesondere bevorzugt C₁-C₆-Alkyl, C₂-C₆-Alkenyl, C₂-C₆-Alkinyl, C₁-C₆-Halogenalkyl, C₂-C₆-Halogenalkenyl, C₁-C₆-Hydroxyalkyl, 3- bis 6-gliedriges Heterocyclyl, C₁-C₆-Alkoxy-C₁-C₄-alkyl, C₁-C₆-Alkoxy-C₁-C₄-alkoxy-C₁-C₄-alkyl, Hydroxycarbonyl-C₁-C₄-alkyl, C₁-C₆-Alkoxycarbonyl-C₁-C₄-alkyl, [Di(C₁-C₆-alkyl)aminocarbonyloxy]C₁-C₄-alkyl, Formylamino-C₁-C₄-alkyl; Phenyl-C₁-C₄-alkyl, Phenyl-C₂-C₄-alkenyl, Phenyl-C₁-C₄-hydroxyalkyl oder Phenylthio-C₁-C₄-alkyl;
außerordentlich bevorzugt C₁-C₆-Alkyl, C₂-C₆-Alkenyl, C₁-C₆-Halogenalkyl, C₂-C₆-Halogenalkenyl, C₁-C₆-Hydroxyalkyl, C₁-C₆-Alkoxy-C₁-C₄-alkyl, C₁-C₆-Alkoxy-C₁-C₄-alkoxy-C₁-C₄-alkyl, 3- bis 6-gliedriges Heterocyclyl, Hydroxycarbonyl-C₁-C₄-alkyl, Formylamino-C₁-C₄-alkyl, Phenyl-C₁-C₄-alkyl oder Phenyl-C₁-C₄-hydroxyalkyl;
bedeutet.

Ebenso bevorzugt sind die benzoylsubstituierten Serin-Amide der Formel I, in der
- R¹²: C₁-C₆-Alkyl, C₁-C₆-Halogenalkyl oder Phenyl,
wobei der Phenylrest partiell oder teilweise halogeniert sein kann und/oder durch C₁-C₄-Alkyl substituiert sein kann;
besonders bevorzugt C₁-C₄-Alkyl, C₁-C₄-Halogenalkyl oder Phenyl;
insbesondere bevorzugt Methyl, Trifluormethyl oder Phenyl
bedeutet.

Besonders bevorzugt sind die benzoylsubstituierten Serin-Amide der Formel I, in der
- R¹: Fluor, Chlor oder CF₃;
- R² und R³: unabhängig voneinander Wasserstoff, Fluor oder Chlor;
- R⁴, R⁵, R⁶ und R⁷: Wasserstoff;
- R⁸: C₁-C₄-Alkyl, besonders bevorzugt CH₃;
- R⁹: Wasserstoff, Formyl, C₁-C₄-Alkylcarbonyl, C₁-C₄-Alkylaminocarbonyl, Di-(C₁-C₄-Alkyl)-aminocarbonyl, Phenylaminocarbonyl, N-(C₁-C₄-alkyl)-N-(phenyl)-aminocarbonyl, SO₂CH₃, SO₂CF₃ oder SO₂(C₆H₅);
- R¹⁰: Wasserstoff: und
- R¹¹: C₁-C₆-Alkyl, C₂-C₆-Alkenyl, C₁-C₆-Halogenalkyl, C₂-C₆-Halogenalkenyl, C₁-C₆-Hydroxyalkyl, C₁-C₆-Alkoxy-C₁-C₄-alkyl, C₁-C₆-Alkoxy-C₁-C₄-alkoxy-C₁-C₄-alkyl, 3- bis 6-gliedriges Heterocyclyl, Hydroxycarbonyl-C₁-C₄-alkyl, Phenyl-C₁-C₄-alkyl oder Phenyl-C₁-C₄-hydroxyalkyl
bedeuten.

Außerordentlich bevorzugt sind die Verbindungen der Formel I.a. (entspricht Formel I mit R¹= CF₃, R², R³, R⁴, R⁵, R⁶, R⁷ und R¹⁰ = H; R⁸ = CH₃), insbesondere die Verbindungen der Formel I.a.1 bis I.a.138 der Tabelle 1, wobei die Definitionen der Variablen R¹ bis R¹¹ nicht nur in Kombination miteinander, sondern auch jeweils für sich allein betrachtet für die erfindungsgemäßen Verbindungen eine besondere Rolle spielen.

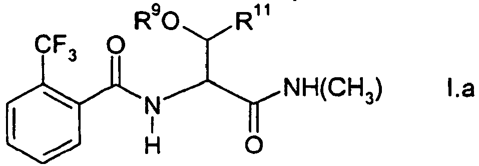

**Tabelle 1**

| Nr. | R9 | R¹¹ |
|---|---|---|
| I.a.1 | H | CH₃ |
| I.a.2 | H | CH₂CH₃ |
| I.a.3 | H | CH=CH₂ |
| I.a.4 | H | CH=CHCH₃ |
| I.a.5 | H | CH=C(CH₃)₂ |
| I.a.6 | H | C≡CH |
| I.a.7 | H | CF₃ |
| I.a.8 | H | CHF₂ |
| I.a.9 | H | CF₂CF₃ |
| I.a.10 | H | CF₂CHF₂ |
| I.a.11 | H | CH=CCl₂ |
| I.a.12 | H | CH=CF₂ |
| I.a.13 | H | CH₂OH |
| I.a.14 | H | CH₂CH₂OH |
| I.a.15 | H | CH(OH)CH₂OH |
| I.a.16 | H | Cyclopropyl |
| I.a.17 | H | Cyclohexyl |
| I.a.18 | H | CH₂COOH |
| I.a.19 | H | CH₂O(CO)CH₃ |
| I.a.20 | H | CH₂O(CO)N(CH₃)₂ |
| I.a.21 | H | C≡C(C₆H₅) |
| I.a.22 | H | CH(OH)CH(OH)C₆H₅ |
| I.a.23 | H | CH₂S(2-F-C₆H₄) |
| I.a.24 | C(O)CH₃ | CH₃ |
| I.a.25 | C(O)CH₃ | CH₂CH₃ |
| I.a.26 | C(O)CH₃ | CH=CH₂ |
| I.a.27 | C(O)CH₃ | CH=CHCH₃ |
| I.a.28 | C(O)CH₃ | CH=C(CH₃)₂ |
| I.a.29 | C(O)CH₃ | C≡CH |
| I.a.30 | C(O)CH₃ | CF₃ |
| I.a.31 | C(O)CH₃ | CHF₂ |
| I.a.32 | C(O)CH₃ | CF₂CF₃ |
| I.a.33 | C(O)CH₃ | CF₂CHF₂ |
| I.a.34 | C(O)CH₃ | CH=CCl₂ |
| I.a.35 | C(O)CH₃ | CH=CF₂ |
| I.a.36 | C(O)CH₃ | CH₂OH |
| I.a.37 | C(O)CH₃ | CH₂CH₂OH |
| I.a.38 | C(O)CH₃ | CH(OH)CH₂OH |
| I.a.39 | C(O)CH₃ | Cyclopropyl |
| I.a.40 | C(O)CH₃ | Cyclohexyl |
| I.a.41 | C(O)CH₃ | CH₂COOH |
| I.a.42 | C(O)CH₃ | CH₂O(CO)CH₃ |
| I.a.43 | C(O)CH₃ | CH₂O(CO)N(CH₃)₂ |
| I.a.44 | C(O)CH₃ | C≡C(C₆H₅) |
| I.a.45 | C(O)CH₃ | CH(OH)CH(OH)C₆H₅ |
| I.a.46 | C(O)CH₃ | CH₂S(2-F-C₆H₄) |
| I.a.47 | C(O)*tert*C₄H₉ | CH₃ |
| I.a.48 | C(O)*tert*C₄H₉ | CH₂CH₃ |
| I.a.49 | C(O)*tert*C₄H₉ | CH=CH₂ |
| I.a.50 | C(O)*tert*C₄H₉ | CH=CHCH₃ |
| I.a.51 | C(O)*tert*C₄H₉ | CH=C(CH₃)₂ |
| I.a.52 | C(O)*tert*C₄H₉ | C=CH |
| I.a.53 | C(O)*tert*C₄H₉ | CF₃ |
| I.a.54 | C(O)*tert*C₄H₉ | CHF₂ |
| I.a.55 | C(O)*tert*C₄H₉ | CF₂CF₃ |
| I.a.56 | C(O)*tert*C₄H₉ | CF₂CHF₂ |
| I.a.57 | C(O)*tert*C₄H₉ | CH=CCl₂ |
| I.a.58 | C(O)*tert*C₄H₉ | CH=CF₂ |
| I.a.59 | C(O)*tert*C₄H₉ | CH₂OH |
| I.a.60 | C(O)*tert*C₄H₉ | CH₂CH₂OH |
| I.a.61 | C(O)*tert*C₄H₉ | CH(OH)CH₂OH |
| I.a.62 | C(O)*tert*C₄H₉ | Cyclopropyl |
| I.a.63 | C(O)*tert*C₄H₉ | Cyclohexyl |
| I.a.64 | C(O)*tert*C₄H₉ | CH₂COOH |
| I.a.65 | C(O)*tert*C₄H₉ | CH₂O(CO)CH₃ |
| I.a.66 | C(O)*tert*C₄H₉ | CH₂O(CO)N(CH₃)₂ |
| I.a.67 | C(O)*tert*C₄H₉ | C≡C(C₆H₅) |
| I.a.68 | C(O)*tert*C₄H₉ | CH(OH)CH(OH)C₆H₅ |
| I.a.69 | C(O)*tert*C₄H₉ | CH₂S(2-F-C₆H₄) |
| I.a.70 | C(O)N(CH₃)₂ | CH₃ |
| I.a.71 | C(O)N(CH₃)₂ | CH₂CH₃ |
| I.a.72 | C(O)N(CH₃)₂ | CH=CH₂ |
| I.a.73 | C(O)N(CH₃)₂ | CH=CHCH₃ |
| I.a.74 | C(O)N(CH₃)₂ | CH=C(CH₃)₂ |
| I.a.75 | C(O)N(CH₃)₂ | C=CH |
| I.a.76 | C(O)N(CH₃)₂ | CF₃ |
| I.a.77 | C(O)N(CH₃)₂ | CHF₂ |
| I.a.78 | C(O)N(CH₃)₂ | CF₂CF₃ |
| I.a.79 | C(O)N(CH₃)₂ | CF₂CHF₂ |
| I.a.80 | C(O)N(CH₃)₂ | CH=CCl₂ |
| I.a.81 | C(O)N(CH₃)₂ | CH=CF₂ |
| I.a.82 | C(O)N(CH₃)₂ | CH₂OH |
| I.a.83 | C(O)N(CH₃)₂ | CH₂CH₂OH |
| I.a.84 | C(O)N(CH₃)₂ | CH(OH)CH₂OH |
| I.a.85 | C(O)N(CH₃)₂ | Cyclopropyl |
| I.a.86 | C(O)N(CH₃)₂ | Cyclohexyl |
| I.a.87 | C(O)N(CH₃)₂ | CH₂COOH |
| I.a.88 | C(O)N(CH₃)₂ | CH₂O(CO)CH₃ |
| I.a.89 | C(O)N(CH₃)₂ | CH₂O(CO)N(CH₃)₂ |
| I.a.90 | C(O)N(CH₃)₂ | C≡C(C₆H₅) |
| I.a.91 | C(O)N(CH₃)₂ | CH(OH)CH(OH)C₆H₅ |
| I.a.92 | C(O)N(CH₃)₂ | CH₂S(2-F-C₆H₄) |
| I.a.93 | C(O)N(CH₃)(C₆H₅) | CH₃ |
| I.a.94 | C(O)N(CH₃)(C₆H₅) | CH₂CH₃ |
| I.a.95 | C(O)N(CH₃)(C₆H₅) | CH=CH₂ |
| I.a.96 | C(O)N(CH₃)(C₆H₅) | CH=CHCH₃ |
| I.a.97 | C(O)N(CH₃)(C₆H₅) | CH=C(CH₃)₂ |
| I.a.98 | C(O)N(CH₃)(C₆H₅) | C=CH |
| I.a.99 | C(O)N(CH₃)(C₆H₅) | CF₃ |
| I.a.100 | C(O)N(CH₃)(C₆H₅) | CHF₂ |
| I.a.101 | C(O)N(CH₃)(C₆H₅) | CF₂CF₃ |
| I.a.102 | C(O)N(CH₃)(C₆H₅) | CF₂CHF₂ |
| I.a.103 | C(O)N(CH₃)(C₆H₅) | CH=CCl₂ |
| I.a.104 | C(O)N(CH₃)(C₆H₅) | CH=CF₂ |
| I.a.105 | C(O)N(CH₃)(C₆H₅) | CH₂OH |
| I.a.106 | C(O)N(CH₃)(C₆H₅) | CH₂CH₂OH |
| I.a.107 | C(O)N(CH₃)(C₆H₅) | CH(OH)CH₂OH |
| I.a.108 | C(O)N(CH₃)(C₆H₅) | Cyclopropyl |
| I.a.109 | C(O)N(CH₃)(C₆H₅) | Cyclohexyl |
| I.a.110 | C(Q)N(CH₃)(C₆H₅) | CH₂COOH |
| I.a.111 | C(O)N(CH₃)(C₆H₅) | CH₂O(CO)CH₃ |
| I.a.112 | C(O)N(CH₃)(C₆H₅) | CH₂O(CO)N(CH₃)₂ |
| I.a.113 | C(O)N(CH₃)(C₆H₅) | C≡C(C₆H₅) |
| I.a.114 | C(O)N(CH₃)(C₆H₅) | CH(OH)CH(OH)C₆H₅ |
| I.a.115 | C(O)N(CH₃)(C₆H₅) | CH₂S(2-F-C₆H₄) |
| I.a.116 | SO₂CH₃ | CH₃ |
| I.a.117 | SO₂CH₃ | CH₂CH₃ |
| I.a.118 | SO₂CH₃ | CH=CH₂ |
| I.a.119 | SO₂CH₃ | CH=CHCH₃ |
| I.a.120 | SO₂CH₃ | CH=C(CH₃)₂ |
| I.a.121 | SO₂CH₃ | C≡CH |
| I.a.122 | SO₂CH₃ | CF₃ |
| I.a.123 | SO₂CH₃ | CHF₂ |
| I.a.124 | SO₂CH₃ | CF₂CF₃ |
| I.a.125 | SO₂CH₃ | CF₂CHF₂ |
| I.a.126 | SO₂CH₃ | CH=CCl₂ |
| I.a.127 | SO₂CH₃ | CH=CF₂ |
| I.a.128 | SO₂CH₃ | CH₂OH |
| I.a.129 | SO₂CH₃ | CH₂CH₂OH |
| I.a.130 | SO₂CH₃ | CH(OH)CH₂OH |
| I.a.131 | SO₂CH₃ | Cyclopropyl |
| I.a.132 | SO₂CH₃ | Cyclohexyl |
| I.a.133 | SO₂CH₃ | CH₂COOH |
| I.a.134 | SO₂CH₃ | CH₂O(CO)CH₃ |
| I.a.135 | SO₂CH₃ | CH₂O(CO)N(CH₃)₂ |
| I.a.136 | SO₂CH₃ | C≡C(C₆H₅) |
| I.a.137 | SO₂CH₃ | CH(OH)CH(OH)C₆H₅ |
| I.a.138 | SO₂CH₃ | CH₂S(2-F-C₆H₄) |

Ebenso außerordentlich bevorzugt sind die Verbindungen der Formel I.b, insbesondere die Verbindungen der Formel I.b.1 bis I.b.138, die sich von den entsprechenden Verbindungen der Formel I.a.1 bis I.a.138 dadurch unterscheiden, daß R² für Fluor steht.

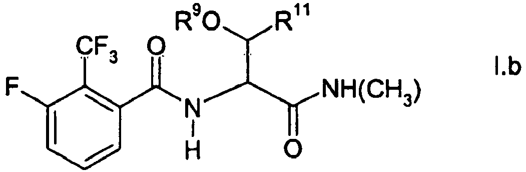

Ebenso außerordentlich bevorzugt sind die Verbindungen der Formel I.c, insbesondere die Verbindungen der Formel I.c.1 bis I.c.138, die sich von den entsprechenden Verbindungen der Formel I.a.1 bis I.a.138 dadurch unterscheiden, daß R³ für Fluor steht.

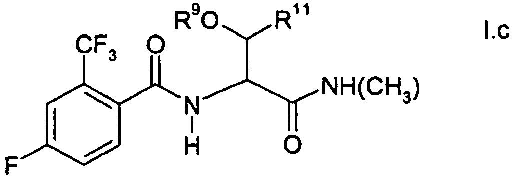

Ebenso außerordentlich bevorzugt sind die Verbindungen der Formel I.d, insbesondere die Verbindungen der Formel I.d.1 bis I.d.138, die sich von den entsprechenden Verbindungen der Formel I.a.1 bis I.a.138 dadurch unterscheiden, daß R⁴ für Fluor steht.

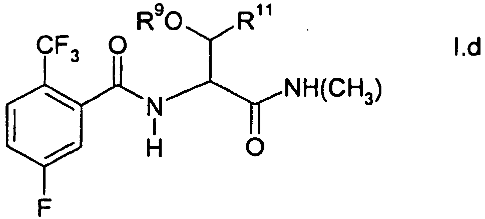

Ebenso außerordentlich bevorzugt sind die Verbindungen der Formel I.e, insbesondere die Verbindungen der Formel I.e.1 bis I.e.138, die sich von den entsprechenden Verbindungen der Formel I.a.1 bis I.a.138 dadurch unterscheiden, daß R² für Chlor steht.

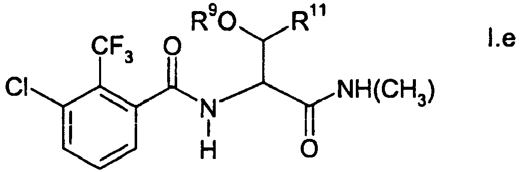

Ebenso außerordentlich bevorzugt sind die Verbindungen der Formel I.f, insbesondere die Verbindungen der Formel I.f.1 bis I.f.138, die sich von den entsprechenden Verbindungen der Formel I.a.1 bis I.a.138 dadurch unterscheiden, daß R³ für Chlor steht.

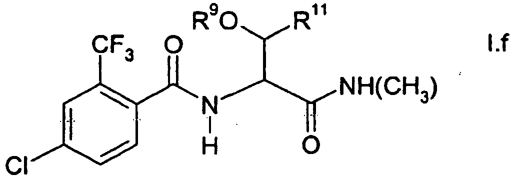

Ebenso außerordentlich bevorzugt sind die Verbindungen der Formel I.g, insbesondere die Verbindungen der Formel I.g.1 bis I.g.138, die sich von den entsprechenden Verbindungen der Formel I.a.1 bis I.a.138 dadurch unterscheiden, daß R³ und R⁴ für Fluor stehen.

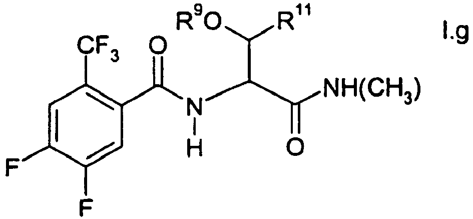

Ebenso außerordentlich bevorzugt sind die Verbindungen der Formel I.h, insbesondere die Verbindungen der Formel I.h.1 bis I.h.138, die sich von den entsprechenden Verbindungen der Formel I.a.1 bis I.a.138 dadurch unterscheiden, daß R¹ für Chlor und R² für CF₃ steht.

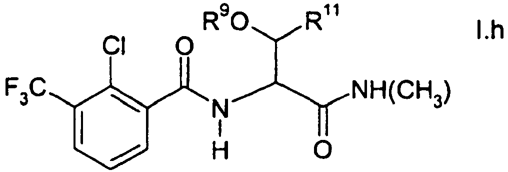

Ebenso außerordentlich bevorzugt sind die Verbindungen der Formel I.j, insbesondere die Verbindungen der Formel I.j.1 bis I.j.138, die sich von den entsprechenden Verbindungen der Formel I.a.1 bis I.a.138 dadurch unterscheiden, daß R¹ und R² für Chlor stehen.

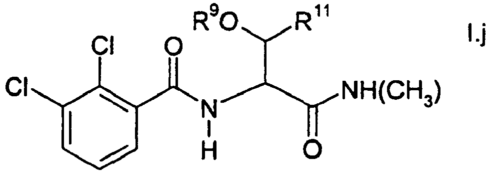

Ebenso außerordentlich bevorzugt sind die Verbindungen der Formel I.k, insbesondere die Verbindungen der Formel I.k.1 bis 1.k.138, die sich von den entsprechenden Verbindungen der Formel I.a.1 bis I.a.138 dadurch unterscheiden, daß R¹ und R³ für Chlor stehen.

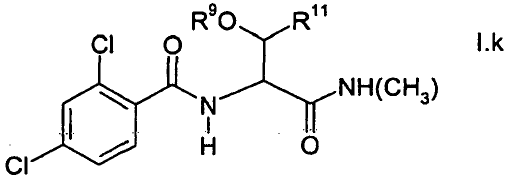

Die benzoylsubstituierten Serin-Amide der Formel I sind auf verschiedene Art und Weise erhältlich, beispielsweise nach folgenden Verfahren:

### Verfahren A

Serinderivate der Formel V werden zunächst mit Benzoesäure(derivate)n der Formel IV zu entsprechenden Benzoylderivaten der Formel 111 umgesetzt, welche anschließend mit Aminen der Formel II zu den gewünschten benzoylsubstituierten Serin-Amiden der Formel I reagieren:

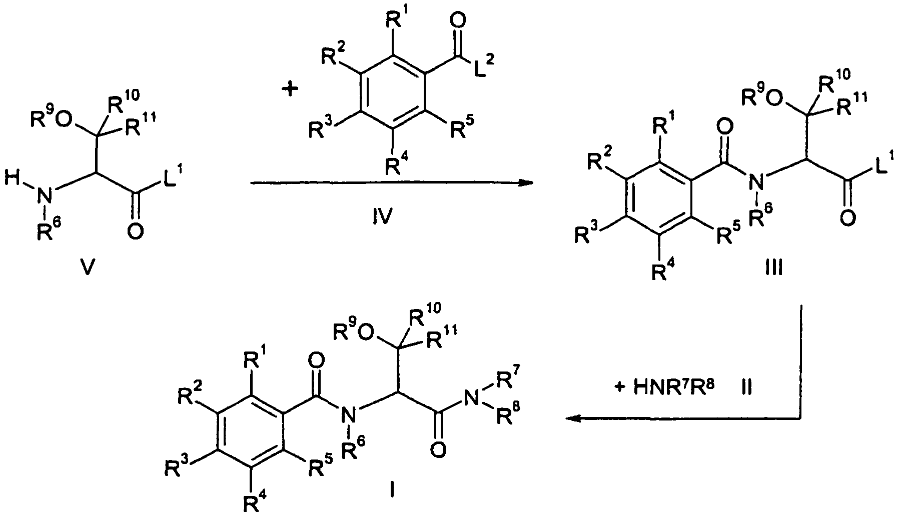

L¹ steht für eine nucleophil verdrängbare Abgangsgruppe, z.B. für Hydroxy oder C₁-C₆-Alkoxy.

L² steht für eine nucleophil verdrängbare Abgangsgruppe, z.B. für Hydroxy, Halogen, C₁-C₆-Alkylcarbonyl, C₁-C₆-Alkoxycarbonyl, C₁-C₄-Alkylsulfonyl, Phosphoryl oder Isoureyl.

Die Umsetzung der Serinderivate der Formel V mit Benzoesäure(derivate)n der Formel IV, wobei L² für Hydroxy steht, zu Benzoylderivaten der Formel III erfolgt in Gegenwart eines Aktivierungsreagenz und einer Base üblicherweise bei Temperaturen von 0°C bis zum Siedepunkt des Reaktionsgemisches, vorzugsweise 0°C bis 110°C, besonders bevorzugt bei Raumtemperatur, in einem inerten organischen Lösungsmittel [vgl. Bergmann, E. D.; et al., J Chem Soc 1951, 2673; Zhdankin, V. V.; et al., Tetrahedron Lett. 2000, 41 (28), 5299-5302; Martin, S. F. et al., Tetrahedron Lett.1998, 39 (12), 1517-1520; Jursic, B. S. et al., Synth Commun 2001, 31 (4), 555-564; Albrecht, M. et al., Synthesis 2001, (3), 468-472; Yadav, L. D. S. et al., Indian J. Chem B. 41(3),593-595(2002); Clark, J. E. et al., Sythesis (10),891-894 (1991)].

Geeignete Aktivierungsreagenzien sind Kondensationsmittel wie z.B. polystyrolgebundenes Dicyclohexylcarbodiimid, Diisopropylcarbodiimid, Carbonyldiimidazol, Chlorkohlensäureester wie Methylchloroformiat, Ethylchloroformiat, Isoropylchloroformiat, Isobutylchloroformiat, *sec*-Butylchloroformiat oder Allylchloroformiat, Pivaloylchlorid, Polyphosphorsäure, Propanphosphonsäureanhydrid, Bis(2-oxo-3-oxazolidinyl)-phosphorylchlorid (BOPCI) oder Sulfonylchloride wie Methansulfonylchlorid, Toluolsulfonylchlorid oder Benzolsulfonylchlorid.

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Benzol, Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran (THF), Nitrile wie Acetonitril und Propionitril, Ketone wie Aceton, Methylethylketon, Diethylketon und tert.-Butylmethylketon, sowie Dimethylsulfoxid, Dimethylformamid (DMF), Dimethylacetamid (DMA) und N-Methylpyrrolidon (NMP) oder auch in Wasser, besonders bevorzugt sind Methylenchlorid, THF und Wasser.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, außerdem organische Basen, z.B. tertiäre Amine wie Trimethylamin, Triethylamin, Diisopropylethylamin, N-Methylmorpholin, und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Triethylamin und Pyridin.

Die Basen werden im allgemeinen in äquimolar Mengen eingesetzt. Sie können aber auch im Überschuß oder gegebenenfalls als Lösungsmittel verwendet werden.

Die Edukte werden im allgemeinen in äquimolaren Mengen miteinander umgesetzt. Es kann vorteilhaft sein, IV in einem Überschuß bezogen auf V einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z. T. in Form zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Umsetzung der Serinderivate der Formel V mit Benzoesäure(derivate)n der Formel IV, wobei L² für Halogen, C₁-C₆-Alkylcarbonyl, C₁-C₆-Alkoxycarbonyl, C₁-C₄-Alkylsulfonyl, Phosphoryl oder Isoureyl steht, zu Benzoylderivaten der Formel III erfolgt in Gegenwart einer Base üblicherweise bei Temperaturen von 0°C bis zum Siedepunkt des Reaktionsgemisches, vorzugsweise 0°C bis 100°C, besonders bevorzugt bei Raumtemperatur in einem inerten organischen Lösungsmittel [vgl. Bergmann, E. D.; et al., J Chem Soc 1951, 2673; Zhdankin, V. V.; et al., Tetrahedron Lett. 2000, 41 (28), 5299-5302; Martin, S. F. et al., Tetrahedron Lett.1998, 39 (12), 1517-1520; Jursic, B. S. et al., Synth Commun 2001, 31 (4), 555-564; Albrecht, M. et al., Synthesis 2001, (3), 468-472; Yadav, L. D. S. et al., Indian J. Chem B. 41(3),593-595(2002); Clark, J. E. et al., Synthesis (10),891-894 (1991)].

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Benzol, Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran (THF), Nitrile wie Acetonitril und Propionitril, Ketone wie Aceton, Methylethylketon, Diethylketon und tert.-Butylmethylketon, sowie Dimethylsulfoxid, Dimethylformamid (DMF), Dimethylacetamid (DMA) und N-Methylpyrrolidon (NMP) oder auch in Wasser, besonders bevorzugt sind Methylenchlorid, THF und Wasser.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, außerdem organische Basen, z.B. tertiäre Amine wie Trimethylamin, Triethylamin, Diisopropylethylamin, N-Methylmorpholin, und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Triethylamin und Pyridin.

Die Basen werden im allgemeinen in äquimolar Mengen eingesetzt. Sie können aber auch im Überschuß oder gegebenenfalls als Lösungsmittel verwendet werden.

Die Edukte werden im allgemeinen in äquimolaren Mengen miteinander umgesetzt. Es kann vorteilhaft sein, IV in einem Überschuß bezogen auf V einzusetzen.

Die Aufarbeitung und Isolierung der Produkte kann in an sich bekannter Weise erfolgen.

Natürlich können auch in analoger Weise zunächst die Serinderivate der Formel V mit Aminen der Formel II zu den entsprechenden Amiden umgesetzt werden, welche dann mit Benzoesäure(derivate)n der Formel IV zu den gewünschten benzoylsubstituierten Serin-Amiden der Formel I reagieren.

Die für die Herstellung der Benzoylderivate der Formel III benötigten Serinderivate der Formel V (z.B. mit L¹ = Hydroxy oder C₁-C₆-Alkoxy) sind, auch in enantiomeren- und diastereomerenreiner Form, in der Literatur bekannt oder können gemäß der zitierten Literatur hergestellt werden:
- durch Kondensation von Glycinenolat-Equivalenten mit Aldehyden oder Ketonen [Blaser, D. et al., Liebigs Ann. Chem. 10, 1067-1078 (1991); Seethaler, T. et al., Liebigs Ann. Chem. 1, 11-17 (1991); Weltenauer, G. et al., Gazz. Chim. Ital. 81, 162 (1951); Dalla Croce, P. et al., Heterocycles 52(3), 1337-1344 (2000); Van der Werf, A. W. et al., J. Chem. Soc. Chem. Commun. 100, 682-683 (1991); Caddick, S. et al., Tetrahedron 57 (30), 6615-6626 (2001); Owa, T. et al., Chem. Lett. 1, 83-86 (1988); Alker, D. et al., Tetrahedron 54 (22), 6089-6098 (1998); Rousseau, J. F. et al., J. Org. Chem. 63 (8), 2731-2737 (1998); Saeed, A. et al., Tetrahedron 48 (12), 2507-2514 (1992); Dong, L. et al., J. Org. Chem. 67 (14), 4759-4770 (2002)].
- durch Aminohydroxylierung von Acrylsäure-Derivaten [Zhang, H. X. et al., Tetrahedron Asymmetr. 11(16), 3439-3447 (2000); Fokin, V. V. et al., Angew. Chem. Int. Edit. 40(18), 3455 (2001); Sugiyama, H. et al., Tetrahedron Lett. 43(19), 3489-3492 (2002); Bushey, M. L. et al., J. Org. Chem. 64(9), 2984-2985 (1999); Raatz, D. et al., Synlett (12), 1907-1910 (1999)].
- durch nukleophile Substitution von Abgangsgruppen in 2-Position von 3-Hydroxy-Propionsäurederivaten [Owa, T. et al., Chem. Lett. (11), 1873-1874 (1988); Boger, D. L. et al., J. Org. Chem. 57(16), 4331-4333 (1992); Alcaide, B. et al., Tetrahedron Lett. 36(30), 5417-5420 (1995)].
- durch Kondensation von Aldehyden mit Nukleophilen unter Ausbildung von Oxazolinen sowie anschließender Hydrolyse [Evans, D. A. et al., Angew. Chem. Int. Edit. 40(10), 1884-1888 (2001); Ito, Y. et al., Tetrahedron Lett. 26(47), 5781-5784 (1985); Togni, A. et al., J. Organomet. Chem. 381(1), C21-5 (1990); Longmire, J. M. et al., Organometallics 17(20), 4374-4379 (1998); Suga, H. et al., J. Org. Chem. 58(26), 7397-7405 (1993)].
- durch oxidative Cyclisierung von 2-Acylamino-Propionsäurederivaten zu Oxazolinen sowie anschließende Hydrolyse (JP10101655).
- durch Diels-Alder-Reaktion von Vinyliminen mit Aldehyden zu Oxazinen und anschließende Hydrolyse [Bongini, A. et al., Tetrahedron Asym. 12(3), 439-454 (2001)].

Die für die Herstellung der Benzoylderivate der Formel III benötigten Benzoesäure-(derivate) der Formel IV können käuflich erworben werden oder können analog zu literaturbekannten Vorschrift über eine Grignard-Reaktion aus dem entsprechenden Halogenid hergestellt werden [z.B. A. Mannschuk et al., Angew. Chem. 100, 299 (1988)].

Die Umsetzung der Benzoylderivate der Formel III mit L¹ = Hydroxy bzw. deren Salze mit Aminen der Formel II zu den gewünschten benzoylsubstituierten Serin-Amiden der Formel I erfolgt in Gegenwart eines Aktivierungsreagenz und gegebenenfalls in Gegenwart einer Base üblicherweise bei Temperaturen von 0°C bis zum Siedepunkt des Reaktionsgemisches, vorzugsweise 0°C bis 100°C, besonders bevorzugt bei Raumtemperatur in einem inerten organischen Lösungsmittel. [vgl. Perich, J. W., Johns, R. B., J. Org. Chem. 53 (17), 4103-4105 (1988); Somlai, C. et al., Synthesis (3), 285-287 (1992) ; Gupta, A. et al., J. Chem. Soc. Perkin Trans. 2, 1911 (1990); Guan et al., J. Comb. Chem. 2, 297 (2000)].

Geeignete Aktivierungsreagenzien sind Kondensationsmittel wie z.B. polystyrolgebundenes Dicyclohexylcarbodiimid, Diisopropylcarbodiimid, Carbonyldiimidazol, Chlorkohlensäureester wie Methylchloroformiat, Ethylchloroformiat, Isoropylchloroformiat, Isobutylchloroformiat, *sec*-Butylchloroformiat oder Allylchloroformiat, Pivaloylchlorid, Polyphosphorsäure, Propanphosphonsäureanhydrid, Bis(2-oxo-3-oxazolidinyl)-phosphorylchlorid (BOPCI) oder Sulfonylchloride wie Methansulfonylchlorid, Toluolsulfonylchlorid oder Benzolsulfonylchlorid.

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Benzol, Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran (THF), Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid (DMF), Dimethylacetamid (DMA) und N-Methylpyrrolidon (NMP) oder auch in Wasser, besonders bevorzugt sind Methylenchlorid, THF, Methanol, Ethanol und Wasser.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, außerdem organische Basen, z.B. tertiäre Amine wie Trimethylamin, Triethylamin, Diisopropylethylamin, N-Methylmorpholin, und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Triethylamin, Ethyldiisopropylamin, N-methylmorpholin und Pyridin.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann vorteilhaft sein II in einem Überschuß bezogen auf III einzusetzen.

Die Aufarbeitung und Isolierung der Produkte kann in an sich bekannter Weise erfolgen.

Die Umsetzung der Benzoylderivate der Formel III mit L¹ = C₁-C₆-Alkoxy mit Aminen der Formel II zu den gewünschten benzoylsubstituierten Serin-Amiden der Formel I erfolgt üblicherweise bei Temperaturen von 0°C bis zum Siedepunkt des Reaktionsgemisches, vorzugsweise 0°C bis 100°C, besonders bevorzugt bei Raumtemperatur in einem inerten organischen Lösungsmittel gegebenenfalls in Gegenwart einer Base [vgl. Kawahata, N. H. et al., Tetrahedron Lett. 43 (40), 7221-7223 (2002); Takahashi, K. et al., J. Org. Chem. 50 (18), 3414-3415 (1985); Lee, Y. et al., J. Am. Chem. Soc. 121 (36), 8407-8408 (1999)].

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Benzol, Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran (THF), Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid (DMF), Dimethylacetamid (DMA) und N-Methylpyrrolidon (NMP) oder auch in Wasser, besonders bevorzugt sind Methylenchlorid, THF, Methanol, Ethanol und Wasser.

Es können auch Gemische der genannten Lösungsmittel verwendet werden.

Die Umsetzung kann gegebenenfalls in Gegenwart einer Base erfolgen. Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, außerdem organische Basen, z.B. tertiäre Amine wie Trimethylamin, Triethylamin, Diisopropylethylamin, N-Methylmorpholin, und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Triethylamin, Ethyldiisopropylamin, N-methylmorpholin und Pyridin.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann vorteilhaft sein, II in einem Überschuß bezogen auf III einzusetzen.

Die Aufarbeitung und Isolierung der Produkte kann in an sich bekannter Weise erfolgen.

Die für die Herstellung der benzoylsubstituierten Serin-Amide der Formel I benötigten Amine der Formel II können käuflich erworben werden.

### Verfahren B

Benzoylderivate der Formel III mit R⁹ = Wasserstoff können auch erhalten werden, indem acylierte Glycin-Derivate der Formel VIII, wobei die Acylgruppe eine abspaltbare Schutzgruppe wie Benzyloxycarbonyl (vgl. VIIIa mit Σ = Benzyl) oder tert.-Butyloxycarbonyl (vgl. VIIIa mit Σ = tert-Butyl) sein kann, mit Carbonylverbindungen VII zu entsprechenden Aldolprodukten VI kondensiert wird. Anschließend wird die Schutzgruppe abgespalten und die so entstandenen Serinderivate der Formel V mit R⁹ = Wasserstoff mit Benzoesäure(derivate)n der Formel IV acyliert.

Analog kann auch ein acyliertes Glycin-Derivat der Formel VIII, wobei die Acylgruppe ein substituierter Benzoylrest (vgl. VIIIb) ist, unter Baseneinfluß mit einer Carbonylverbindung VII zum Benzoylderivat III mit R⁹ = Wasserstoff umgesetzt werden:

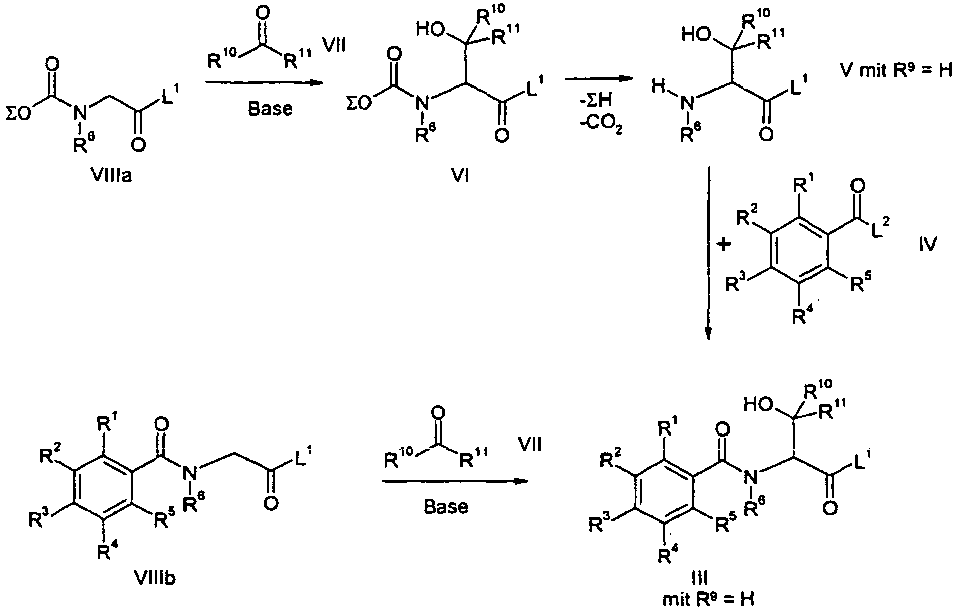

L¹ steht für eine nucleophil verdrängbare Abgangsgruppe, z.B. für Hydroxy oder C₁-C₆-Alkoxy.

L² steht für eine nucleophil verdrängbare Abgangsgruppe, z.B. für Hydroxy, Halogen, C₁-C₆-Alkylcarbonyl, C₁-C₆-Alkoxycarbonyl, C₁-C₄-Alkylsulfonyl, Phosphoryl oder Isoureyl.

Die Umsetzung der Glycinderivate VIII mit Carbonylerbindungen VII zum entsprechenden Aldolprodukt VI bzw. Benzoylderivat III mit R⁹ = Wasserstoff erfolgt üblicherweise bei Temperaturen von -100°C bis zum Siedepunkt der Reaktionsmischung, bevorzugt - 80°C bis 20°C, insbesondere bevorzugt -80°C bis -20°C, in einem inerten organischen Lösungsmittel in Gegenwart einer Base [vgl. J.-F. Rousseau et al., J. Org. Chem. 63, 2731-2737 (1998)].

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, Ether wie Diethylether, Diisopropylether, tert.-Butylmethylether, Dioxan, Anisol und Tetrahydrofuran, sowie Dimethylsulfoxid, Dimethylformamid und Dimethylacetamid, besonders bevorzugt Diethylether, Dioxan und Tetrahydrofuran.

Es können auch Gemische der genannten Lösungsmittel verwendet werden.

Als Basen kommen allgemein anorganische Verbindungen wie Alkalimetall- und Erdalkalimetallhydride wie Lithiumhydrid, Natriumhydrid, Kaliumhydrid und Calciumhydrid, Alkalimetallamide wie Lithiumdiisopropylamid und Lithiumhexamethyldisilazid, metallorganische Verbindungen, insbesondere Alkalimetallalkyle wie Methyllithium, Butyllithium und Phenyllithium, sowie Alkalimetall- und Erdalkalimetallalkoholate wie Natriummethanolat, Natriumethanolat, Kaliumethanolat, Kalium- tert.-Butanolat, Kalium-tert.-Pentanolat und Dimethoxymagnesium, außerdem organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriumhydrid, Lithiumhexamethyldisilazid und Lithiumdiisopropylamid.

Die Basen werden im allgemeinen in äquimolaren Mengen eingesetzt, sie können aber auch katalytisch, im Überschuß oder gegebenenfalls als Lösungsmittel verwendet werden.

Die Edukte werden im allgemeinen in äquimolaren Mengen miteinander umgesetzt. Es kann vorteilhaft sein, die Base und/oder die Carbonylverbindungen VII in einem Überschuß bezogen auf die Glycinderivate VIII einzusetzen.

Die Aufarbeitung und Isolierung der Produkte kann in an sich bekannter Weise erfolgen.

Die für die Herstellung der Verbindungen I benötigten Glycinderivate der Formel VIII können käuflich erworben werden, sind in der Literatur bekannt [z.B. H. Pessoa-Mahana et al., Synth. Comm. 32, 1437 (2002] oder können gemäß der zitierten Literatur hergestellt werden.

Die Abspaltung der Schutzgruppe zu Serinderivaten der Formel V mit R⁹ = Wasserstoff erfolgt nach literaturbekannten Methoden [vgl. J.-F. Rousseau et al., J. Org. Chem. 63, 2731-2737 (1998) ); J. M. Andres, Tetrahedron 56, 1523 (2000)];
im Fall von Σ = Benzyl durch Hydrogenolyse, bevorzugt durch Wasserstoff und Pd/C in Methanol; im Fall von Σ = tert.-Butyl durch Säure, bevorzugt Salzsäure in Dioxan.

Die Umsetzung der Serinderivate V mit R⁹= Wasserstoff mit Benzoesäure(derivate)n IV zu Benzoylderivaten III mit R⁹ = Wasserstoff erfolgt üblicherweise analog der unter Verfahren A genannten Umsetzung der Serinderivate der Formel V mit Benzoesäure(derivate)n der Formel IV zu Benzoylderivaten III.

Die Benzoylderivate der Formel III mit R⁹ = Wasserstoff lassen sich anschließend mit Aminen der Formel II analog zu Verfahren A zu den gewünschten benzoylsubstituierten Serin-Amiden der Formel I mit R⁹ = Wasserstoff umsetzen, welche dann mit Verbindungen der Formel IX zu benzoylsubstituierten Serin-Amiden der Formel I derivatisiert werden können [vgl. z.B. Yokokawa, F. et al., Tetrahedron Lett. 42 (34), 5903-5908 (2001); Arrault, A. et al., Tetrahedron Lett. 43(22), 4041-4044 (2002)];

ebenso können die Benzoylderivate der Formel III mit R⁹ = Wasserstoff zunächst mit Verbindungen der Formel IX zu weiteren Benzoylderivaten der Formel III derivatisiert werden [vgl. z.B. Troast, D. et al., Org. Lett. 4 (6), 991-994 (2002); Ewing W. et al., Tetrahedron Lett., 30 (29), 3757-3760 (1989); Paulsen, H. et al., Liebigs Ann. Chem. 565 (1987)] und anschließend analog zu Verfahren A mit Aminen der Formel II zu den gewünschten benzoylsubstituierten Serin-Amiden der Formel I umgesetzt werden:

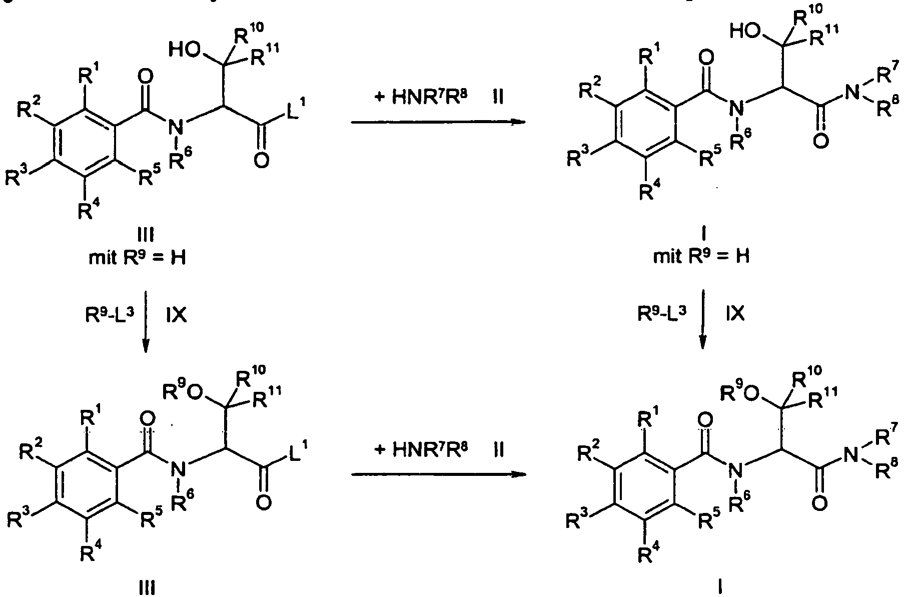

L¹ steht für eine nucleophil verdrängbare Abgangsgruppe, z.B. für Hydroxy oder C₁-C₆-Alkoxy.

L³ steht für eine nucleophil verdrängbare Abgangsgruppe, z.B. für Halogen, Hydroxy, oder C₁-C₆-Alkoxy.

Die Umsetzung der Benzoylderivate der Formel III (gegebenenfalls mit R⁹ = Wasserstoff) mit Aminen der Formel II zu benzoylsubstituierten Serin-Amiden der Formel I (gegebenenfalls mit R⁹ = Wasserstoff) erfolgt üblicherweise analog der unter Verfahren A geschilderten Umsetzung der Benzoylderivate der Formel III mit Aminen der Formel II.

Die Umsetzung der Benzoylderivate der Formel III mit R⁹ = Wasserstoff bzw. der benzoylsubstituierten Serin-Amide der Formel I mit R⁹ = Wasserstoff mit Verbindungen der Formel IX zu Benzoylderivaten der Formel III bzw. benzoylsubstituierten Serin-Amiden der Formel I erfolgt üblicherweise bei Temperaturen von 0°C bis 100°C, vorzugsweise 10°C bis 50°C, in einem inerten organischen Lösungsmittel in Gegenwart einer Base [vgl. z.B. Troast, D. et al., Org. Lett. 4 (6), 991-994 (2002); Ewing W. et al., Tetrahedron Lett., 30 (29), 3757-3760 (1989); Paulsen, H. et al., Liebigs Ann. Chem. 565 (1987)].

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt Dichlormethan, tert.-Butylmethylether, Dioxan und Tetrahydrofuran.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall und Erdalkalimetallcarbonate wie Lithiumcarbonat, Kaliumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat, Kalium-tert.-Pentanolat und Dimethoxymagnesium, außerdem organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Natriumhydrid und Triethylamin.

Die Basen werden im allgemeinen in äquimolaren Mengen eingesetzt, sie können aber auchkatalytisch, im Überschuß oder gegebenenfalls als Lösungsmittel verwendet werden.

Die Edukte werden im allgemeinen in äquimolaren Mengen miteinander umgesetzt. Es kann vorteilhaft sein, die Base und/oder IX in einem Überschuß bezogen auf III bzw. I einzusetzen.

Die Aufarbeitung und Isolierung der Produkte kann in an sich bekannter Weise erfolgen.

Die benötigten Verbindungen der Formel VIII können käuflich erworben werden.

### Verfahren C

Benzoylderivate der Formel III mit R⁹ = Wasserstoff können auch erhalten werden, indem Aminomalonyl-Verbindungen der Formel XI zunächst mit Benzoesäure(derivate)n der Formel IV zu entsprechenden N-Acyl-Aminomalonyl-Verbindungen der Formel X acyliert werden und anschließend mit einer Carbonylverbindung der Formel VII unter Decarboxylierung kondensiert werden:

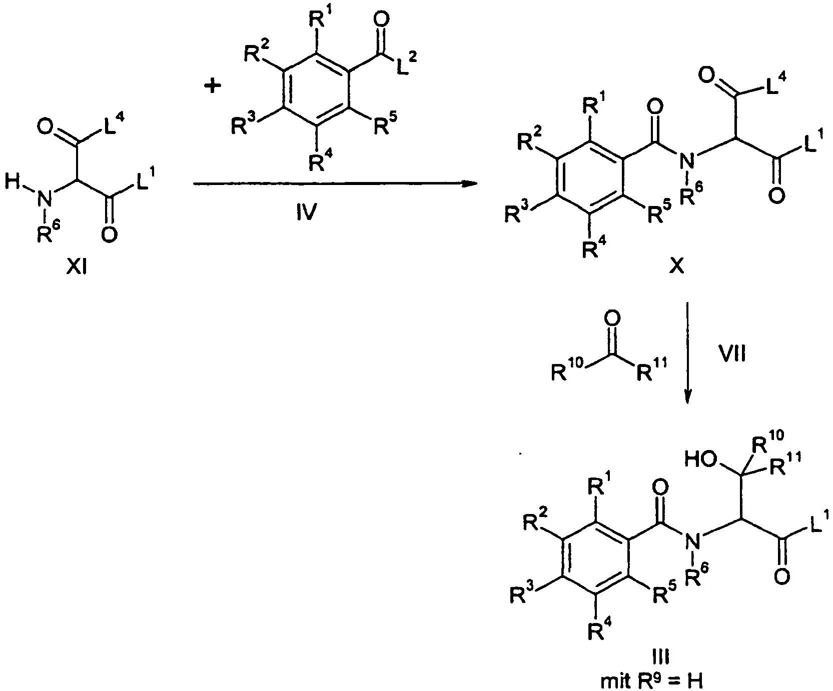

L¹ steht für eine nucleophil verdrängbare Abgangsgruppe, z.B. für Hydroxy oder C₁-C₆-Alkoxy.

L² steht für eine nucleophil verdrängbare Abgangsgruppe, z.B. für Hydroxy, Halogen, C₁-C₆-Alkylcarbonyl, C₁-C₆-Alkoxycarbonyl, C₁-C₆-Alkylsulfonyl, Phosphoryl oder Isoureyl.

L⁴ steht für eine nucleophil verdrängbare Abgangsgruppe, z.B. für Hydroxy oder C₁-C₆-Alkoxy.

Die Acylierung der Aminomalonyl-Verbindungen der Formel XI mit Benzoesäure(derivate)n der Formel IV zu entsprechenden N-Acyl-Aminomalonyl-Verbindungen der Formel X erfolgt üblicherweise analog der unter Verfahren A genannten Umsetzung der Serinderivate der Formel V mit Benzoesäure(derivate)n der Formel IV zu den entsprechenden Benzoylderivaten der Formel III.

Die Umsetzung der N-Acyl-Aminomalonyl-Verbindungen der Formel X mit Carbonylverbindungen der Formel VII zu Benzoylderivaten der Formel III mit R⁹ = Wasserstoff erfolgt üblicherweise bei Temperaturen von 0°C bis 100°C, vorzugsweise 10°C bis 50°C, in einem inerten organischen Lösungsmittel in Gegenwart einer Base [vgl. z.B. US 4904674; Hermann, H. et al., Liebigs Ann. Chem. 631, 175-179 (1960)]

Falls L⁴ bei den N-Acyl-Aminomalonyl-Verbindungen der Formel X für C₁-C₆-Akoxy steht, ist es von Vorteil, L⁴ zunächst durch Esterverseifung [z.B. Hellmann, H. et al., Liebigs Ann. Chem. 631, 175-179 (1960)] in eine Hydroxygruppe zu überführen.

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid,besonders bevorzugt Diethylether, Dioxan und Tetrahydrofuran.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat, Kalium-tert.-Pentanolat und Dimethoxymagnesium, außerdem organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Triethylamin und Diisopropylethylamin.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann vorteilhaft sein, die Base in einem Überschuß bezogen auf X einzusetzen.

Die Aufarbeitung und Isolierung der Produkte kann in an sich bekannter Weise erfolgen.

Die so erhaltenen Benzoylderivate der Formel III mit R⁹ = Wasserstoff können anschließend gemäß den voranstehend genannten Verfahren A bzw. B zu den gewünschten benzoylsubstituierten Serin-Amiden der Formel I umgesetzt werden.

Die benötigten Aminomalonyl-Verbindungen der Formel XI können käuflich erworben werden bzw. sind in der Literatur bekannt [z.B. US 4904674; Hellmann, H. et al., Liebigs Ann. Chem. 631, 175-179 (1960)] oder können gemäß der zitierten Literatur hergestellt werden.

Die benötigten Carbonylverbindungen der Formel VII können käuflich erworben werden.

### Verfahren D

Benzoylderivate der Formel III mit R⁹ und R¹⁰ = Wasserstoff können auch erhalten werden, indem Ketoverbindungen der Formel XIII zunächst mit Benzoesäure(derivate)n der Formel IV zu entsprechenden N-Acyl-Ketoverbindungen der Formel XII acyliert werden und anschließend die Ketogruppe reduziert wird [Girard A, Tetrahedron Lett. 37(44),7967-7970(1996); Nojori R., J. Am. Chem. Soc. 111(25),9134-9135(1989); Schmidt U., Synthesis (12),1248-1254 (1992); Bolhofer, A.; J. Am. Chem. Soc. 75, 4469 (1953)]:

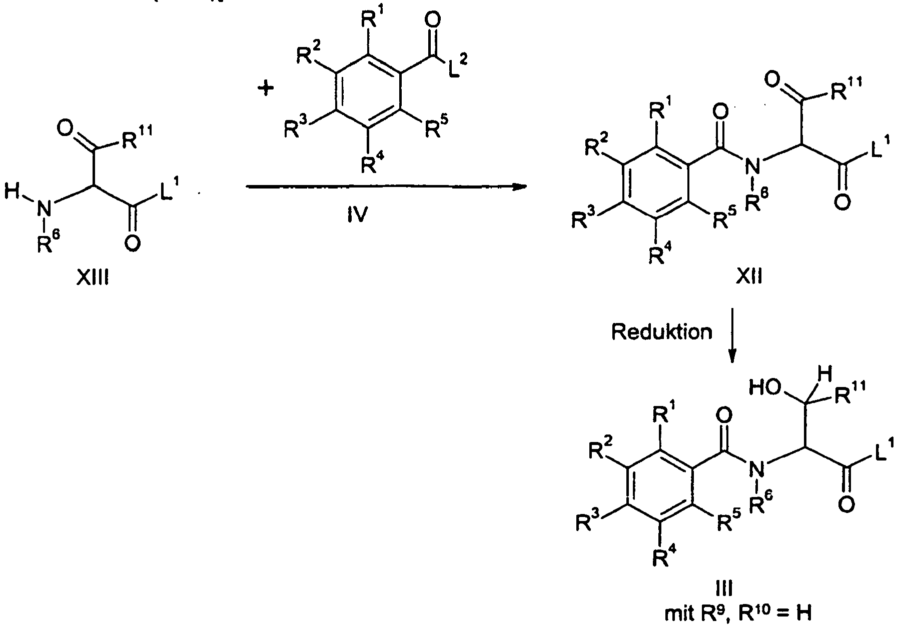

L¹ steht für eine nucleophil verdrängbare Abgangsgruppe, z.B. für Hydroxy oder C₁-C₆-Alkoxy.

L² steht für eine nucleophil verdrängbare Abgangsgruppe, z.B. für Hydroxy, Halogen, C₁-C₆-Alkylcarbonyl, C₁-C₆-Alkoxycarbonyl, C₁-C₆-Alkylsulfonyl, Phosphoryl oder Isoureyl.

Die Acylierung der Ketoverbindungen der Formel XIII mit Benzoesäure(derivate)n der Formel IV zu N-Acyl-Ketoverbindungen der Formel XII erfolgt üblicherweise analog der unter Verfahren A genannten Umsetzung der Serinderivate der Formel V mit Benzoesäure(derivate)n der Formel IV zu den entsprechenden Benzoylderivaten der Formel III.

Die für die Herstellung der Benzoylderivate der Formel III mit R⁹ und R¹⁰ = Wasserstoff benötigten Ketoverbindungen der Formel XIII sind in der Literatur bekannt [WO 02/083111; Boto, A. et al., Tetrahedron Letters 39 (44), 8167-8170 (1988); von Geldern, T. et al., J. of Med. Chem. 39(4), 957-967 (1996); Singh, J. et al., TetrahedronLetters 34 (2), 211-214 (1993); ES 2021557; Maeda, S: et al., Chem. & Pharm. Bull. 32 (7), 2536-2543 (1984); Ito, S. et al., J. of Biol. Chem. 256 (15), 7834-4783 (1981); Vinograd, L. et al., Zhurnal Organicheskoi Khimii 16 (12), 2594-2599 (1980); Castro, A. et al., J. Org. Chem. 35 (8), 2815-2816 (1970); JP 02-172956; Suzuki, M. et al., J. Org. Chem. 38 (20), 3571-3575 (1973) ; Suzuki, M. et al, Synthetic Communications 2 (4), 237-242 (1972)] oder können gemäß der zitierten Literatur hergestellt werden.

Die Reduktion der N-Acyl-Ketoverbindungen der Formel XII zu Benzoylderivaten der Formel III mit R⁹ und R¹⁰ = Wasserstoff erfolgt üblicherweise bei Temperaturen von 0°C bis 100°C, vorzugsweise 20°C bis 80°C, in einem inerten organischen Lösungsmittel in Gegenwart eines Reduktionsmittels.

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt Toluol, Methylenchlorid oder tert.-Butylmethylether.

Es können auch Gemische der genannten Lösungsmittel verwendet werden.

Als Reduktionsmittel eignen sich z.B. Natriumborhydrid, Zinkborhydrid, Natriumcyanoborhydrid, Litium-triethylborhydrid (Superhydrid®), Litium-tri-sec.butylbohydrid (L-Selectrid®), Litiumaluminiumhydrid oder Boran [vgl. z.B. WO 00/20424; Marchi, C. et al.., Tetrahedron 58(28), 5699-(2002); Blank, S. et al., Liebigs Ann. Chem. (8), 889-896 (1993); Kuwano, R. et al., J. Org. Chem. 63 (10), 3499-3503 (1998); Clariana, J. et al., Tetrahedron 55 (23), 7331-7344 (1999)].

Weiterhin kann die Reduktion auch in Gegenwart von Wasserstoff und eines Katalysator erfolgen. Als Katalysatoren eignen sich z.B. [Ru(BINAP)Cl₂] oder Pd/C [vgl. Noyori, R. et al., J. Am. Chem. Soc. 111 (25), 9134-9135 (1989); Bolhofer, A. et al., J. Am. Chem. Soc. 75, 4469 (1953)].

Daneben kann die Reduktion auch Gegenwart eines Mikroorganismus erfolgen. Als Mikroorganismus eignet sich z.B. *Saccharomyces Rouxii*[vgl. Soukup, M. et al., Helv. Chim. Acta 70, 232 (1987)].

Die N-Acyl-Ketoverbindungen der Formel XII und das jeweilige Reduktionsmittel werden im allgemeinen in äquimolaren Mengen miteinander umgesetzt. Es kann vorteilhaft sein, das Reduktionsmittel in einem Überschuß bezogen auf XII einzusetzen.

Die Aufarbeitung und Isolierung der Produkte kann in an sich bekannter Weise erfolgen.

Die so erhaltenen Benzoylderivate der Formel III mit R⁹ und R¹⁰ = Wasserstoff können anschließend gemäß den voranstehend genannten Verfahren A und B zu den gewünschten benzoylsubstituierten Serin-Amiden der Formel I umgesetzt werden.

### Verfahren E

Benzoylderivate der Formel III mit R⁹ = Wasserstoff und R¹¹ = -C(OH)R'R" können auch erhalten werden, indem Vinylglycine der Formel XIV mit einem Oxidationsmittel wie Osmiumtetroxid oder Permanganat dihydroxyliert werden:

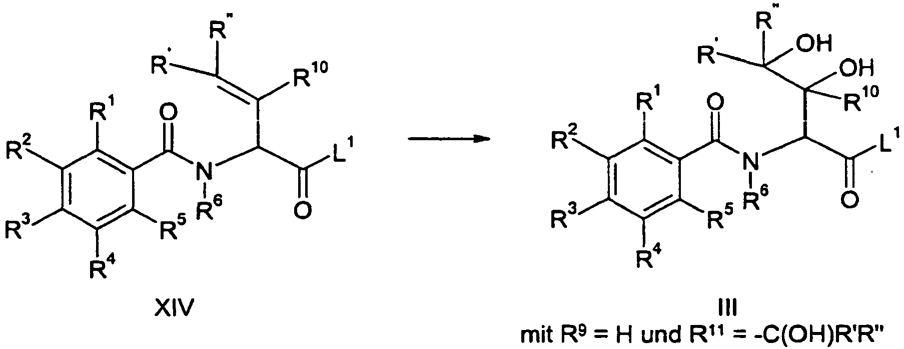

L¹ steht für eine nucleophil verdrängbare Abgangsgruppe, z.B. für Hydroxy oder C₁-C₆-Alkoxy.

R' steht für Wasserstoff, C₁-C₆-Alkyl, C₁-C₆-Halogenalkyl, C₁-C₆-Hydroxyalkyl, Phenyl oder C₁-C₆-Alkoxycarbonyl.

R" steht für Wasserstoff, C₁-C₆-Alky), C₁-C₆-Halogenalkyl, C₁-C₆-Hydroxyalkyl, Phenyl oder C₁-C₆-Alkoxycarbonyl.

Diese Umsetzung erfolgt üblicherweise bei Temperaturen von -78°C bis zum Siedepunkt der Reaktionsmischung, bevorzugt -10°C bis 120°C, insbesondere bevorzugt 0°C bis 50°C, in einem inerten organischen Lösungsmittel in gegebenenfalls in Gegenwart eines Reoxidationsmittels wie z.B. N-Methylmorpholin-N-oxid (D. Johnson et al., Tetrahedron 2000, 56, 5, 781).

Geeignete Lösungsmittel sind halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid, Dimethylacetamid und Wasser; besonders bevorzugt Aceton oder Wasser.

Es können auch Gemische der genannten Lösungsmittel verwendet werden.

Die Edukte werden im allgemeinen in äquimolaren Mengen miteinander umgesetzt. Es kann vorteilhaft sein, das Oxidationsmittel in einem Überschuß bezogen auf XIV einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die für die Herstellung der Benzoylderivate der Formel III mit R⁹ = Wasserstoff und R¹¹ = -C(OH)R'R" benötigten Vinylglycine der Formel XIV sind in der Literatur bekannt [D. B. Berkowitz et al., J. Org. Chem. 2000, 65, 10, 2907; M. Koen et al., J. Chem. Soc. Perkin I 1997, 4, 487] oder können gemäß der zitierten Literatur hergestellt werden.

Die Benzoylderivate der Formel III mit R⁹ = Wasserstoff und R¹¹ = -C(OH)R'R" lassen sich anschließend mit Aminen der Formel II analog zu Verfahren A zu den gewünschten benzoylsubstituierten Serin-Amiden der Formel I mit R⁹ = Wasserstoff und R¹¹ = - C(OH)R'R" umsetzen, welche dann mit Verbindungen der Formel IX zu benzoylsubstituierten Serin-Amiden der Formel I mit R¹¹ = -C(OR⁹)R'R" derivatisiert werden können [vgl. z.B. Yokokawa, F. et al., Tetrahedron Lett. 42 (34), 5903-5908 (2001); Arrault, A. et al., Tetrahedron Lett. 43( 22), 4041-4044 (2002)];

ebenso können die Benzoylderivate der Formel III mit R⁹ = Wasserstoff zunächst mit Verbindungen der Formel IX zu weiteren Benzoylderivaten der Formel III mit R¹¹ = - C(OR⁹)R'R" analog Verfahren B derivatisiert werden [vgl. z.B. Troast, D. et al., Org. Lett. 4 (6), 991-994 (2002); Ewing W. et al., Tetrahedron Lett., 30 (29), 3757-3760 (1989); Paulsen, H. et al., Liebigs Ann. Chem. 565 (1987)] und anschließend analog zu Verfahren A mit Aminen der Formel II zu den gewünschten benzoylsubstituierten Serin-Amiden der Formel mit R¹¹ = -C(OR⁹)R'R" umgesetzt werden:

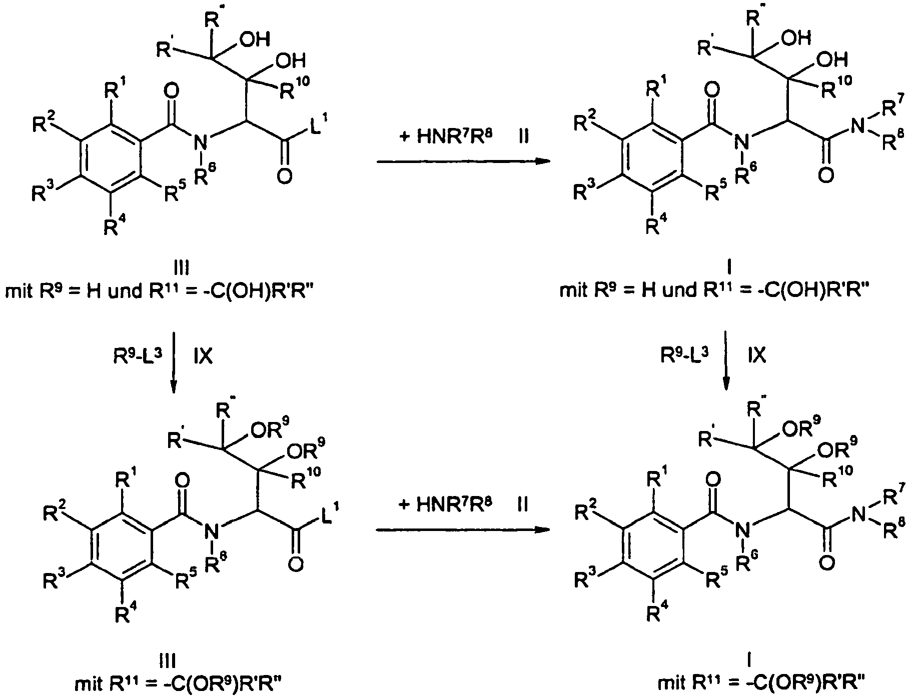

L¹ steht für eine nucleophil verdrängbare Abgangsgruppe, z.B. für Hydroxy oder C₁-C₆-Alkoxy.
L³ steht für eine nucleophil verdrängbare Abgangsgruppe, z.B. für Halogen, Hydroxy, oder C₁-C₆-Alkoxy.
R' steht für Wasserstoff, C₁-C₆-Alkyl, C₁-C₆-Halogenalkyl, C₁-C₆-Hydroxyalkyl, Phenyl oder C₁-C₆-Alkoxycarbonyl.
R" steht für Wasserstoff, C₁-C₆-Alkyl, C₁-C₆-Halogenalkyl, C₁-C₆-Hydroxyalkyl, Phenyl oder C₁-C₆-Alkoxycarbonyl.

### Verfahren F

Benzoylderivate der Formel III mit R⁹ = Wasserstoff und R¹¹ = -C(Nuc)R'R" können auch erhalten werden, indem Vinylglycine der Formel XIV mit einem Epoxidierunsreagenz zu Epoxyglycinen der Formel XV epoxydiert werden und anschließend eine nucleophile Epoxidöffnung erfolgt:

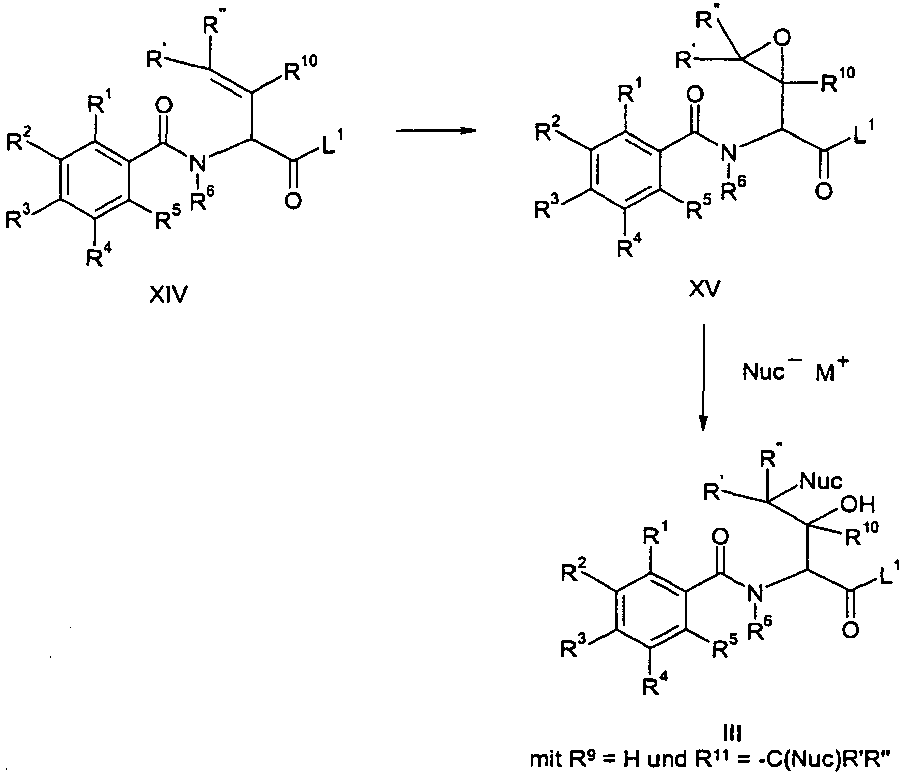

L¹ steht für eine nucleophil verdrängbare Abgangsgruppe, z.B. für Hydroxy oder C₁-C₆-Alkoxy.

R' steht für Wasserstoff, C₁-C₆-Alkyl, C₁-C₆-Halogenalkyl, C₁-C₆-Hydroxyalkyl, Phenyl oder C₁-C₆-Alkoxycarbonyl.

R¹¹ steht für Wasserstoff, C₁-C₆-Alkyl, C₁-C₆-Halogenalkyl, C₁-C₆-Hydroxyalkyl, Phenyl oder C₁-C₆-Alkoxycarbonyl.

Nuc-M⁺ steht beispielsweise für Thiolate wie z.B. Natriumthiophenolat, Alkoholate wie Kaliumphenolat, oder Amide wie Natriumimidazolat.

Die Epoxidierung erfolgt üblicherweise bei Temperaturen von -78°C bis zum Siedepunkt der Reaktionsmischung, bevorzugt-20°C bis 50°C, insbesondere bevorzugt 0°C bis 30°C, in einem inerten organischen Lösungsmittel [vgl. P. Meffre et al., Tetrahedron Lett. 1990, 31, 16, 2291

Als Epoxidierungsreagenzien finden Verwendung Persäuren und Peroxide (z.B. Metachlorperbenzoesäure, Peressigsäure, Dimethyldioxiran, Wasserstoffperoxid).

Geeignete Lösungsmittel sind halogenierte Kohlenwasserstoffe wie Methylenchlorid, Chloroform und Chlorbenzol, Alkohole wie Methanol, Ethanol, n-Propanol, Isopropanol, n-Butanol und tert.-Butanol, sowie Wasser,

besonders bevorzugt halogenierte Kohlenwasserstoffe und Wasser.

Es können auch Gemische der genannten Lösungsmittel verwendet werden.

Die Edukte werden im allgemeinen in äquimolaren Mengen miteinander umgesetzt. Es kann vorteilhaft sein, das Epoxidierungsmittel in einem Überschuß bezogen auf XIV einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die für die Herstellung der Benzoylderivate der Formel III mit R⁹ = Wasserstoff und R¹¹ = -C(OH)R'R" benötigten Vinylglycine der Formel XIV sind in der Literatur bekannt [ D. B. Berkowitz et al., J. Org. Chem. 2000, 65, 10, 2907; M. Koen et al., J. Chem. Soc. Perkin I 1997, 4, 487] oder können gemäß der zitierten Literatur hergestellt werden.

Die Epoxidöffnung erfolgt üblicherweise bei Temperaturen von -78°C bis zum Siedepunkt der Reaktionsmischung, bevorzugt -20°C bis 100°C, insbesondere bevorzugt 0°C bis 50°C, in einem inerten organischen Lösungsmittel gegebenenfalls in Gegenwart eines Katalysators [ vgl. P. Meffre et al., Tetrahedron Lett. 1990, 31, 16, 2291; M. R. Paleo et al., J. Org. Chem. 2003, 68, 1, 130].

Geeignete Lösungsmittel sind Alkohole wie Methanol, Ethanol, n-Propanol, Isopropanol, n-Butanol und tert.-Butanol, sowie Dimethylsulfoxid, Dimethylformamid und Dimethylacetamid und Wasser, besonders bevorzugt Methanol und Wasser.

Es können auch Gemische der genannten Lösungsmittel verwendet werden.

Als saure Katalysatoren finden Lewis-Säuren wie Bortrifluorid, Aluminiumtrichlorid, Eisen-III-chlorid, Zinn-IV-chlorid, Titan-IV-chlorid, Zink-II-chlorid und Magnesiumperchlorat Verwendung.

Der Katalysator wird üblicher Weise in einem Mengenverhältnis von 1 bis 100 mol%, bevorzugt 1 bis 10 mol% bezogen auf die Verbindung XV eingesetzt.

Die Edukte werden im allgemeinen in äquimolaren Mengen miteinander umgesetzt. Es kann vorteilhaft sein, Nuc-M⁺ in einem Überschuß bezogen auf XV einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Benzoylderivate der Formel III mit R⁹ = Wasserstoff und R¹¹ = -C(Nuc)R'R" lassen sich anschließend mit Aminen der Formel II analog zu Verfahren A zu den gewünschten benzoylsubstituierten Serin-Amiden der Formel **I** mit R⁹ = Wasserstoff und R¹¹= - C(Nuc)R'R" umsetzen, welche dann mit Verbindungen der Formel IX zu benzoylsubstituierten Serin-Amiden der Formel I mit R¹¹ = -C(Nuc)R'R" derivatisiert werden können [vgl. z.B. Yokokawa, F. et al., Tetrahedron Lett. 42 (34), 5903-5908 (2001); Arrault, A. et al., Tetrahedron Lett. 43( 22), 4041-4044 (2002)];

ebenso können die Benzoylderivate der Formel III mit R⁹ = Wasserstoff zunächst mit Verbindungen der Formel IX zu weiteren Benzoylderivaten der Formel III mit R¹¹ =-.G(Nuc)R'R" analog Verfahren B derivatisiet werden [vgl. z.B. Troast, D. et al., Org. Lett. 4 (6), 991-994 (2002); Ewing W. et al., Tetrahedron Lett., 30 (29), 3757-3760 (1989); Paulsen, H. et al., Liebigs Ann. Chem. 565 (1987)] und anschließend analog zu Verfahren A mit Aminen der Formel II zu den gewünschten benzoylsubstituierten Serin-Amiden der Formel I mit R¹¹ = -C(Nuc)R'R" umgesetzt werden:

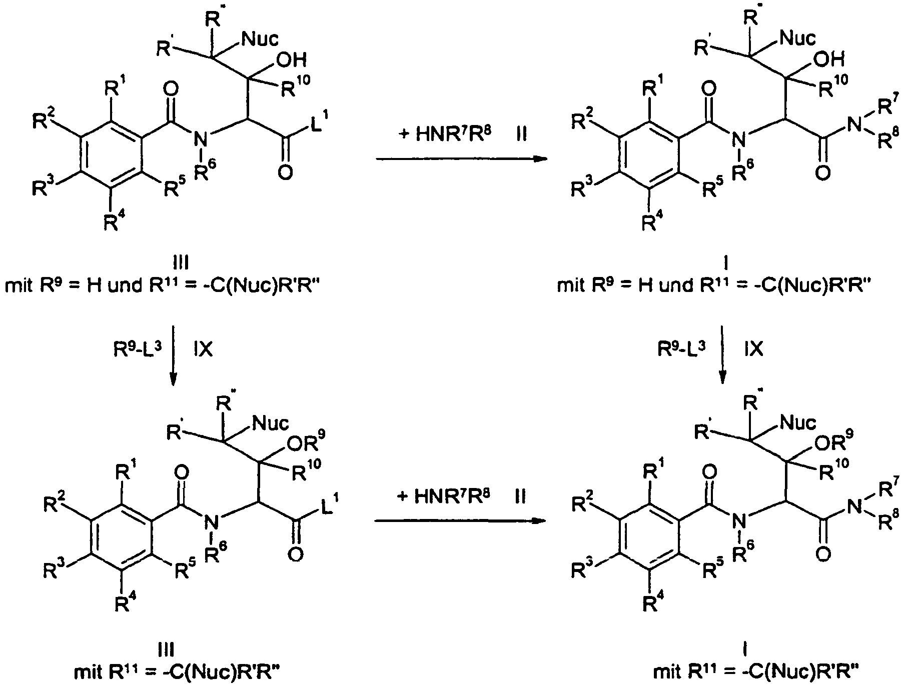

L¹ steht für eine nucleophil verdrängbare Abgangsgruppe, z.B. für Hydroxy oder C₁-C_{S}-Alkoxy.
L³ steht für eine nucleophil verdrängbare Abgangsgruppe, z.B. für Halogen, Hydroxy, oder C₁-C₆-Alkoxy.
R' steht für Wasserstoff, C₁-C₆-Alkyl, C₁-C₆-Hälogenalkyl, C₁-C₆-Hydroxyalkyl, Phenyl oder C₁-C₆-Alkoxycarbonyl.
R" steht für Wasserstoff, C₁-C₆-Alkyl, C₁-C₆-Halogenalkyl, C₁-C₆-Hydroxyalkyl, Phenyl oder C₁-C₆-Alkoxycarbonyl.
Nuc-M⁺ steht beispielsweise für Thiolate wie z.B. Natriumthiophenolat, Alkoholate wie Kaliumphenolat, oder Amide wie Natriumimidazolat.

Benzoylderivate der Formel III 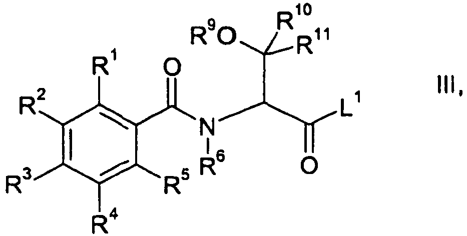 wobei R¹ für Fluor, Chlor oder CF₃ steht und R⁶ für Wasserstoff steht; sowie R² bls R⁴, R⁶ und R⁹ bis R¹¹ die voranstehend genannten Bedeutungen haben und L¹ für Hydroxy oder C₁-C₆-Alkoxy steht, sind ebenfalls ein Gegenstand der vorliegenden Erfindung.

Die besonders bevorzugten Ausführungsformen der Zwischenprodukte in Bezug auf die Variablen entsprechen im Übrigen denen der Reste R¹ bis R⁸ sowie R⁶ bis R¹¹ der Formel I.

Besonders bevorzugt werden Benzoylderivate der Formel III, in der
- R¹: Fluor, Chlor oder CF₃;
- R² und R³: unabhängig voneinander Wasserstoff, Fluor oder Chlor;
- R⁴, R⁵ und R⁶: Wasserstoff-,
- R⁹: Wasserstoff, Formyl, C₁-C₄-Akylcarbonnyl, C₁-C₄-Alkylaminocarbonyl, Di-(C₁-C₄-Alkyl)-aminocarbonyl, Phenylaminocarbonyl, N-(C1-C₄-alkyl)-N-(phenyl)-aminocarbonyl, SO₂CH₃, SO₂CF₃ oder SO₂(C₆H₅);
- R¹⁰: Wasserstoff; und
- R¹¹: C₁-C₆-Alkyl, C₂-C₆-Alkenyl, C₁-C₆-Halogenalkyl, C₂-C₆-Halogenalkenyl, C₁-C₈-Hydroxyalkyl, Hydroxycarbonyl-C₁-C₄-alkyl, Phenyl-C₁-C₄-alkyl oder Phenyl-C₁-C₄-hydroxyalkyl
bedeuten.

Die folgenden Beispiele dienen der Erläuterung der Erfindung.

### Herstellungsbeispiele

### -Beispiel 1

### 4-(3-Fluor-phenylsulfanyl)-2-(4-fluoro-2-trifluormethyl-benzoylamino)-3-hydroxy-buttersäuremethyl amid (Tab. 3, Nr. 3.12)

### 1.1) 4-Fluoro-N-(1-hydroxymethyl-alkyl)-2-trifluoromethylbenzamid

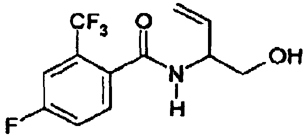

19 g (146 mmol) 2-S-2-Aminobut-3-en-1-ol-Hydrochlorid wurden in CH₂Cl₂ suspendiert und bei 0°c nacheinander 59 g (584 mmol) Triethylamin und 68.2 g 4-Fluor-2-trifluomethylbenzoylchlorid zugetropft. Anschließend wurde 15 h bei RT gerührt. Das Lösungsmittel wurde abgezogen, der Rückstand in Essigester aufgenommen und mit 5 %iger NaHCO₃-Lösung gerührt. Danach wurde der unlösliche Rückstand abfiltriert und das Filtrat mit 5 %iger NaHCO₃ nachextrahiert. Die organische Phase wurde eingeengt und der Rückstand (65.8 g) in THF gelöst. Zu der Lösung wurden bei 0°C 7.3 g (300 mmol) LiOH in H₂O zugetropft. Die Lösung wurde 15 h bei RT gerührt, danach die Lösungsmittel entfernt und der Rückstand mit CH₂Cl₂ extrahiert. Die vereinigten organischen Phasen wurden gewaschen, getrocknet und anschließend das Lösungsmittel entfernt. Man erhielt 34.8 g (86 % der Theoie) der Titelverbindung als farblosen Feststoff.
¹H-NMR (DMSO): δ = 3.4-3.6 (m, 2H); 4.50 (t, 1H); 4.85 (t, 1H); 5.15 (d, 1H); 5.25 (d, 1H); 5.8-6.0 (m, 1H); 7.5-7.7 (m, 3H); 8.55 (d, 1H).

### 1.2) 2-(4-Fluor-2-trifluormethyl-benzoylamino)-but-3-ensäure

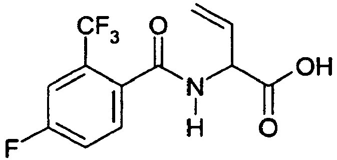

51.5 g (226 mmol) Periodsäure und 9.9 g (99 mmol) CrO₃ wurden in Acetonitril/ Wasser bei RT gelöst. Weiterhin wurden 25 g (90.3 mmol) 4-Fluor-N-(1-hydroxymethyl-allyl)-2-trifluormethyl-benzamid in Acetonitril/ Wasser gelöst und zuvor hergestellte NalO₄/CrO₃-Lösung bei 0-5°C über 3 h zugetropft. Anschließend wurde 15 h bei RT gerührt. Danach tropfte man 400 ml 6%ige Na₂HPO₄-Lösung zu, gab Toluol und - Essigsäureethylester zu und trennte die Phasen. Die wässrige Phase wurde mit Essigester nachextrahiert. Die vereinigten organischen Phasen wurden gewaschen, getrocknet und eingeengt. Man erhielt 24.1 g (92 % der Theorie) der Titelverbindung. ¹H-NMR (DMSO): δ = 5.00 (t, 1H); 5.30 (d, 1H); 5.40 (d, 1H); 5.9-6.1 (m, 1H); 7.5-7.7 (m; 3H); 9.10 (d, 1-H); 13.0 (br, 1H).

### 1.3) 2-(4-Fluor-2-trifluormethyl-benzoylamino)-but-3-ensäure-methylester

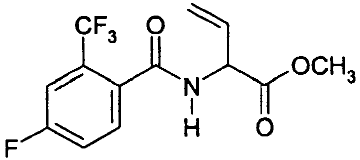

25 g (90.3 mmol) 2-(4-Fluoro-2-trifluormethyl-benzoylamino)-but-3-ensäure wurden in Methanol gelöst und 9.9 g(82.8 mmol) Thionylchlorid über 30 min zugetropft. Die Lösung wurde drei Tage bei RT gerührt. Nach Entfernen der Lösungsmittel erhielt man 25.2 g Produkt (100 % der Theorie) der Titelverbindung als farblosen Feststoff. ¹H-NMR (DMSO): δ = 3.70 (s, 3H); 5.05 (t, 1H); 5.30 (d, 1H); 5.40 (d, 1H); 5.9-6.0 (m, 1H); 7.5-7.7 (m, 3H); 9.30 (d, 1H).

### 1.4) (4-Fluor-2-trifluormethyl-benzoylamino)-oxiranyl-essigsäuremethylester

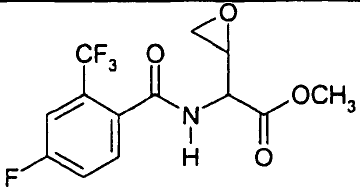

16.0 g (52.5 mmol) 2-(4-Fluoro-2-trifluormethyl-benzoylamino)-but-3-ensäure-methylester wurden in CH₂Cl₂ gelöst, 14.1 g (63.0 mmol) 70-%ige m-Chlorperbenzoesäure (MCPBA) zugegeben und 15h bei RT gerührt. Danach wurden die Lösungsmittel entfernt und der Rückstand chromatographisch gereinigt

Man erhielt eine Mischung von Edukt und Produkt, die nochmals mit 6 g (26.8 mmol) MCPBA in CH₂Cl₂ für 60 h gerührt wurde. Nach analoger Aufarbeitung und Chromatographie erhielt man 7 g (42 % der Theorie) diasteromerenreine Titelverbindung als farblosen Feststoff, sowie 2.5 g Edukt.

¹H-NMR (DMSO): δ = 2.70 (m, 1H); 2.85 (t, 1H); 3.40 (m, 1H); 3.70 (s, 3H); 4.45 (t, 1H); 7.55-7.75 (m, 3H); 9.25 (d, 1H).

### 1.5) 4-(3-Fluor-phenylsulfanyl)-2-(4-fluor-2-trfiuoromethyl-benzoylamino)-3-hydroxy-buttersäuremethylester (Tab. 2. Nr. 2.7)

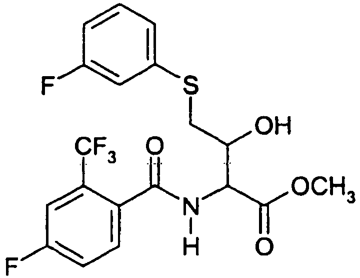

480 mg (1.5 mmol) (4-Fluor-2-trifluormethyl-benzoylamino)-oxiranyl-essigsäure-methylester wurden in Methanol gelöst und danach wurden 215 mg (1.5 mmol) 3-Fluorthiophenol und 280 mg (3.8 mmol) Triethylamin zugegeben. Anschließend wurde15 h bei RT gerührt und danach das Lösungsmittel enfemt. Man erhielt 760 mg diasteromerenreines Rohprodukt, das ohne weitere Reinigung in der nächsten Stufe eingesetzt wurde.
¹H-NMR (DMSO): δ = 3.15 (d, 2H); 3.70 (s, 3H); 4.65 (m, 1H); 4.85 (d, 1H); 5.65 (d, 1H); 7.0-7.4 (m, 4H); 7.6-7.8 (m, 3H); 8.85 (d, 1H).

### 1.6) 4-(3-Fluor-phenylsulfanyl)-2-(4-fluor-2-trifluormethyl-benzoylamino)-3-hydroxy-buttersäure-methylamid (Tab. 3, Nr. 3.12)

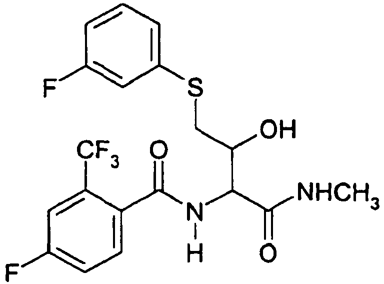

760 mg (ca. 1.5 mmol) Rohprodukt aus der Stufe 1.5 wurden in Methanol gelöst. Danach wurde unter leichter Kühlung 30 min Methylamin eingegast. Es wurde 15 h bei RT gerührt. Danach wurden die Lösungsmittel eingeengt, der Rückstand mit MTBE verrührt und der Niederschlag abgesaugt. Man erhielt 416 mg (62 % der Theorie über 2 Stufen) der Titelverbindung (Diastereomerengemisch 4: 1) als farblosen Feststoff mit dem Fp. 172°C.
¹H-NMR (DMSO, Hauptdiastereomer): δ = 2.65 (d, 3H); 3.0-3.2 (m, 2H); 4.05 (m, 1H); 4.65 (dd, 1H); 5.55 (d, 1H); 6.9-7.4 (m, 4H); 7.6-8.8 (m, 4H); 8.45 (d, 1H).

### Beispiel 2

### Dimethyl-carbaminsäure-2-dimethylcarbamoyloxy-3-(4-fluor-2-trifluoromethyl-benzoylamino)-3-methylcarbamoyl-propylester (Tab. 3, Nr.3.56)

### 2.1) 2-(4-Fluor-2-trifluormethyl-benzoylamino)-3,4-dihydroxy-buttersäuremethylester (Tab. 2, Nr. 2.8)

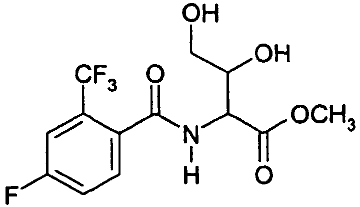

460 mg (1.5 mmol) 2-(4-Fluor-2-trifluormethyl-benzoylamino)-but-3-ensäure-methylester wurden in tert-Butanol/Wasser gelöst und 360 mg (3 mmol) N-Methylmorpholin-N-Oxid und 1.5 g (0.15 mmol) 2.5 %-ige Lösung von OsO4 in tert-Butanol zugegeben. Es wurde 60 Stunden bei RT gerührt. Danach gab man 3.2 g Natriumsulfit, Wasser und Essigsäureethylester zu und trennte die Phasen. Die wässrige Phase wurde mit Essigester nachextrahiert. Die vereinigten organischen Phasen wurden gewaschen, getrocknet und das Lösungsmittel entfernt. Man erhielt 440 mg (86 % der Theorie) Rohprodukt (Diastereomerenverhältnis ca. 2:1), das ohne weitere Reinigung in der nächsten Stufe eingesetzt wurde.

### 2.2) 2-(4-Fluor-2-trifluormethyl-benzoylamino)-3,4-dihydroxy-buttersäuremethylamid (Tab. 3, Nr. 3.8)

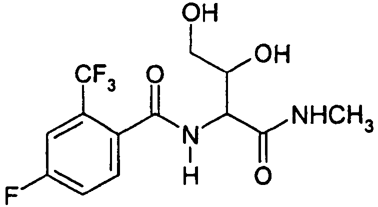

650 mg (1.5 mmol) Rohprodukt aus der vorherigen Stufe 2.1. wurden in Methanol gelöst und unter leichter Kühlung 30 min Methylamin eingegast. Es wurde 15 h bei RT gerührt. Danach wurden die Lösungsmittel eingeengt, der Rückstand mit MTBE verrührt und der Niederschlag abgesaugt. Man erhielt 340 mg (67 % der Theorie über 2 Stufen) der Titelverbindung als farblosen Feststoff (Diasteromerenverhältnis 2:1) mit dem Fp. 163°C.
¹H-NMR (DMSO, Hauptdiastereomer): δ = 2.65 (d, 3H); 3.4-3.6 (m, 2H); 3.90 (m, 1H); 4.50 (d, 1H); 4.60 (m, 1H); 5.05 (m, 1H); 7.6-7.8 (m, 4H); 8.15 (d, 1H).

### 2.3) Dimethyl-carbaminsäure-2-dimethylcarbamoyloxy-3-(4-fluor-2-trifluormethylbenzoylamino)-3-methylcarbamoyl-propylester (Tab. 3, Nr. 3.54)

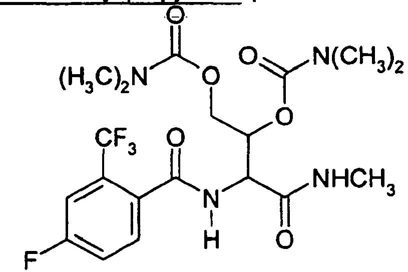

170 mg (0.5 mmol) 2-(4-Fluor-2-trifluoromethyl-benzoylamino)-3,4-dihydroxybuttersäure-methylamid wurden in 550 mg (5.0 mmol) Dimethylcarbaminsäurechlorid und 150 mg (1.5 mmol) Triethylamin gelöst. Man gab 10 mg Dimethylaminopyridin zu und erwärmte die Reaktionsmischung 18 h auf 50°C. Während dieser Zeit wurden dreimal weitere jeweils 200 mg Dimethylcarbaminsäurechlorid und 50 mg Triethylamin zugegeben. Anschließend wurde die Reaktionsmischung eingeengt, in Essigsäureethylester aufgenommen, gewaschen, getrocknet und wieder eingeengt. Man erhielt 140 mg (58 % der Theorie) der Titelverbindung als farblosen Feststoff (Diasteromerenverhältnis 4:1) mit dem Fp. 64°C.
¹H-NMR (DMSO, Hauptdiasteromer): δ = 2.65 (d, 3H); 2.80 (s, 6H); 3.00 (s, 3H); 3.10 (s, 3H); 4.0-4.2 (m, 3H), 4.7-4.8 (m, 1H); 7.6-7.8 (m, 3H); 8.85 (d, 1H).

### Beispiel 3

### N-(2-Hdroxy-1-methylcarbamoyl-but-3-enyl)-4-fluoro-2-trifluormethyl-benzamide (Tab. 3, Nr. 3.3)

### 3.1) (4-Fluor-2-trifluoromethyl-benzoylamino)-essigsäureethylester

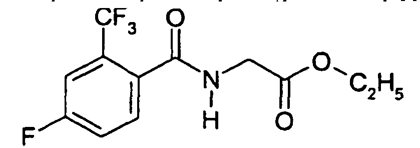

30.7 g (0.22 mol) Glycinethylester-Hydrochlorid wurden in CH₂Cl₂ suspendiert. Bei 0°C tropfte man nacheinander 86.3 g (0.854 mol) Triethylamin und 50 g (0.22 mol) 2-Trifluormethyl-4-fluor-benzoylchlorid gelöst in 250 ml CH₂Cl₂ zu. Nach 48 h bei RT wurde die Lösung gewaschen, getrocknet und das Lösungsmittel entfernt. Man erhielt 62.2 g (97 % der Theorie) der Titelverbindung als farblosen Feststoff.
¹H-NMR (DMSO): δ = 1.2 (t, 3H); 4.00 (d, 2H); 4.15 (q, 2H); 7.6-7.8 (m, 3H); 9.00 (t, 1H).

### 3.2) 3-Hydroxy-2-(4-Fluor-2-trifluormethyl-benzoylamino)-pent-4-ensäureethylester (Tab. 2, Nr. 2.6)

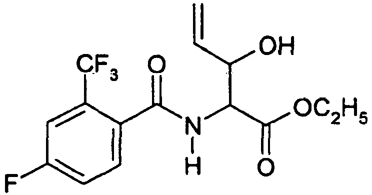

Zu 24.3 ml (0.049 mol) 2M Lithiumdiisopropylamid-Lösung in THF wurden bei -78°C 6.0 g (0.0205 mol) (4-Fluor-2-trifluormethyl-benzoylamino)-essigsäureethylester gelöst in THF zugetropft. Nach 1 h bei -78°C wurden 1.4 g (0.025 mol) Acrolein gelöst in THF zugetropft und 1 h bei -78°C gerührt. Anschließend gab man gesättigte NH₄Cl-Lösung zu und ließ auf RT erwärmen. Es wurde mit CH₂Cl₂ extrahiert und die vereinigten organischen Phasen anschließend gewaschen, getrocknet und das Lösungsmittel entfernt. Der Rückstand wurde chromtographisch gereinigt (SiO₂; Cyclohexan / Essigester). Man erhielt 4.7 g (66% der Theorie) der Ttelverbindung als farblosen Feststoff (Diastereomerengemisch), das ohne weitere Reinigung weiter umgesetzt wurde.

### 3.3) N-(2-Hydroxy-1-methylcarbamoyl-but-3-enyl)-4-fluoro-2-trifluormethyl-benzamid (Tab. 3, Nr. 3.3)

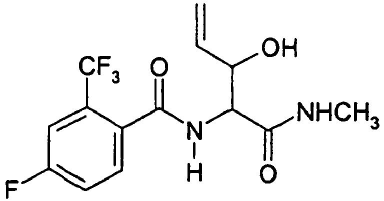

4.5 g (12.9 mmol) 3-Hydroxy-2-(4-Fluor-2-trifluoromethyl-benzoylamino)-pent-4-ensäureethylester wurden in Methanol gelöst. Unter Eiskühlung leitete man über 2 h Methylamin-Gas ein. Anschließend wurde die Reaktionslösung eingeengt und gewaschen. Man erhielt 3.1 g (80 % der Theorie) der Titelverbindung als farblosen Feststoff (Diastereomerenverhältnis 2:1).
¹H-NMR (Hauptdiastereomer): δ = 2.60 (d, 3H); 4.25 (br, 1H); 4.35 (t, 1H);5.15 (d, 1H); 5.30 (d, 1H); 5.35 (d, 1H); 5.85 (m, 1H); 7.5-7.8 (m, 3H); 7.90 (d, 1H); 8.65 (d, 1H).

### Beispiel 4

### Dimethyl-carbaminsäure-2-chlor-1-[(4-fluor-2-trifluormethyl-benzoylamino)-methylcarbamoyl-methyl]-3-phenyl-allylester (Tab. 3, Nr. 3.53)

### 4.1.) 4-Chlor-3-hydroxy-5-phenyl-2-(4-fluor-2-trifluormethyl-benzoylamino)-pent-4-ensäureethylester (Tab. 2, Nr. 2.6)

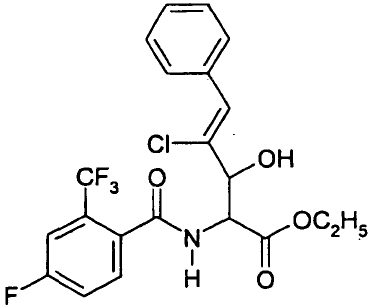

Zu 25.0 ml (0.050 mol) 2M Lithiumdüsopropylamid-Lösung in THF wurden bei -75°C 5.0 g (0.0170 mol) (4-Fluor-2-trifluormethyl-benzoylamino)-essigsäureethylester gelöst in THF zugetropft. Nach 1h bei -75°C wurden 3.50 g (0.021 mol) 2-Chlor-Zimtaldehyd gelöst in THF zugetropft und 1 h bei -75°C gerührt. Es wurde gesättigte NH₄Cl-Lösung zugetropft und auf RT erwärmt. Es wurde mit CH₂Cl₂ extrahiert. Die vereinigten organischen Phasen wurden gewaschen, getrocknet und man enfernte das Lösunsmittel. Der Rückstand wurde chromatographisch gereinigt (SiO₂, Cyclohexan/Essigester). Man erhielt 7.5 g (96 % der Theorie) der Titelverbindung als farblosen Feststoff (Diastereomerengemisch), die ohne weitere Reinigung weiter umgesetzt wurde.

### 4.2) N-(3-Chlor-2-hydroxy-1-methylcarbamoyl-4-phenyl-but-3-enyl)-4-fluor-2-trifluoromethyl-benzamid (Tab. 3, Nr. 3.6)

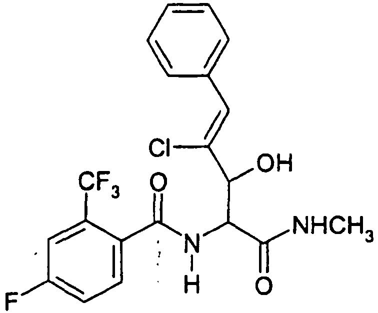

7.8 g (16.9 mmol) 4-Chlor-3-hydroxy-5-phenyl-2-(4-fluoro-2-trifluormethylbenzoylamino)-pent-4-ensäureethylester wurden in Methanol gelöst. Unter Eiskühlung leitete man über 3 h Methylamin-Gas ein. Danach wurde die Rektionslösung eingeengt, mit Pentan gewaschen und aus Aceton umkristallisiert. Der Rückstand ergab 1.2g Zielprodukt als reines Diastereomer. Das Filtrat wurde eingeengt und ergab 7.0 g einer Diastereomerenmischung. Man erhielt daher insgesamt 1.9 g (100% der Theorie) der Titelverbindung als farblosen Feststoff mit dem Fp 140°C.
¹H-NMR (DMSO) (Rückstand): δ = 2.65 (d, 3H); 4.60 (m, 1H); 4.65 (t, 1H);5.95 (d, 1H); 6.85 (s, 1H); 7.2-7.8 (m, 8H); 8.10 (d, 1H); 8.75 (d, 1H).

### 4.3) Dimethyl-carbaminsäure-2-chlor-1-[(4-fluor-2-trifluormethyl-benzoylamino)-methylcarbamoyl-methyl]-3-phenyl-allylester (Tab. 3, Nr. 3.53)

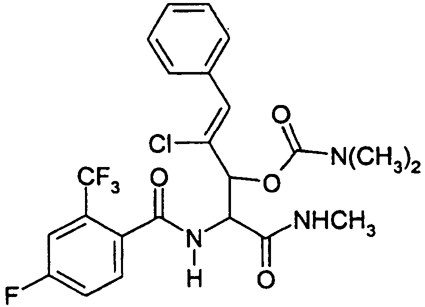

400 mg (0.90 mmol) N-(3-Chlor-2-hydroxy-1-methylcarbamoyl-4-phenyl-but-3-enyl)-4-fluor-2-trifluormethyl-benzamid, 2.36 g (22.0 mmol) Dimethylaminocarbonylchlorid, 1.81 g (17.8 mmol) Triethylamin und ca. 10 ml Dimethylaminopyridin wurden in Dioxan gelöst und 6 h unter Rückfluß erhitzt. Danach wurde die Reaktionslösung eingeengt, der Rückstand mit CH₂Cl₂ aufgenommen, gewaschen und das Lösungsmittel entfernt. Der Rückstand wurde mit Pentan/Diisopropylether gewaschen und getrocknet. Man erhielt 0.30 g (65 % der Theorie) der Titelverbndung als farblosen Feststoff mit dem Fp. 210°C.
¹H-NMR (DMSO): δ = 2.70 (s, 3H); 2.80 (s, 3H); 2.90 (s, 3H); 5.00 (t, 1H); 5.55 (d, 1H); 6.95 (s, 1H); 7.3-7.8 (m, 8H); 8.45 (m,1H); 9.00 (s, 1H).

### Beispiel 5

### 4-Fluor-N-(3,3,3-trifluor-2-hydroxy-1-methylcarbamoyl-propyl)-2-trifluoromethylbenzamid (Tab. 3, Nr. 3.1)

### 5.1) 4,4,4-Trifluor-2-(4-fluoro-2-trifluormethyl-benzoylamino)-3-hydroxybuttersäureethylester (Tab. 2, Nr. 2.1)

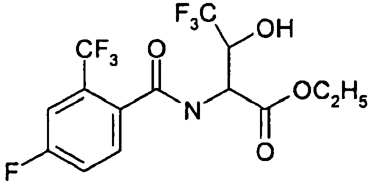

1.7 g (8.5 mmol) 2-Amino-4,4,4-trifluor-3-hydroxy-buttersäureethylester wurden in THF gelöst, zunächst 1.8 g 4-Fluor-2-Trifluormethyl-Benzoesäure und 2.6 g (25.4 mmol) Triethylamin und dannn bei 5°C 1.9 g (8.5 mmol) Bis(2-oxo-3-oxazolinidylphosphorylchlorid zugegeben. Es wurde 16 h bei RT gerührt. Danach engte man die REaktionslösung ein, verdünnte den Rückstand mit Wasser und extrahierte mit Essigsäureethylester. Die vereinigten organischen Phasen wurden getrocknet und das Lösungsmittel entfernt. Man erhielt 3.1 g (93 % der Theorie) der Titelverbindung als farblosen Rückstand.
¹H-NMR (DMSO): δ = 1.20 (t, 3H); 4.20 (m, 2H); 4.65 (m, 1H); 5.05 (q, 1H); 6.95 (d, 1H); 7.4-7.8 (m, 3H); 9.05 (d, 1H).

### 5.2.) 4-Fluor-N-(3,3,3-trifluor-2-hydroxy-1-methylcarbamoyl-propyl)-2-trifluormethylbenzamid (Tab. 3, Nr. 3.1)

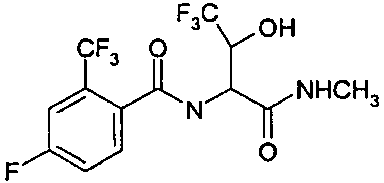

3.1 g (7.9 mmol) 4,4,4-Trifluor-2-(4-fluor-2-trifluormethyl-benzoylamino)-3-hydroxybuttersäureethylester wurden in Ethanol gelöst. Man gab bei RT 20 ml 3.9%-ige Lösung von Methylamin in Ethanol zugegeben. Nach 5h Rühren bei RT wurde 10 min Methylamingas eingegast und 16 h bei RT gerührt. Danach engte man die Reaktionsösung ein und wusch den Rückstand mit MTBE. Man erhielt 1.8 g (61 % der Theorie) der Titelverbindung als farblose Kristalle (Diastereomerenverhältnis 3:1) mit dem Fp. 212°C.
¹H-NMR (DMSO) (Hauptdiastereoisomer): δ = 2.65 (d, 3H); 4.50 (br, 1H); 4.80 (d, 1H); 6.80 (br, 1H); 7.6-7.8 (m, 3H); 7.85 (br, 1H); 8.55 (d, 1H).

In den nachfolgenden Tabellen 2, 3 und 4 werden neben den voranstehenden Verbindungen noch weitere Benzoylderivate der Formel III sowie benzoylsubstituierte Serin-Amide der Formel I aufgeführt, die in analoger Weise nach den voranstehend beschriebenen Verfahren hergestellt wurden oder herstellbar sind.

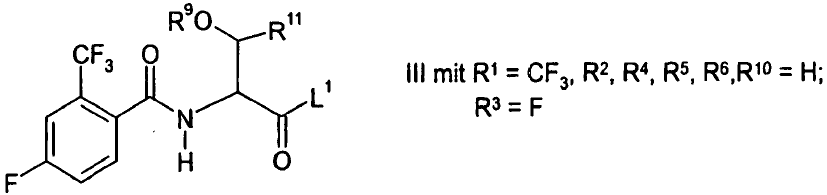

**Tabelle 2**

| Nr. | R⁹ | R¹¹ | L¹ | Diastereomerenverhältnis | Chiralität | M+(m/z) |
|---|---|---|---|---|---|---|
| 2.1. | H | CF₃ | OC₂H₅ | 3:1 | rac | 391 |
| 2.2. | H | CH=CH₂ | OC₂H₅ | 1:1 | rac | 349 |
| 2.3. | H | CH=C(CH₃)₂ | OC₂H₅ | 1:1 | rac | 377 |
| 2.4. | H | CH=C(CH₃)₂ | OC₂H₅ | 1:0 | rac | 377 |
| 2.5. | H | C(CH₃)=CHCH₃ (anti) | OC₂H₅ | 1:2 | rac | 377 |
| 2.6. | H | CCl=CH(C₆H₅) (syn) | OC₂H₅ | 1:0 | rac | 459 |
| 2.7. | H | CH₂OH | OCH₃ | 1:0 | 2-S | 339 |
| 2.8. | H | -CH₂-S-(3-F-Phenyl) | OCH₃ | 1:0 | 2.S | 449 |

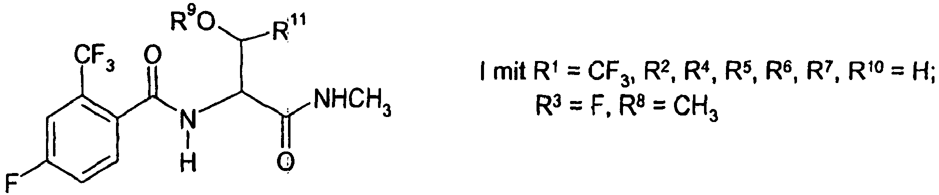

**Tabelle 3**

| Nr. | R9 | R¹¹ | Diastereomerenverhältnis | Chiralität | Fp. | M+ (m/z) |
|---|---|---|---|---|---|---|
| 3.1. | H | CF₃ | 3:1 | rac | 212 | |
| 3.2. | H | CF₂CHF₂ | 0:1 | rac | 210 | 408 |
| 3.3. | H | CH=CH₂ | 1:1 | rac | 163 | 334 |
| 3.4. | H | CH=C(CH₃)₂ | 1:1 | rac | 125 | 362 |
| 3.5. | H | CH=C(CH₃)₂ | 1:0 | rac | 169 | 362 |
| 3.6. | H | C(CH₃)=CHCH₃ (anti) | 1:2 | rac | 115 | 362 |
| 3.7. | H | CCI=CH(C₆H₅) (syn) | 1:0 | rac | 140 | 444 |
| 3.8. | H | CH₂OH | 2:1 | 2-S | 163 | 338 |
| 3.9. | H | CH(OH)CH(OH)(C₆H₅) | 3:2 | rac | 97 | 414 |
| 3.10. | H | CH₂-S-(C₆H₅) | 1:0 | 2-S | 157 | |
| 3.11. | H | CH₂-S-(2-F-Phenyl) | 4:1 | 2-S | 151 | |
| 3.12. | H | CH₂-S-(3-F-Phenyl) | 4:1 | 2-S | 172 | |
| 3.13. | H | CH₂-S-(2-Cl-Phenyl) | 8:1 | 2-S | 161 | |
| 3.14. | H | CH₂-S-(2-CH₃-Phenyl) | 4:1 | 2-S | 143 | |
| 3.15. | H | CH₂-S-(1-Imidazolyl) | 4:1 | 2-S | 171 | |
| 3.16. | H | CH₂-S-(2-Imidazolyl) | 4:1 | 2-S | 159 | |
| 3.17. | H | CH₂-S-[2-(4,6-OCH₃)-Pyrimidyl] | 6:1 | 2-S | 209 | |
| 3.18. | H | CH₂-SO₂-(C₆H₅) | | | | |
| 3.19. | H | CH₂-SO₂-(2-F-Phenyl) | 8:1 | 2-S | 128 | |
| 3.20. | H | CH₂-SO₂-(3-F-Pheny) | 4:1 | 2-S | 131 | |
| 3.21. | H | CH₂-SO₂-(2-Cl-Phenyl) | 8:1 | 2-S | 165 | |
| 3.22. | H | CH₂-SO₂-(2-CH₃-Phenyl) | 4:1 | 2-S | 132 | |
| 3.23. | H | CH₂-SO₂-[2-(4,6-OCH₃)-Pyrimidy)] | 4:1 | 2-S | 164 | |
| 0.24. | CH₂(C₆H₅) | CH₃ | 1:0 | 2-S | | 412 |
| 3.25. | Si(CH₂CH₃)₃ | CF₃ | 5:1 | rac | 128 | 490 |
| 3.26. | (CO)CH₃ | CF₃ | 7:3 | rac | 182 | 418 |
| 3.27. | (CO)CH₃ | CF₂CHF₂ | 4:1 | rac | 155 | 450 |
| 3.28. | (CO)CH₃ | CH=CH₂ | 3:1 | rac | 137 | 376 |
| 3.29. | (CO)CH₃ | CH=C(CH₃)₂ | 1:0 | rac | 170 | |
| 3.30. | (CO)C(CH₃)₃ | CF₃ | 1:0 | rac | 196 | |
| 3.31. | (CO)C(CH₃)₃ | CF₂CHF₂ | 1:0 | rac | 156 | 492 |
| 3.32. | (CO)CH₂OCH₃ | CF₃ | 2:1 | rac | 163 | 448 |
| 3.33. | (CO)CH₂OCH₃ | CF₂CHF₂ | 4:1 | rac | 151 | 480 |
| 3.34. | (CO)CH₂OCH₂CH₂OCH₃ | CF₃ | 1:1 | rac | 144 | 492 |
| 3.35. | (CO)CH₂OCH₂CH₂OCH₃ | CF₂CHF₂ | 4:1 | rac | 134 | 524 |
| 3.36. | (CO)CH₂O(2,4-Cl₂-C₆H₃) | CF₃ | 4:1 | rac | 204 | 579 |
| 3.37. | (CO)CH₂O(2,4-Cl₂-C₆H₃) | CF₂CHF₂ | 4:1 | rac | 137 | 611 |
| B.38. | (CO)CH₂SCH₃ | CF₃ | 2:1 | rac | 161 | 464 |
| 3.39. | (CO)CH₂SCH₃ | CF₂CHF₂ | 4:1 | rac | 165 | 496 |
| 3.40. | (CO)CH₂SC₆H₅ | CF₃ | 2:1 | rac | 154 | 526 |
| 3.41. | (CO)CH₂SC₆H₅ | CF₂CHF₂ | 4:1 | rac | 137 | 558 |
| 3.42. | (CO)CH₂S[2-(4,6-OCH₃)pyrimidyl] | CF₂CHF₂ | 4:1 | rac | 170 | 620 |
| 3.43. | (CO)CH₂NH₃⁺Cl⁻ | CF₃ | 2:1 | rac | 193 | |
| 3.44. | (CO)CH₂NH(CO)OC(CH₃)₃ | CF₃ | 4:1 | rac | 109 | 533 |
| 3.45. | (CO)CH₂NH(CO)OC(CH₃)₃ | CF₂CHF₂ | 4:1 | rac | 164 | 565 |
| 3.46. | (CO)N(CH₃)₂ | CHC(CH₃)₂ | 1:0 | rac | 197 | |
| 3.47. | (CO)N(CH₃)₂ | CH(OH)CH₂OH | 2:1 | rac | 140 | 439 |
| 3.48. | (CO)N(CH₃)₂ | [1,3]-Dioxolan-2on-4-yl | 2:1 | rac | 1* | 465 |
| 3.49. | (CO)N(CH₃)₂ | CF₃ | 2:1 | rac | | 447 |
| 3.50. | (CO)N(CH₃)₂ | CF₂CHF₂ | 1:0 | rac | 170 | 479 |
| 3.51. | (CO)N(CH₃)₂ | CF₂CHF₂ | 0:1 | rac | 211 | 479 |
| 3.52. | (CO)N(CH₃)₂ | CH=CH₂ | 3:1 | rac | 128 | 405 |
| 3.53. | (CO)N(CH₃)₂ | CCl=CH(C₆H₅) (syn) | 1:0 | rac | 210 | 515 |
| 3.54. | (CO)N(CH₃)₂ | CH₂O(CO)N(CH₃)₂ | 1:0 | 2-S | 64 | |
| 3.55. | (CO)N(CH₃)₂ | CH[OCON(CH₃)₂][Phenyl] | 2:1 | rac | | 578 |
| 3.56. | (CO)N(CH₃)₂ | CH[O(CO)N(CH₃)₂][CH₂O(CO)N(CH₃)₂] | 2:1 | rac | 135 | |
| 3.57. | (CO)N(CH₃)₂ | CH₂-S-(2-F-Phenyl) | 4:1 | 2-S | | 519 |
| 3.58. | (CO)N(CH₃)₂ | CH₂-S-(3-F-Phenyl) | 4:1 | 2-S | | 519 |
| 3.59. | (CO)N(CH₃)₂ | CH₂-S-(2-Cl-Phenyl) | 4:1 | 2-S | | 535 |
| 3.60. | (CO)N(CH₃)₂ | CH₂-S-[2-(4,6-OCH₃)-Pyrimidyl] | 4:1 | 2-S | 2* | |
| 3.61. | O(CO)CH₃ | CH=CH₂ | 3:1 | rac | 137 | 376 |
| 3.62. | O(CO)CH₃ | CH=C(CH₃)₂ | 1:1 | rac | 170 | 404 |

| | | | | | | |
|---|---|---|---|---|---|---|
| 1* ¹H-NMR (d4-MeOH): δ = 2.75(2+d, 3H); 2.9 (br, 6H); 4.6-4.8 (m, 3H); 5.0-5.4 (m, 2H);7.4-7.6 (m, 3H) 2* ¹H-NMR (d4-MeOH): δ = 2.75 (s, 3H); 3.05 (s, 3H); 3.20 (s, 3H); 3.4-3.6 (m, 2H); 3.95 (s, 6H); 5.00 (d, 1H); 5.50 (q. 1H); 5.85 (s, 1H); 7.4-7.6 (m, 3H) | | | | | | |

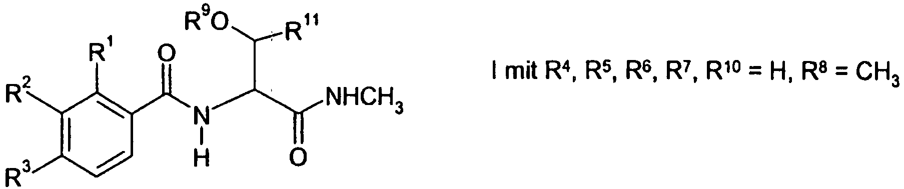

**Tabelle 4**

| Nr. | R¹ | R² | R3 | R9 | R¹¹ | Diastereomerenverhältnis | Chiralität | Fp. | M+ (m/z) |
|---|---|---|---|---|---|---|---|---|---|
| 4.1. | CF₃ | H | H | H | CH=CH₂ | 1:0 | rac | | 316 |
| 4.2. | CF₃ | H | H | H | CH(OH)CH(OH)(C₆H₅) | 3:2 | rac | 75 | 396 |
| 4.3. | CF₃ | H | H | CH₂(C₆H₅) | CH₃ | 1:0 | 2-S | | 394 |
| 4.4. | CF₃ | H | H | (CO)N(CH₃)₂ | CH=CH₂ | 1:0 | rac | 142 | 387 |
| 4.5. | CF₃ | H | H | O(CO)CH₃ | CH=CH₂ | 1:0 | rac | 146 | |
| 4.6. | Cl | Cl | H | H | CH(OH)CH(OH)(C₆H₅) | 3:2 | rac | | 397 |
| 4.7. | Cl | H | H | CH₂(C₆H₅) | CH₃ | 1:0 | 2-S | | 395 |
| 4.8. | Cl | H | Cl | CH₂(C₆H₅) | CH₃ | 1:0 | 2-S | | 395 |
| 4.9. | Cl | CF₃ | H | H | CH(OH)CH(OH)(C₆H₅) | 1:1 | rac | 128 | 431 |
| 4.10. | Cl | CF₃ | H | CH₂(C₆H₅) | CH₃ | 1:0 | 2-S | | 428 |

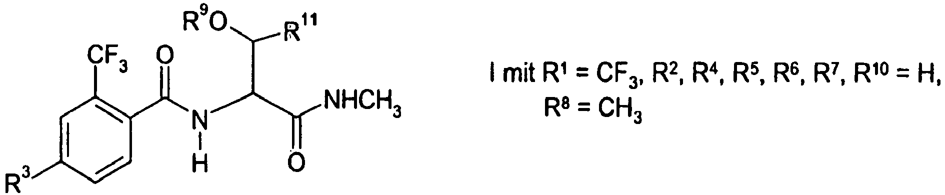

**Tabelle 5**

| Nr. | R3 | R9 | R¹¹ | Diastereomerenverhältnis | Chiralität | Fp. | M+ (m/z) |
|---|---|---|---|---|---|---|---|
| 5.1. | H | (CH₃)₂NCO | CH=C(CH₃)₂ | 3:2 | rac. | 132 | |
| 5.2. | H | (CH₃)₂NCO | CH₂OSCH₂OH | 4:1 | rac. | | 421 |
| 5.3. | H | (CH₃)₂NCO | Cyclopropyl | 0:1 | rac. | 188 | |
| 5.4. | H | CH₃CO | CH=O(CH₃)₂ | 3:2 | rac. | 165 | |
| 5.5. | H | CH₃HNCO | CH=C(CH₃)₂ | 4:1 | rac. | 143 | |
| 5.6. | H | H | CH=C(CH₃)₂ | 3:2 | rac. | 175 | |
| 5.7. | H | H | CH=C(CH₃)₂ | 3:1 | rac. | 169 | |
| 5.8. | F | (CH₃)₂NCO | 2-(CONHCH₃)-Cyclopropyl | 1:1 | rac. | 233 | |
| 5.9. | F | (CH₃)₂NCO | 2-(COOC₂H₅)-Cyclopropyl | 0:1 | rac. | 195 | |
| 5.10. | F | (CH₃)₂NCO | 2-(COOH)-Cyclopropyl | 0:1 | rac. | 194 | |
| 5.11. | F | (CH₃)₂NCO | 3-(2H-Tetrahydrothiopyranyl) | 0:1 | rac. | 212 | |
| 5.12. | F | (CH₃)₂NCO | 3-(2H-Tetrahydrothiopyranylsulfoxid) | 0:1 | rac. | 215 | |
| 5.13. | F | (CH₃)₂NCO | 3-2H-Tetrahydropyranyl | 1:0 | rac. | | 463 |
| 5.14. | F | (CH₃)₂NCO | 3-2H-Tetrahydropyranyl | 2:1 | rac. | 162 | |
| 5.15. | F | (CH₃)₂NCO | 3-2H-Tetrahydropyranyl | 4:1 | rac. | 190 | |
| 5.16. | F | (CH₃)₂NCO | CH₂(C₆H₅) | 0:1 | rac. | 228 | |
| 5.17. | F | (CH₃)₂NCO | CH₂CH₂(C₆H₅) | 1:1 | rac. | | 483 |
| 5.18. | F | (CH₃)₂NCO | CH₂CH₂COOH | 1:1 | rac. | 176 | |
| 5.19. | F | (CH₃)₂NCO | CH₂CH₂SCH₃ | 1:1 | rac. | 148 | |
| 5.20. | F | (CH₃)₂NCO | CH₂CH₂SO₂CH₃ | 1:1 | rac. | 175 | |
| 5.21. | F | (CH₃)₂NCO | CH₂CH₂SOCH₃ | 1:1 | rac. | | 469 |
| 5.22. | F | (CH₃)₂NCO | CH₂NH₃⁺Cl⁻ | 4:1 | rac. | 180 | |
| 5.23. | F | (CH₃)₂NCO | CH₂NHCO(2-F-C₆H₄) | 7:3 | rac. | 149 | |
| 5.24. | F | (CH₃)₂NCO | CH₂NHCOCH₂OCH₃ | 7:3 | rac. | 45 | |
| 5.25. | F | (CH₃)₂NCO | CH₂NHCOCH₃ | 4:1 | rac. | 176 | |
| 5.26. | F | (CH₃)₂NCO | CH₂NHCOH | 7:3 | rac. | 105 | |
| 5.27. | F | (CH₃)₂NCO | CH₂NHCON(CH₃)₂ | 3:2 | rac. | 207 | |
| 5.28. | F | (CH₃)₂NCO | CH₂NHCONHCH₃ | 1:0 | rac. | | 465 |
| 5.29. | F | (CH₃)₂NCO | CH₂NHCOOC(CH₃)₃ | 5:1 | rac. | 193 | |
| 5.30. | F | (CH₃)₂NCO | CH₂SCH₃ | 0:1 | rac. | 214 | |
| 5.31. | F | (CH₃)₂NCO | CH₂SCH₃ | 1:0 | rac. | 156 | |
| 5.32. | F | (CH₃)₂NCO | CH₂SO₂CH₃ | 1:9 | rac. | 238 | |
| 5.33. | F | (CH₃)₂NCO | CH₂SO₂CH₃ | 1:0 | rac. | 180 | |
| 5.34. | F | (CH₃)₂NCO | CH₂SOCH₃ | 1:9 | rac. | 171 | |
| 5.35. | F | (CH₃)₂NCO | CH₂SOCH₃ | 1:0 | rac. | 150 | |
| 5.36. | F | (CH₃)₂NCO | cis-CH=CH(C₆H₅) | 3:2 | rac. | | 481 |
| 5.37. | F | (CH₃)₂NCO | Cyclohexyl | 0:1 | rac. | 211 | |
| 5.38. | F | (CH₃)₂NCO | Cyclopentyl | 0:1 | rac. | 154 | |
| 5.39. | F | (CH₃)₂NCO | Cyclopentyl | 3:2 | rac. | 45 | |
| 5.40. | F | (CH₃)₂NCO | Cyclopropyl | 1:1 | rac. | 211 | |
| 5.41. | F | (CH₃)₂NCO | Cyclopropyl | 1:1 | rac. | 189 | |
| 5.42. | F | CH₃CO | 2-(CONHCH₃)-Cyclopropyl | 1:1 | rac. | 235 | |
| 5.43. | F | CH₃CO | CH₂(C₆H₅) | 0:1 | rac. | 208 | |
| 5.44. | F | CH₃CO | CH₂CH₂(C₆H₅) | 1:0 | rac. | | 454 |
| 5.45. | F | CH₃CO | CH₂CH₂(C₆H₅) | 0:1 | rac. | 180 | |
| 5.46. | F | CH₃CO | CH₂CH₂COOH | 1:0 | rac. | 197 | |
| 5.47. | F | CH₃CO | CH₂COOH | 0:1 | rac. | 212 | |
| 5.48. | F | CH₃CO | CH₂NHCOCH₃ | 1:1 | rac. | 197 | |
| 5.49. | F | CH₃CO | CH₂SCH₃ | 0:1 | rac. | 166 | |
| 5.50. | F | CH₃CO | CH₂SCH₃ | 7:3 | rac. | 170 | |
| 5.51. | F | CH₃CO | CH₂SO₂CH₃ | 0:1 | rac. | 229 | |
| 5.52. | F | CH₃CO | CH₂SO₂CH₃ | 7:3 | rac. | 198 | |
| 5.53. | F | CH₃CO | CH₂SOCH₃ | 0:1 | rac. | 196 | |
| 5.54. | F | CH₃CO | CH₂SOCH₃ | 7:3 | rac. | 183 | |
| 5.55. | F | CH₃CO | Cyclohexyl | 0:1 | rac. | 186 | |
| 5.56. | F | CH₃CO | Cyclopentyl | 3:2 | rac. | 186 | |
| 5.57. | F | CH₃CO | cyclopropyl | 0:1 | rac. | 205 | |
| 5.58. | F | CH₃CO | Cyclopropyl | 1:1 | rac. | 180 | |
| 5.59. | F | CH₃HNCO | 2-(CONHCH₃)-Cyclopropyl | 1:1 | rac. | 199 | |
| 5.60. | F | CH₃HNCO | 3-2H-Tetrahydropyranyl | 1:1 | rac. | 195 | |
| 5.61. | F | CH₃HNCO | CH=C(CH₃)₂ | 3:1 | rac. | 182 | |
| 5.62. | F | CH₃HNCO | Cyclohexyl | 1:4 | rac. | 202 | |
| 5.63. | F | CH₃HNCO | Cyclopentyl | 0:1 | rac. | 184 | |
| 5.64. | F | CH₃HNCO | Cyclopentyl | 3:2 | rac. | 205 | |
| 5.65. | F | CH₃HNCO | Cyclopropyl | 1:1 | rac. | 220 | |
| 5.66. | F | CH₃HNCO | Cyclopropyl | 0:1 | rac. | 234 | |
| 5.67. | F | H | 2-(CONHCH₃)-Cyclopropyl | 1:1 | rac. | 235 | |
| 5.68. | F | H | 2-(COOC₂H₅)-Cyclopropyl | 0:1 | rac. | | 420 |
| 5.69. | F | H | 3-(2H-Tetrahydrothiopyranyl) | 0:1 | rac. | 207 | |
| 5.70. | F | H | 3-2H-Tetrahydropyranyl | 5:1 | rac. | 191 | |
| 5.71. | F | H | 3-2H-Tetrahydropyranyl | 1:1 | rac. | 87 | |
| 5.72. | F | H | 4-2H-Tetrahydropyranyl | 1:1 | rac. | 207 | |
| 5.73. | F | H | CH₂(C₆H₅) | 0:1 | rac. | 187 | |
| 5.74. | F | H | CH₂CH₂(C₆H₅) | 0:1 | rac. | 209 | |
| 5.75. | F | H | CH₂CH₂SCH₃ | 1:1 | rac. | 191 | |
| 5.76. | F | H | CH₂NH₃⁺Cl⁻ | 1:1 | rac. | 188 | |
| 5.77. | F | H | CH₂NHCOCH₃ | 3:2 | rac. | 211 | |
| 5.78. | F | H | CH₂NHCON(CH₃)₂ | 0:1 | rac. | | 408 |
| 5.79. | F | H | CH₂NHCON(CH₃)₂ | 1:0 | rac. | | 408 |
| 5.80. | F | H | CH₂NHCOOC(CH₃)₃ | 1:1 | rac. | 144 | |
| 5.81. | F | H | CH₂NHCOOC(CH₃)₃ | 1:0 | rac. | 155 | |
| 5.82. | F | H | CH₂NHSO₂CF₃ | 2:1 | rac. | 187 | |
| 5.83. | F | H | CH₂SCH₃ | 1:9 | rac. | 209 | |
| 5.84. | F | H | CH₂SCH₃ SCH₃ | 7:3 | rac. | 179 | |
| 5.85. | F | H | Cyclohexyl | 1:3 | rac. | 166 | |
| 5.86. | F | H | Cyclopentyl | 0:1 | rac. | 214 | |
| 5.87. | F | H | Cyclopentyl | 2:3 | rac. | 175 | |
| 5.88. | F | H | Cyclopropyl | 0:1 | rac. | | 348 |
| 5.89. | F | H | Cyclopropyl | 1:1 | rac. | 199 | |

### Biologische Wirksamkeit

Die benzoylsubstituierten Serin-Amide der Formel I und deren landwirtschaftlich brauchbaren Salze eignen sich - sowohl als Isomerengemische als auch in Form der reinen Isomeren - als Herbizide. Die Verbindungen der Formel I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa , Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Darüber hinaus können die Verbindungen der Formel I auch in Kulturen, die durch Züchtung einschließlich gentechnischer Methoden gegen Insekten- oder Pilzbefall tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsmittel.

Als inerte Hilfsstoffe kommen im Wesentlichen in Betracht:
Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Im allgemeinen enthalten die Formulierungen etwa von 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile eines Wirkstoffs der Formel I werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs der Formel I enthält.
II. 20 Gewichtsteile eines Wirkstoffs der Formel I werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs der Formel I enthält.
III. 20 Gewichtsteile eines Wirkstoffs der Formel I werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs der Formel I enthält.
IV. 20 Gewichtsteile eines Wirkstoffs der Formel I werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs der Formel I enthält.
V. 3 Gewichtsteile eines Wirkstoffs der Formel I werden mit 97 Gewichtsteilen feinteiligem Kaolin vermischt. Man erhält auf diese Weise ein Stäubemittel, das 3 Gew.-% des Wirkstoffs der Formel I enthält.
VI. 20 Gewichtsteile eines Wirkstoffs der Formel I werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil eines Wirkstoffs der Formel I wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil eines Wirkstoffs der Formel I wird in einer Mischung gelöst, die aus 80 Gewichtsteilen Cyclohexanon und 20 Gewichtsteilen Wettol^{R} EM 31 (= nichtionischer Emulgator auf der Basis von ethoxyliertem Rizinusöl) besteht. Man erhält ein stabiles Emulsionskonzentrat.

Die Applikation der Verbindungen der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Verbindung der Formel I betragen je nach Bekämpfungsziel, Jahreszeit, Zielpflanzen und Wachstumsstadium 0,001 bis 3,0, vorzugsweise 0,01 bis 1,0 kg/ha aktive Substanz (a. S.).

Zur Verbreiterung des Wirkungsspektrums und zur Erzielung synergistischer Effekte können die benzoylsubstituierten Serin-Amide der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3- cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Anwendungsbeispiele

Die herbizide Wirkung der benzoylsubstituierten Serin-Amide der Formel I ließ sich durch die folgenden Gewächshausversuche zeigen:
Als Kulturgefäße dienten Plastikblumentöpfe mit lehmigem Sand mit etwa 3,0 % Humus als Substrat. Die Samen der Testpflanzen wurden nach Arten getrennt eingesät.
Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 1,0 kg/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name |
|---|---|
| Amaranthus retroflexus | Fuchsschwanz |
| Chenopodium album | Weißer Gänsefuß |
| Galium aparine | Klettenlabkraut |
| Polygonum convolvulus | Windenknöterich |
| Setaria viridis | Grüne Borstenhirse |

Bei Aufwandmengen von 1 kg/ha zeigte die Verbindung 3.4 (Tabelle 3) im Nachauflauf eine sehr gute Wirkung gegen die unerwünschten Pflanzen Amaranthus retroflexus, Chenopodium album, Galium aparine und Polygonum convolvulus.

Weiterhin bekämpfte Verbindung 3.11 (Tabelle 3) im Nachauflauf bei Aufwandmengen von 1 kg/ha die Schadpflanzen Amaranthus retroflexus, Chenopodium album, Galium aparine und Polygonum convolvulus sehr gut.

Die Wirkung von Verbindung 3.14 (Tabelle 3) im Nachauflauf bei Aufwandmengen von 1 kg/ha auf die unerwünschten Pflanzen Amaranthus retroflexus, Chenopodium album, Galium aparine und Polygonum convolvulus war sehr gut.

Bei Aufwandmengen von 1 kg/ha zeigte die Verbindung 3.18 (Tabelle 3) im Nachauflauf eine sehr gute Wirkung gegen die unerwünschten Pflanzen Amaranthus retroflexus, Chenopodium album, Galium aparine und Setaria viridis.

Weiterhin bekämpfte Verbindung 3.56 (Tabelle 3) im Nachauflauf bei Aufwandmengen von 1 kg/ha die Schadpflanzen Amaranthus retroflexus, Chenopodium album, Galium aparine und Setaria viridis sehr gut.

## Patentansprüche

1. Benzoylsubstituierte Serin-Amide der Formel I 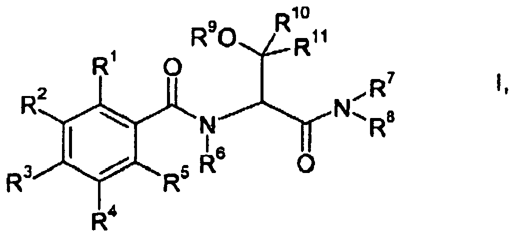 in der die Variablen die folgenden Bedeutungen haben:
R¹ Halogen, Cyano, C₁-C₆-Alkyl, C₁-C₆-Halogenalkyl oder C₁-C₆-Halogenalkoxy:
R², R³, R⁴, R⁵ Wasserstoff, Halogen, Cyano, C₁-C₆-Alkyl, C₁-C₆-Halogenalkyl, C₁-C₆-Alkoxy oder C₁-C₆-Halogenalkoxy;
R⁸, R⁷ Wasserstoff, Hydroxy oder C₁-C₆-Alkoxy:
R⁸ C₁-C₆-Alkyl, C₁-C₄-Cyanoalkyl oder C₁-C₆-Halogenalkyl;
R⁹ Wasserstoff, C₁-C₆-Alkyl, C₃-C₆-Cycloalkyl, C₃-C₆-Alkenyl, C₃-C₆-Alkinyl, C₃-C₆-Halogenalkenyl, C₃-C₆-Halogenalkinyl, Formyl, C₁-C₆-Alkylcarbonyl, C₃-C₆-Cycloalkylcarbonyl, C₂-C₆-Alkenylcarbonyl, C₂-C₆-Alkinylcarbonyl, C₁-C₆-Alkoxycarbonyl, C₃-C₆-Alkenyloxycarbonyl, C₃-C₆-Alkinyloxycarbonyl, C₁-C₆-Alkylaminocarbonyl, C₃-C₆-Alkenylaminocarbonyl, C₃-C₆-Alkinylaminocarbonyl, C₁-C₆-Alkylsulfonylaminocarbonyl, Di-(C₁-C₆-alkyl)-aminocarbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-amino-carbonyl, N-(C₃-C₆-Alkenyl)-N-(C₁-C₆-alkoxy)-aminocarbonyl, N-(C₃-C₆-Alklnyl)-N-(C₁-C₆-alkoxy)-aminocarbonyl, Di-(C₁-C₆-alkyl)-aminothiocarbonyl, (C₁-C₆-Alkyl)cyanoimino, (Amino)cyanoimino, [(C₁-C₆-alkyl)amino]cyanoimino, [Di(C₁-C₆-alkyl)aminolcyanoimino, C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl, N-(Di-C₁-C₆-alkylamino)-imino-C₁-C₆-alkyl oder Tri-C₁-C₄-alkylsilyl,
wobei die genannten Alkyl-, Cycloalkyl- und Alkoxyreste partiell oder vollständig halogeniert sein können und/oder eine bis drei der folgenden Gruppen tragen können: Cyano, Hydroxy, C₃-C₆-Cycloalkyl, C₁-C₈-Alkoxy-C₁-C₄-alkyl, C₁-C₄-Alkoxy-C₁-C₄-alkoxy-C₁-C₄-alkyl, C₁-C₄-Alkoxy, C₁-C₄-Alkylthio, Di-(C₁-C₄-alkyl)-amino, C₁-C₄-Alkyl-C₁-C₆-alkoxycarbonylamino, C₁-C₄-Alkylcarbonyl, Hydroxycarbonyl, C₁-C₄-Alkoxycarbonyl, Aminocarbonyl, C₁-C₄-Alkylaminocarbonyl, Di(C₁-C₄-alkyl)aminocarbonyl oder C₁-C₄-Alkylcarbonyloxy;
Phenyl, Phenyl-C₁-C₆-alkyl, Phenylcarbonyl, Phenylcarbonyl-C₁-C₆-alkyl, Phenoxycarbonyl, Phenylaminocarbonyl, Phenylsulfonylaminocarbonyl, N-(C₁-C₆-Alkyl)-N-(phenyl)-aminocarbonyl, Phenyl-C₁-C₆-alkylcarbonyl, wobei der Phenylrest partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy; oder
SO₂R¹²;
R¹⁰ Wasserstoff oder C₁-C₆-Alkyl;
R¹¹ C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₁-C₆-Cyanoalkyl, C₂-C₃-Cyanoalkenyl, C₂-C₆-Cyanoalkinyl, C₁-C₆-Hydroxyalkyl, C₂-C₆-Hydroxyalkenyl, C₂-C₆-Hydroxyalkinyl, C₃-C₆-Cycloalkyl, C₃-C₆-Cycloalkenyl, 3- bis 6-gliedriges Heterocyclyl, 3- bis 6-gliedriges Heterocyclyl-C₁-C₄-alkyl,
wobei die voranstehen genannten Cycloalkyl, Cycloalkenyl oder 3-bis 6-gliedrigen Heterocyclylreste partiell oder vollständig halogeniert sein können und/oder ein bis drei Reste aus der Gruppe Oxo, Cyano, Nitro, C₁-C₆-Alkyl, C₁-C₆-Halogenalkyl, Hydroxy, C₁-C₆-Alkoxy, C₁-C₆-Halogenalkoxy, Hydroxycarbonyl, C₁-C₆-Alkoxycarbonyl, Hydroxycarbonyl-C₁-C₆-alkoxy, C₁-C₆-Alkoxycarbonyl-C₁-C₆-alkoxy, Amino, C₁-C₆-Alkylamino, Di(C₁-C₆-alkyl)amino, C₁-C₆-Alkylsulfonylamino, C₁-C₆-Halogenalkylsulfonylamino, Aminocarbonylamino, (C₁-C₆-Alkylamino)carbonylamino, Di-(C₁-C₆-alkyl)-aminocarbonylamino, Aryl und Aryl(C₁-C₆-alkyl) tragen können;
C₁-C₆-Alkoxy-C₁-C₄-alkyl, C₂-C₆-Alkenyloxy-C₁-C₄-alkyl, C₂-C₆-Alkinyloxy-C₁-C₄-alkyl, C₁-C₆-Halogenalkoxy-C₁-C₄-alkyl, C₂-C₆-Halogenalkenyloxy-C₁-C₄-alkyl, C₂-C₆-Halogenalkinyloxy-C₁-C₄-alkyl, C₁-C₆-Alkoxy-C₁-C₄-alkoxy-C₁-C₄-alkyl, C₁-C₆-Alkylthio-C₁-C₄-alkyl, C₂-C₆-Alkenylthio-C₁-C₄-alkyl, C₂-C₆-Alkinylthio-C₁-C₄-alkyl, C₁-C₆-Halogenalkyl-C₁-C₄-thioalkyl, C₂-C₆-Halogenalkenyl-C₁-C₄-thioalkyl, C₂-C₆-Halogenalkinyl-C₁-C₄-thioalkyl, C₁-C₆-Alkylsulfinyl-C₁-C₄-alkyl, C₁-C₆-Halogenalkylsulfinyl-C₁-C₄-alkyl, C₁-C₆-Alkylsulfonyl-C₁-C₄-alkyl, C₁-C₆-Halogenalkylsulfonyl-C₁-C₄-alkyl, Amino-C₁-C₄-alkyl, C₁-C₆-Alkylamino-C₁-C₄-alkyl, Di(C₁-C₆-Alkyl)amino-C₁-C₄-alkyl, C₁-C₆-Alkylsulfonylamino-C₁-C₄-alkyl, C₁-C₆-Alkylsulfonyl-(C₁-C₆-alkylamino)-C₁-C₄-alkyl, C₁-C₆-Alkylcarbonyl, Hydroxycarbonyl, C₁-C₆-Alkoxycarbonyl, Aminocarbonyl, C₁-C₆-Alkylaminocarbonyl, Di(C₁-C₆-alkyl)aminocarbonyl, C₁-C₆-Alkylcarbonyl-C₁-C₆-alkyl, Hydroxycarbonyl-C₁-C₄-alkyl, C₁-C₆-Alkoxycarbonyl-C₁-C₄-alkyl, C₁-C₆-Halogenalkoxycarbonyl-C₁-C₄-alkyl, C₁-C₆-Alkylcarbonyloxy-C₁-C₄-alkyl, Aminocarbonyl-C₁-C₄-alkyl, C₁-C₆-Alkylaminocarbonyl-C₁-C₄-alkyl, Di(C₁-C₆-Alkyl)aminocarbonyl-C₁-C₄-alkyl, Formylamino-C₁-C₄-alkyl, C₁-C₆-Alkoxycarbonylamino-C₁-C₄-alkyl, C₁-C₆-Alkylcarbonylamino-C₁-C₄-alkyl, C₁-C₆-Alkylcarbonyl-(C₁-C₆-alkylamino)-C₁-C₄-alkyl, [(C₁-C₆-alkyl)aminocarbonyloxy]-C₁-C₄-alkyl, [Di(C₁-C₆-alkyl)aminocarbonyloxy]C₁-C₄-alkyl, {Di[di(C₁-C₆-alkyl)aminolcarbonyloxy}-C₁-C₄-alkyl, [(C₁-C₆-alkylamino)carbonylamino]C₁-C₄-alkyl, [Di(C₁-C₆-alkyl)aminocarbonylamino]C₁-C₄-alkyl;
Phenyl-C₁-C₄-alkyl, Phenyl-C₂-C₄-alkenyl, Phenyl-C₂-C₄-alkinyl, Phenyl-C₁-C₄-halogenalkyl, Phenyl-C₂-C₄-halogenalkenyl, Phenyl-C₂-C₄-halogenalkinyl, Phenyl-C₁-C₄-hydroxyalkyl, Phenyl-C₂-C₄-hydroxyalkenyl, Phenyl-C₂-C₄-hydroxyalkinyl, Phenylcarbonyl-C₁-C₄-alkyl, Phenylcarbony-lamino-C₁-C₄-alkyl, Phenylcarbonyloxy-C₁-C₄-alkyl, Phenyloxycarbonyl-C₁-C₄-alkyl, Phenyloxy-C₁-C₄-alkyl, Phenylthio-C₁-C₄-alkyl, Phenylsulfinyl-C₁-C₄-alkyl, Phenylsulfonyl-C₁-C₄-alkyl,
Heteroaryl-C₁-C₄-alkyl, Heteroaryl-C₂-C₄-alkenyl, Heteroaryl-C₂-C₄-alkinyl, Heteroaryl-C₁-C₄-halogenalkyl, Heteroaryl-C₂-C₄-halogenalkenyl, Heteroaryl-C₂-C₄-halogenalkinyl, Heteroaryl-C₁-C₄-hydroxyalkyl, Heteroaryl-C₂-C₄-hydroxyalkenyl, Heteroaryl-C₂-C₄-hydroxyalkinyl, Heteroarylcarbonyl-C₁-C₄-alkyl, Heteroarylcarbonyloxy-C₁-C₄-alkyl, Heteroaryloxycarbonyl-C₁-C₄-alkyl, Heteroaryloxy-C₁-C₄-alkyl, Heteroarylthio-C₁-C₄-alkyl, Heteroarylsulfinyl-C₁-C₄-alkyl, Heteroarylsulfonyl-C₁-C₄-alkyl,
wobei die vorstehend genannten Phenyl- und Heteroarylreste partiell oder vollständig halogeniert sein können und/oder ein bis drei Reste aus der Gruppe Cyano, Nitro, C₁-C₆-Alkyl, C₁-C₆-Halogenalkyl, Hydroxy, -C₁-C₆-Alkoxy, C₁-C₆-Halogenalkoxy, Hydroxycarbonyl, C₁-C₆-Alkoxycarbonyl, Hydroxycarbonyl-C₁-C₆-alkoxy, C₁-C₆-Alkoxycarbonyl-C₁-C₆-alkoxy, Amino, C₁-C₆-Alkylamino, Di(C₁-C₆-alkyl)-amino, C₁-C₆-Alkylsulfonylamino, C₁-C₆-Halogenalkylsulfonylamino, (C₁-C₆-Alkylamino)carbonylamino, Di-(C₁-C₆-alkyl)-aminocarbonylamino, Aryl und Aryl(C₁-C₆-alkyl) tragen können;
R¹² C₁-C₆-Alkyl, C₁-C₆-Halogenalkyl oder Phenyl,
wobei der Phenylrest partiell oder vollständig halogeniert sein kann und/oder eine bis drei der folgenden Gruppen tragen kann: C₁-C₆-Alkyl, C₁-C₆-Halogen-alkyl oder C₁-C₆-Alkoxy;
sowie deren landwirtschaftlich brauchbaren Salze.

2. Benzoylsubstituierte Serin-Amide der Formel I gemäß Anspruch 1, wobei R¹ für Halogen oder C₁-C₆-Halogenalkyl steht.

3. Benzoylsubstituierte Serin-Amide der Formel I gemäß Ansprüchen 1 oder 2, wobei R² und R³ unabhängig voneinander für Wasserstoff, Halogen oder C₁-C₆-Halogenalkyl stehen.

4. Benzoylsubstituierte Serin-Amide der Formel I gemäß Ansprüchen 1 bis 3, wobei R⁴, R⁵, R⁶, R⁷ und R¹⁰ für Wasserstoff stehen.

5. Benzoylsubstituierte Serin-Amide der Formel I gemäß Ansprüchen 1 bis 4, wobei R¹¹ für C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₁-C₆-Cyanoalkyl, C₁-C₆-Hydroxyalkyl, C₂-C₆-Hydroxyalkenyl, C₂-C₆-Hydroxyalkinyl, C₃-C₆-Cycloalkyl, C₃-C₆-Cycloalkenyl, 3- bis 6-gliedrige Heterocyclyl,
wobei die voranstehend genannten Cycloalkyl, Cycloalkenyl oder 3- bis 6-gliedrigen Heterocyclylreste partiell oder vollständig halogeniert sein können und/oder einen bis drei Rest aus der Gruppe Oxo, C₁-C₆-Alkyl, C₁-C₆-Halogenalkyl, Hydroxycarbonyl und C₁-C₆-Alkoxycarbonyl tragen können,
C₁-C₆-Alkoxy-C₁-C₄-alkyl, C₁-C₆-Halogenalkoxy-C₁-C₄-alkyl, C₁-C₆-Alkoxy-C₁-C₄-alkoxy-C₁-C₄-alkyl, C₁-C₆-Alkylthio-C₁-C₄-alkyl, C₁-C₆-Alkylsulfonylamino-C₁-C₄-alkyl, Hydroxycarbonyl, C₁-C₆-Alkoxycarbonyl, Hydroxycarbonyl-C₁-C₄-alkyl, C₁-C₆-Alkoxycarbonyl-C₁-C₄-alkyl, C₁-C₆-Halogenalkoxycarbonyl-C₁-C₄-alkyl, C₁-C₆-Alkylcarbonyloxy-C₁-C₄-alkyl, C₁-C₆-Alkylcarbonylamino-C₁-C₄-alkyl, Di(C₁-C₆-Alkyl)carbonylamino-C₁-C₄-alkyl, Di(C₁-C₆-alkyl)aminocarbonylamino-C₁-C₄-alkyl, [(C₁-C₆-alkyl)aminocarbonyl]amino-C₁-C₄-alkyl, [Di(C₁-C₆-alkyl)amino-carbonyloxy]C₁-C₄-alkyl, Formylamino-C₁-C₄-alkyl,
Phenyl-C₁-C₄-alkyl, Phenyl-C₂-C₄-alkenyl, Phenyl-C₂-C₄-alkinyl, Phenyl-C₁-C₄-halogenalkyl, Phenyl-C₂-C₄-halogenalkenyl, Phenyl-C₁-C₄-hydroxyalkyl, Phenyloxy-C₁-C₄-alkyl, Phenylthio-C₁-C₄-alkyl, Phenylsulfinyl-C₁-C₄-alkyl, Phenylsulfonyl-C₁-C₄-alkyl,
Heteroaryl-C₁-C₄-alkyl, Heteroaryl-C₁-C₄-hydroxyalkyl, Heteroaryloxy-C₁-C₄-alkyl, Heteroarylthio-C₁-C₄-alkyl, Heteroarylsulfinyl-C₁-C₄-alkyl, Hetetoarylsulfonyl-C₁-C₄-alkyl,
wobei die vorstehend genannten Phenyl- und Heteroarylreste oder vollständig halogeniert sein können und/oder ein bis drei Reste aus der Gruppe Cyano, Nitro, C₁-C₆-Alkyl, C₁-C₆-Halogenalkyl, Hydroxy, C₁-C₆-Alkoxy, C₁-C₆-Halogenalkoxy, Hydroxycarbonyl, C₁-C₆-Alkoxycarbonyl, Hydroxycarbonyl-C₁-C₆-alkoxy, C₁-C₆-Alkylsulfonylamino und C₁-C₆-Halogenalkylsulfonylamino tragen können;
steht.

6. Verfahren zur Herstellung von benzoylsubstituierten Serin-Amiden der Formel I gemäß Anspruch 1, **dadurch gekennzeichnet dass** Serinderivate der Formel V 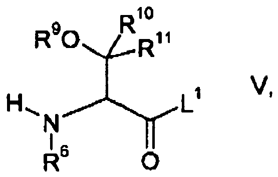 wobei R⁶, R⁹, R¹⁰ und R¹¹ die unter Anspruch 1 genannten Bedeutungen haben und L¹ für Hydroxy oder C₁-C₆-Alkoxy steht,
mit Benzoesäure(derivate)n der Formel IV 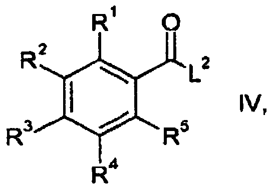 wobei R¹ bis R⁵ die unter Anspruch 1 genannten Bedeutungen haben und L² für Hydroxy, Halogen, C₁-C₆-Alkylcarbonyl, C₁-C₆-Alkoxycarbonyl, C₁-C₄-Alkylsulfonyl, Phosphoryl oder Isoureyl steht,
zu entsprechenden Benzoylderivaten der Formel III 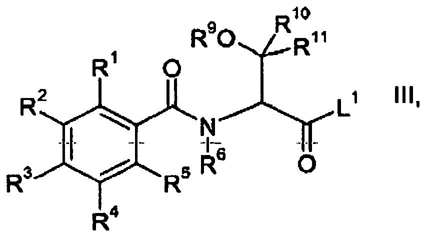 wobei R¹ bis R³ und R⁹ bis R¹¹ die unter Anspruch 1 genannten Bedeutungen haben und L¹ für Hydroxy oder C₁-C₆-Alkoxy steht,
und anschließend die erhaltenen Benzoylderivate der Formel III mit einem Amin der Formel II
HNR⁷R⁸ II,
wobei R⁷ und R⁸ die unter Anspruch 1 genannten Bedeutungen haben, umgesetzt werden.

7. Verfahren zur Herstellung von benzoylsubstituierten Serin-Amiden der Formel I gemäß Anspruch 6, wobei R⁹ und R¹⁰ für Wasserstoff stehen, **dadurch gekennzeichnet dass** Benzoylderivate der Formel III wobei R⁹ und R¹⁰ für Wasserstoff stehen, durch Acylierung von Ketoverbindungen der Formel XIII 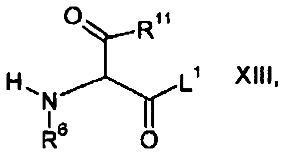 wobei R⁶ und R¹¹ die unter Anspruch 1 genannten Bedeutungen haben und L' für Hydroxy oder C₁-C₆-Alkoxy steht,
mit Benzoesäure(derivate)n der Formel IV zu N-Acyl-Ketoverbindungen der Formel XII 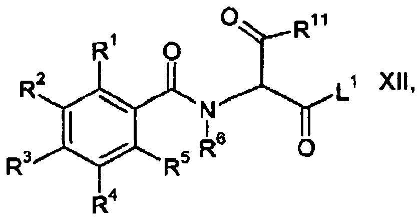 wobei R¹ bis R⁶ sowie R¹¹ die unter Anspruch 1 genannten Bedeutungen haben und L¹ für Hydroxy oder C₁-C₆-Alkoxy steht,
und anschließender Reduktion der Ketogruppe hergestellt werden.

8. Benzoylderivate der Formel III 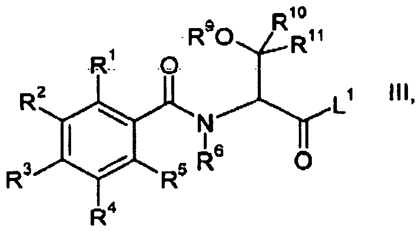 wobei R¹ für Fluor, Chlor oder CF₃, und R⁵ für Wasserstoff steht, sowie R² bis R⁴, R⁸ und R⁹ bis R¹¹ die unter Anspruch 1 genannten Bedeutungen haben, und L' für Hydroxy oder C₁-C₆-Alkoxy steht.

9. Mittel, enthaltend eine herbizid wirksame Menge mindestens eines benzoylsubstituierten Serin-Amids der Formel I oder eines landwirtschaftlich brauchbaren Salzes von I gemäß den Ansprüchen 1 bis 5 und für die Formulierung von Pflanzenschutzmitteln übliche Hilfsmittel.

10. Verfahren zur Herstellung von Mitteln gemäß Anspruch 9, **dadurch gekennzeichnet, daß** man eine herbizid wirksame Menge mindestens eines benzoylsubstituierten Serin-Amids der Formel I oder eines landwirtschaftlich brauchbaren Salzes von I gemäß den Ansprüchen 1 bis 5 und für die Formulierung von Pflanzenschutzmitteln übliche Hilfsmittel mischt.

11. Verfahren zur Bekämpfung von unerwünschtem Pflanzenwuchs, **dadurch gekennzeichnet, daß** man eine herbizid wirksame Menge mindestens eines benzoylsubstituierten Serin-Amids der Formel I oder eines landwirtschaftlich brauchbaren Salzes von I gemäß den Ansprüchen 1 bis 5 auf Pflanzen, deren Lebensraum und/oder auf Samen einwirken läßt.

12. Verwendung der benzoylsubstituierten Serin-Amide der Formel I und deren landwirtschaftlich brauchbaren Salze gemäß den Ansprüchen 1 bis 5 als Herbizide.

## Claims

1. A benzoyl-substituted serineamide of the formula I 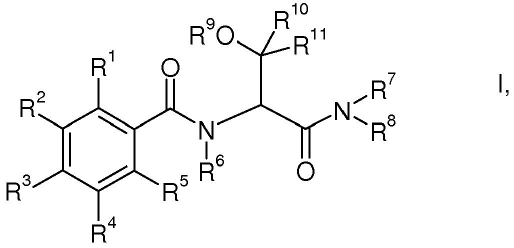 in which the variables are as defined below:
R¹ is halogen, cyano, C₁-C₆-alkyl, C₁-C₆-haloalkyl or C₁-C₆-haloalkoxy;
R², R³, R⁴, R⁵ are hydrogen, halogen, cyano, C₁-C₆-alkyl, C₁-C₆-haloalkyl, C₁-C₆-alkoxy or C₁-C₆-haloalkoxy;
R⁶, R⁷ are hydrogen, hydroxyl or C₁-C₆-alkoxy;
R⁸ is C₁-C₆-alkyl, C₁-C₄-cyanoalkyl or C₁-C₆-haloalkyl;
R⁹ is hydrogen, C₁-C₆-alkyl, C₃-C₆-cycloalkyl, C₃-C₆-alkenyl, C₃-C₆-alkynyl, C₃-C₆-haloalkenyl, C₃-C₆-haloalkynyl, formyl, C₁-C₆-alkylcarbonyl, C₃-C₆-cycloalkylcarbonyl, C₂-C₆-alkenylcarbonyl, C₂-C₆-alkynylcarbonyl, C₁-C₆-alkoxycarbonyl, C₃-C₆-alkenyloxycarbonyl, C₃-C₆-alkynyloxycarbonyl, C₁-C₆-alkylaminocarbonyl, C₃-C₆-alkenylaminocarbonyl, C₃-C₆-alkynylaminocarbonyl, C₁-C₆-alkylsulfonylaminocarbonyl, di(C₁-C₆-alkyl)-aminocarbonyl, N-(C₃-C₆-alkenyl)-N-(C₁-C₆-alkyl)aminocarbonyl, N-(C₃-C₆-alkynyl)-N-(C₁-C₆-alkyl)aminocarbonyl, N-(C₁-C₆-alkoxy)-N-(C₁-C₆-alkyl)aminocarbonyl, N-(C₃-C₆-alkenyl)-N-(C₁-C₆-alkoxy)aminocarbonyl, N-(C₃-C₆-alkynyl)-N-(C₁-C₆-alkoxy)aminocarbonyl, di(C₁-C₆-alkyl)aminothiocarbonyl, (C₁-C₆-alkyl)cyanoimino, (amino)cyanoimino, [(C₁-C₆-alkyl)amino]cyanoimino, [di(C₁-C₆-alkyl)amino]cyanoimino, C₁-C₆-alkylcarbonyl-C₁-C₆-alkyl, C₁-C₆-alkoxyimino-C₁-C₆-alkyl, N-(C₁-C₆-alkylamino)imino-C₁-C₆-alkyl, N-(di-C₁-C₆-alkylamino)imino-C₁-C₆-alkyl or tri-C₁-C₄-alkylsilyl, where the alkyl, cycloalkyl and alkoxy radicals mentioned may be partially or fully halogenated and/or may carry one to three of the following groups: cyano, hydroxyl, C₃-C₆-cycloalkyl, C₁-C₆-alkoxy-C₁-C₄-alkyl, C₁-C₄-alkoxy-C₁-C₄-alkoxy-C₁-C₄-alkyl, C₁-C₄-alkoxy, C₁-C₄-alkylthio, di(C₁-C₄-alkyl)amino, C₁-C₄-alkyl-C₁-C₆-alkoxycarbonylamino, C₁-C₄-alkylcarbonyl, hydroxycarbonyl, C₁-C₄-alkoxycarbonyl, aminocarbonyl, C₁-C₄-alkylaminocarbonyl, di(C₁-C₄-alkyl)aminocarbonyl or C₁-C₄-alkylcarbonyloxy;
phenyl, phenyl-C₁-C₆-alkyl, phenylcarbonyl, phenylcarbonyl-C₁-C₆-alkyl, phenoxycarbonyl, phenylaminocarbonyl, phenylsulfonylaminocarbonyl, N-(C₁-C₆-alkyl)-N-(phenyl)aminocarbonyl, phenyl-C₁-C₆-alkylcarbonyl,
where the phenyl radical may be partially or fully halogenated and/or may carry one to three of the following groups: nitro, cyano, C₁-C₄-alkyl, C₁-C₄-haloalkyl, C₁-C₄-alkoxy or C₁-C₄-haloalkoxy; or SO₂R¹²;
R¹⁰ is hydrogen or C₁-C₆-alkyl;
R¹¹ is C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₁-C₆-cyanoalkyl, C₂-C₆-cyanoalkenyl, C₂-C₆-cyanoalkynyl, C₁-C₆-hydroxyalkyl, C₂-C₆-hydroxyalkenyl, C₂-C₆-hydroxyalkynyl, C₃-C₆-cycloalkyl, C₃-C₆-cycloalkenyl, 3- to 6-membered heterocyclyl, 3- to 6-membered heterocyclyl-C₁-C₄-alkyl,
where the cycloalkyl, cycloalkenyl or 3- to 6-membered heterocyclyl radicals mentioned above may be partially or fully halogenated and/or may carry one to three radicals from the group consisting of oxo, cyano, nitro, C₁-C₆-alkyl, C₁-C₆-haloalkyl, hydroxyl, C₁-C₆-alkoxy, C₁-C₆-haloalkoxy, hydroxycarbonyl, C₁-C₆-alkoxycarbonyl, hydroxycarbonyl-C₁-C₆-alkoxy, C₁-C₆-alkoxycarbonyl-C₁-C₆-alkoxy, amino, C₁-C₆-alkylamino, di(C₁-C₆-alkyl)amino, C₁-C₆-alkylsulfonylamino, C₁-C₆-haloalkylsulfonylamino, aminocarbonylamino, (C₁-C₆-alkylamino)carbonylamino, di(C₁-C₆-alkyl)-aminocarbonylamino, aryl and aryl(C₁-C₆-alkyl); C₁-C₆-alkoxy-C₁-C₄-alkyl, C₂-C₆-alkenyloxy-C₁-C₄-alkyl, C₂-C₆-alkynyloxy-C₁-C₄-alkyl, C₁-C₆-haloalkoxy-C₁-C₄-alkyl, C₂-C₆-haloalkenyloxy-C₁-C₄-alkyl, C₂-C₆-haloalkynyloxy-C₁-C₄-alkyl, C₁-C₆-alkoxy-C₁-C₄-alkoxy-C₁-C₄-alkyl, C₁-C₆-alkylthio-C₁-C₄-alkyl, C₂-C₆-alkenylthio-C₁-C₄-alkyl, C₂-C₆-alkynylthio-C₁-C₄-alkyl, C₁-C₆-haloalkyl-C₁-C₄-thioalkyl, C₂-C₆-haloalkenyl-C₁-C₄-thioalkyl, C₂-C₆-haloalkynyl-C₁-C₄-thioalkyl, C₁-C₆-alkylsulfinyl-C₁-C₄-alkyl, C₁-C₆-haloalkylsulfinyl-C₁-C₄-alkyl, C₁-C₆-alkylsulfonyl-C₁-C₄-alkyl, C₁-C₆-haloalkylsulfonyl-C₁-C₄-alkyl, amino-C₁-C₄-alkyl, C₁-C₆-alkylamino-C₁-C₄-alkyl, di (C₁-C₆-alkyl) amino-C₁-C₄-alkyl, C₁-C₆-alkylsulfonylamino-C₁-C₄-alkyl, C₁-C₆-alkylsulfonyl-(C₁-C₆-alkylamino)-C₁-C₄-alkyl, C₁-C₆-alkylcarbonyl, hydroxycarbonyl, C₁-C₆-alkoxycarbonyl, aminocarbonyl, C₁-C₆-alkylaminocarbonyl, di(C₁-C₆-alkyl)aminocarbonyl, C₁-C₆-alkylcarbonyl-C₁-C₆-alkyl, hydroxycarbonyl-C₁-C₄-alkyl, C₁-C₆-alkoxycarbonyl-C₁-C₄-alkyl, C₁-C₆-haloalkoxycarbonyl-C₁-C₄-alkyl, C₁-C₆-alkylcarbonyloxy-C₁-C₄-alkyl, aminocarbonyl-C₁-C₄-alkyl, C₁-C₆-alkylaminocarbonyl-C₁-C₄-alkyl, di(C₁-C₆-alkyl)aminocarbonyl-C₁-C₄-alkyl, formylamino-C₁-C₄-alkyl, C₁-C₆-alkoxycarbonylamino-C₁-C₄-alkyl, C₁-C₆-alkylcarbonylamino-C₁-C₄-alkyl, C₁-C₆-alkylcarbonyl-(C₁-C₆-alkylamino)-C₁-C₄-alkyl, [(C₁-C₆-alkyl)aminocarbonyloxy]-C₁-C₄-alkyl, [di(C₁-C₆-alkyl)aminocarbonyloxy]C₁-C₄-alkyl, {di[di(C₁-C₆-alkyl) amino]carbonyloxy}C₁-C₄-alkyl, [(C₁-C₆-alkylamino)carbonylamino]-C₁-C₄-alkyl, [di(C₁-C₆-alkyl)aminocarbonylamino]C₁-C₄-alkyl; phenyl-C₁-C₄-alkyl, phenyl-C₂-C₄-alkenyl, phenyl-C₂-C₄-alkynyl, phenyl-C₁-C₄-haloalkyl, phenyl-C₂-C₄-haloalkenyl, phenyl-C₂-C₄-haloalkynyl, phenyl-C₁-C₄-hydroxyalkyl, phenyl-C₂-C₄-hydroxyalkenyl, phenyl-C₂-C₄-hydroxyalkynyl, phenylcarbonyl-C₁-C₄-alkyl, phenylcarbonylamino-C₁-C₄-alkyl, phenylcarbonyloxy-C₁-C₄-alkyl, phenyloxycarbonyl-C₁-C₄-alkyl, phenyloxy-C₁-C₄-alkyl, phenylthio-C₁-C₄-alkyl, phenylsulfinyl-C₁-C₄-alkyl, phenylsulfonyl-C₁-C₄-alkyl, heteroaryl-C₁-C₄-alkyl, heteroaryl-C₂-C₄-alkenyl, heteroaryl-C₂-C₄-alkynyl, heteroaryl-C₁-C₄-haloalkyl, heteroaryl-C₂-C₄-haloalkenyl, heteroaryl-C₂-C₄-haloalkynyl, heteroaryl-C₁-C₄-hydroxyalkyl, heteroaryl-C₂-C₄-hydroxyalkenyl, heteroaryl-C₂-C₄-hydroxyalkynyl, heteroarylcarbonyl-C₁-C₄-alkyl, heteroarylcarbonyloxy-C₁-C₄-alkyl, heteroaryloxycarbonyl-C₁-C₄-alkyl, heteroaryloxy-C₁-C₄-alkyl, heteroarylthio-C₁-C₄-alkyl, heteroarylsulfinyl-C₁-C₄-alkyl, heteroarylsulfonyl-C₁-C₄-alkyl,
where the phenyl and heteroaryl radicals mentioned above may be partially or fully halogenated and/or may carry one to three radicals from the group consisting of cyano, nitro, C₁-C₆-alkyl, C₁-C₆-haloalkyl, hydroxy, C₁-C₆-alkoxy, C₁-C₆-haloalkoxy, hydroxycarbonyl, C₁-C₆-alkoxycarbonyl, hydroxycarbonyl-C₁-C₆-alkoxy, C₁-C₆-alkoxycarbonyl-C₁-C₆-alkoxy, amino, C₁-C₆-alkylamino, di(C₁-C₆-alkyl)amino, C₁-C₆-alkylsulfonylamino, C₁-C₆-haloalkylsulfonylamino, (C₁-C₆-alkylamino)-carbonylamino, di(C₁-C₆-alkyl)aminocarbonylamino, aryl and aryl(C₁-C₆-alkyl);
R¹² is C₁-C₆-alkyl, C₁-C₆-haloalkyl or phenyl, where the phenyl radical may be partially or fully halogenated and/or may carry one to three of the following groups: C₁-C₆-alkyl, C₁-C₆-haloalkyl or C₁-C₆-alkoxy;
or an agriculturally useful salt thereof.

2. The benzoyl-substituted serineamide of the formula I according to claim 1 where R¹ is halogen or C₁-C₆-haloalkyl.

3. The benzoyl-substituted serineamide of the formula I according to claim 1 or 2 where R² and R³ independently of one another are hydrogen, halogen or C₁-C₆-haloalkyl.

4. The benzoyl-substituted serineamide of the formula I according to any of claims 1 to 3 where R⁴, R⁵, R⁶, R⁷ and R¹⁰ are hydrogen.

5. The benzoyl-substituted serineamide of the formula I according to any of claims 1 to 4 where R¹¹ is C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₁-C₆-cyanoalkyl, C₁-C₆-hydroxyalkyl, C₂-C₆-hydroxyalkenyl, C₂-C₆-hydroxyalkynyl, C₃-C₆-cycloalkyl, C₃-C₆-cycloalkenyl, 3- to 6-membered heterocyclyl,
where the cycloalkyl, cycloalkenyl or 3- to 6-membered heterocyclyl radicals mentioned above may be partially or fully halogenated and/or may carry one to three radicals from the group consisting of oxo, C₁-C₆-alkyl, C₁-C₆-haloalkyl, hydroxycarbonyl and C₁-C₆-alkoxycarbonyl,
C₁-C₆-alkoxy-C₁-C₄-alkyl, C₁-C₆-haloalkoxy-C₁-C₄-alkyl, C₁-C₆-alkoxy-C₁-C₄-alkoxy-C₁-C₄-alkyl, C₁-C₆-alkylthio-C₁-C₄-alkyl, C₁-C₆-alkylsulfonylamino-C₁-C₄-alkyl, hydroxycarbonyl, C₁-C₆-alkoxycarbonyl, hydroxycarbonyl-C₁-C₄-alkyl, C₁-C₆-alkoxycarbonyl-C₁-C₄-alkyl, C₁-C₆-haloalkoxycarbonyl-C₁-C₄-alkyl, C₁-C₆-alkylcarbonyloxy-C₁-C₄-alkyl, C₁-C₆-alkylcarbonylamino-C₁-C₄-alkyl, di (C₁-C₆-alkyl)carbonylamino-C₁-C₄-alkyl, [di(C₁-C₆-alkyl)aminocarbonylamino]-C₁-C₄-alkyl, [(C₁-C₆-alkyl)aminocarbonyl]amino-C₁-C₄-alkyl, [di(C₁-C₆-alkyl)aminocarbonyloxy]-C₁-C₄-alkyl, formylamino-C₁-C₄-alkyl,
phenyl-C₁-C₄-alkyl, phenyl-C₂-C₄-alkenyl, phenyl-C₂-C₄-alkynyl, phenyl-C₁-C₄-haloalkyl, phenyl-C₂-C₄-haloalkenyl, phenyl-C₁-C₄-hydroxyalkyl, phenyloxy-C₁-C₄-alkyl, phenylthio-C₁-C₄-alkyl, phenylsulfinyl-C₁-C₄-alkyl, phenylsulfonyl-C₁-C₄-alkyl, heteroaryl-C₁-C₄-alkyl, heteroaryl-C₁-C₄-hydroxyalkyl, heteroaryloxy-C₁-C₄-alkyl, heteroarylthio-C₁-C₄-alkyl, heteroarylsulfinyl-C₁-C₄-alkyl, heteroarylsulfonyl-C₁-C₄-alkyl,
where the phenyl and heteroaryl radicals mentioned above may be partially or fully halogenated and/or may carry one to three radicals from the group consisting of cyano, nitro, C₁-C₆-alkyl, C₁-C₆-haloalkyl, hydroxy, C₁-C₆-alkoxy, C₁-C₆-haloalkoxy, hydroxycarbonyl, C₁-C₆-alkoxycarbonyl, hydroxycarbonyl-C₁-C₆-alkoxy, C₁-C₆-alkylsulfonylamino and C₁-C₆-haloalkylsulfonylamino.

6. A process for preparing benzoyl-substituted serineamides of the formula I according to claim 1, wherein serine derivatives of the formula V 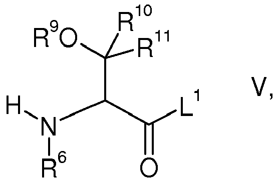 where R⁶, R⁹, R¹⁰ and R¹¹ are as defined in claim 1 and L¹ is hydroxyl or C₁-C₆-alkoxy
are reacted with benzoic acids/benzoic acid derivatives of the formula IV 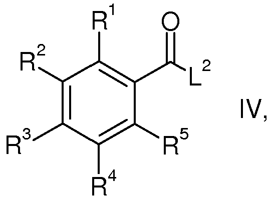 where R¹ to R⁵ are as defined in claim 1 and L² is hydroxyl, halogen, C₁-C₆-alkylcarbonyl, C₁-C₆-alkoxycarbonyl, C₁-C₄-alkylsulfonyl, phosphoryl or isoureyl
to give the corresponding benzoyl derivatives of the formula III 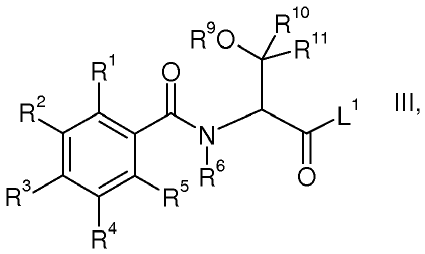 where R¹ to R⁶ and R⁹ to R¹¹ are as defined in claim 1 and L¹ is hydroxyl or C₁-C₆-alkoxy and the resulting benzoyl derivatives of the formula III are then reacted with an amine of the formula II
HNR⁷R⁸ II,
where R⁷ and R⁸ are as defined in claim 1.

7. A process for preparing benzoyl-substituted serineamides of the formula I according to claim 6 where R⁹ and R¹⁰ are hydrogen, wherein benzoyl derivatives of the formula III where R⁹ and R¹⁰ are hydrogen are prepared by acylation of keto compounds of the formula XIII 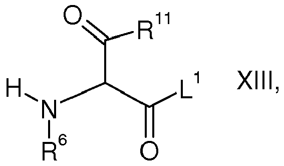 where R⁶ and R¹¹ are as defined in claim 1 and L¹ is hydroxyl or C₁-C₆-alkoxy
with benzoic acids/benzoic acid derivatives of the formula IV to give N-acyl keto compounds of the formula XII 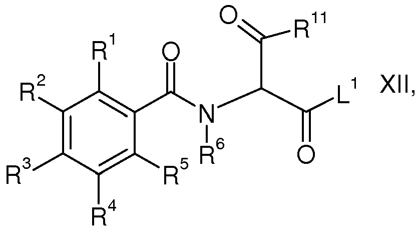 where R¹ to R⁶ and R¹¹ are as defined in claim 1 and L¹ is hydroxyl or C₁-C₆-alkoxy
and subsequent reduction of the keto group.

8. A benzoyl derivative of the formula III 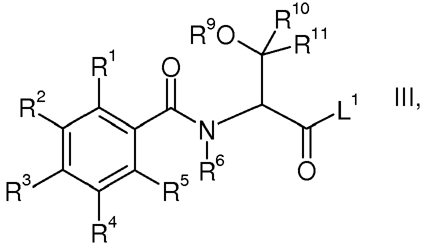 where R¹ is fluorine, chlorine or CF₃ and R⁵ is hydrogen, and R² to R⁴, R⁶ and R⁹ to R¹¹ are as defined in claim 1, and L¹ is hydroxyl or C₁-C₆-alkoxy

9. A composition, comprising a herbicidally effective amount of at least one benzoyl-substituted serineamide of the formula I or an agriculturally useful salt of I according to any of claims 1 to 5 and auxiliaries customary for formulating crop protection agents.

10. A process for preparing compositions according to claim 9, wherein a herbicidally effective amount of at least one benzoyl-substituted serineamide of the formula I or an agriculturally useful salt of I according to any of claims 1 to 5 and auxiliaries customary for formulating crop protection agents are mixed.

11. A method for controlling unwanted vegetation, wherein a herbicidally effective amount of at least one benzoyl-substituted serineamide of the formula I or an agriculturally useful salt of I according to any of claims 1 to 5 is allowed to act on plants, their habitat and/or on seed.

12. The use of a benzoyl-substituted serineamide of the formula I or an agriculturally useful salt thereof according to any of claims 1 to 5 as a herbicide.

## Revendications

1. Sérine-amides substitués par benzoyle, de formule I 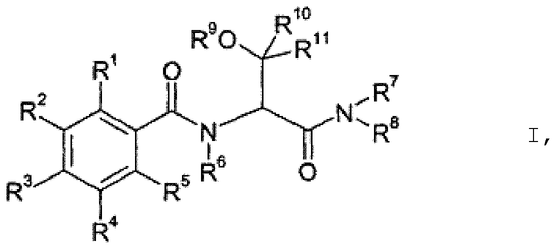 dans laquelle les variables ont les significations suivantes :
R¹ représente un atome d'halogène, un groupe cyano, alkyle en C₁-C₆, halogénoalkyle (C₁-C₆) ou halogénoalcoxy (C₁-C₆) ;
R² R³ R⁴ R⁵ représentent un atome d'hydrogène ou d'halogène, un groupe cyano, alkyle en C₁-C₆, halogénoalkyle (C₁-C₆) , alcoxy en C₁-C₆ ou halogénoalcoxy (C₁-C₆) ;
R⁶ R⁷ représentent un atome d'hydrogène, un groupe hydroxy ou alcoxy en C₁-C₆ ;
R⁸ représente un groupe alkyle en C₁-C₆, cyanoalkyle (C₁-C₄) ou halogénoalkyle (C₁-C₆) ;
R⁹ représente un atome d'hydrogène, un groupe alkyle en C₁-C₆, cycloalkyle en C₃-C₆, alcényle en C₃-C₆, alcynyle en C₃-C₆, halogénoalcényle (C₃-C₆), halogénoalcynyle (C₃-C₆), formyle, alkyl(C₁-C₆)-carbonyle, cycloalkyl(C₃-C₆)carbonyle, alcényl(C₂-C₆)carbonyle, alcynyl(C₂-C₆)carbonyle, alcoxy(C₁-C₆)carbonyle, alcényloxy(C₃-C₆)carbonyle, alcynyloxy(C₃-C₆)carbonyle, alkyl(C₁-C₆)aminocarbonyle, alcényl(C₃-C₆)-aminocarbonyle, alcynyl(C₃-C₆)-aminocarbonyle, alkyl(C₁-C₆)-sulfonylaminocarbonyle, di[alkyl(C₁-C₆)]-aminocarbonyle, N-[alcényl(C₃-C₆)]-N-[alkyl(C₁-C₆)]-aminocarbonyle, N-[alcynyl(C₃-C₆)]-N-[alkyl(C₁-C₆)]-aminocarbonyle, N-[alcoxy(C₁-C₆)]-N-[alkyl(C₁-C₆)]-aminocarbonyle, N-[alcényl(C₃-C₆)]-N-[alcoxy(C₁-C₆)]-aminocarbonyle, N-[alcynyl(C₃-C₆)]-N-[alcoxy(C₁-C₆)]-aminocarbonyle, di[alkyl(C₁-C₆)]-aminothiocarbonyle, [alkyl(C₁-C₆)]cyano-imino, (amino)cyano-imino, [(alkyl[C₁-C₆])amino]cyanoimino, [di(alkyl[C₁-C₆])amino]cyano-imino, alkyl(C₁-C₆)carbonyl-alkyle(C₁-C₆), alcoxy(C₁-C₆)-imino-alkyle(C₁-C₆), N-[alkyl(C₁-C₆)amino]-imino-alkyle(C₁-C₆), N-[dialkyl(C₁-C₆)amino]-imino-alkyle(C₁-C₆) ou trialkyl(C₁-C₄)silyle,
les radicaux alkyle, cycloalkyle et alcoxy nommés pouvant être partiellement ou totalement halogénés et/ou pouvant porter un à trois des groupes suivants : cyano, hydroxy, cycloalkyle en C₃-C₆, alcoxy(C₁-C₆₎-alkyle(C₁-C₄), alcoxy(C₁-C₄)-alcoxy(C₁-C₄)-alkyle(C₁-C₄), alcoxy en C₁-C₄, alkyl(C₁-C₄)thio, di[alkyl(C₁-C₄)]amino, alkyl(C₁-C₄)-alcoxy(C₁-C₆)carbonylamino, alkyl(C₁-C₄)carbonyle, hydroxycarbonyle, alcoxy(C₁-C₄)carbonyle, aminocarbonyle, alkyl(C₁-C₄)aminocarbonyle, di[alkyl(C₁-C₄)]-aminocarbonyle ou alkyl(C₁-C₄)carbonyloxy ; phényle, phényl-alkyle(C₁-C₆), phénylcarbonyle, phénylcarbonyl-alkyle(C₁-C₆), phénoxycarbonyle, phénylaminocarbonyle, phénylsulfonylamino-carbonyle, N-[alkyl(C₁-C₆)]-N-(phényl)-aminocarbonyle, phényl-alkyl(C₁-C₆)carbonyle,
le radical phényle pouvant être partiellement ou totalement halogéné et/ou pouvant porter un à trois des groupes suivants : nitro, cyano, alkyle en C₁-C₄, halogénoalkyle(C₁-C₄), alcoxy en C₁-C₄ ou halogénoalcoxy(C₁-C₄) ; ou SO₂R¹¹ ;
R¹⁰ représente un atome d'hydrogène ou un groupe alkyle en C₁-C₆ ;
R¹¹ représente un groupe alkyle en C₁-C₆, alcényle en C₂-C₆, alcynyle en C₂-C₆, halogénoalkyle (C₁-C₆), halogénoalcényle(C₂-C₆), halogénoalcynyle(C₂-C₆), cyanoalkyle(C₁-C₆), cyanoalcényle(C₂-C₆), cyano-alcynyle(C₂-C₆), hydroxyalkyle(C₁-C₆), hydroxy-alcényle(C₂-C₆), hydroxyalcynyle(C₂-C₆), cycloalkyle en C₃-C₆, cycloalcényle en C₃-C₆, hétérocyclyle à 3-6 chaînons, (hétérocyclyl à 3-6 chaînons)-alkyle(C₁-C₄),
les radicaux cycloalkyle, cycloalcényle ou hétérocyclyle à 3-6 chaînons nommés précédemment pouvant être partiellement ou totalement halogénés et/ou pouvant porter un à trois radicaux choisis dans l'ensemble constitué par les radicaux oxo, cyano, nitro, alkyle en C₁-C₆, halogénoalkyle(C₁-C₆), hydroxy, alcoxy en C₁-C₆, halogénoalcoxy(C₁-C₆), hydroxycarbonyle, alcoxy(C₁-C₆)carbonyle, hydroxycarbonyl-alcoxy(C₁-C₆), alcoxy(C₁-C₆)carbonyl-alcoxy(C₁-C₆), amino, alkyl(C₁-C₆) amino, di[alkyl(C₁-C₆)]amino, alkyl(C₁-C₆)sulfonylamino, halogénoalkyl(C₁-C₆)sulfonylamino, aminocarbonylamino, [alkyl(C₁-C₆)amino]-carbonylamino, di-[alkyl(C₁-C₆)]-aminocarbonylamino, aryle et aryl[alkyle(C₁-C₆)] ;
alcoxy(C₁-C₆)-alkyle(C₁-C₄), alcényloxy(C₂-C₆)-alkyle(C₁-C₄), alcynyloxy(C₂-C₆)-alkyle(C₁-C₄), halogénoalcoxy(C₁-C₆)-alkyle(C₁-C₄), halogénoalcényloxy(C₂-C₆)-alkyle(C₁-C₄), halogéno-alcynyloxy(C₂-C₆)-alkyle(C₁-C₄), alcoxy(C₁-C₆)-alcoxy(C₁-C₄)-alkyle(C₁-C₄), alkyl(C₁-C₆)thioalkyle(C₁-C₄), alcényl(C₂-C₆)thio-alkyle(C₁-C₄), alcynyl(C₂-C₆)thio-alkyle(C₁-C₄), halogénoalkyl(C₁-C₆)-thioalkyle(C₁-C₄), halogénoalcényl(C₂-C₆)-thioalkyle(C₁-C₄), halogénoalcynyl(C₂-C₆)-thioalkyle(C₁-C₄), alkyl(C₁-C₆)-sulfinyl-alkyle(C₁-C₄), halogénoalkyl(C₁-C₆)-sulfinyl-alkyle(C₁-C₄), alkyl(C₁-C₆)-sulfonylalkyle(C₁-C₄), halogénoalkyl(C₁-C₆)-sulfonylalkyle(C₁-C₄), amino-alkyle(C₁-C₄), alkyl(C₁-C₆)-amino-alkyle(C₁-C₄), di[alkyl(C₁-C₆)]aminoalkyle(C₁-C₄), alkyl(C₁-C₆)sulfonylaminoalkyle(C₁-C₄), alkyl(C₁-C₆)sulfonyl-[alkyl(C₁-C₆)-amino]-alkyle(C₁-C₄), alkyl(C₁-C₆) carbonyle, hydroxycarbonyle, alcoxy(C₁-C₆)carbonyle, aminocarbonyle, alkyl(C₁-C₆)aminocarbonyle, di[alkyl(C₁-C₆)]aminocarbonyle, alkyl(C₁-C₆)-carbonyl-alkyle(C₁-C₆), hydroxycarbonylalkyle(C₁-C₄), alcoxy(C₁-C₆)carbonyl-alkyle(C₁-C₄), halogénoalcoxy(C₁-C₆)carbonyl-alkyle(C₁-C₄), alkyl(C₁-C₆)carbonyloxy-alkyle(C₁-C₄), aminocarbonyl-alkyle(C₁-C₄), alkyl(C₁-C₆)aminocarbonyl-alkyle(C₁-C₄), di[alkyl(C₁-C₆)] aminocarbonyl-alkyle(C₁-C₄), formylaminoalkyle(C₁-C₄), alcoxy(C₁-C₆)carbonylaminoalkyle(C₁-C₄), alkyl(C₁-C₆)carbonylaminoalkyle(C₁-C₄), alkyl(C₁-C₆)carbonyl-[alkyl(C₁-C₆)amino]-alkyle(C₁-C₄), [(alkyl[C₁-C₆])-aminocarbonyloxy]-alkyle(C₁-C₄), [di(alkyl[C₁-C₆])-aminocarbonyloxy]alkyle(C₁-C₄), {di[di(alkyl[C₁-C₆])amino]carbonyloxy}-alkyle(C₁-C₄), [(alkyl[C₁-C₆]amino)carbonylamino]-alkyle(C₁-C₄), [di(alkyl[C₁-C₆])aminocarbonylamino]alkyle(C₁-C₄) ;
phényl-alkyle(C₁-C₄), phényl-alcényle(C₂-C₄), phényl-alcynyle(C₂-C₄), phényl-halogénoalkyle(C₁-C₄), phényl-halogénoalcényle(C₂-C₄), phényl-halogénoalcynyle(C₂-C₄), phényl-hydroxy-alkyle(C₁-C₄), phényl-hydroxyalcényle(C₂-C₄), phényl-hydroxyalcynyle(C₂-C₄), phénylcarbonyl-alkyle(C₁-C₄), phénylcarbonylamino-alkyle(C₁-C₄), phénylcarbonyloxy-alkyle(C₁-C₄), phényloxycarbonyl-alkyle(C₁-C₄), phényloxy-alkyle(C₁-C₄), phénylthio-alkyle(C₁-C₄), phénylsulfinyl-alkyle(C₁-C₄), phénylsulfonyl-alkyle(C₁-C₄), hétéroaryl-alkyle(C₁-C₄), hétéroaryl-alcényle(C₂-C₄), hétéroaryl-alcynyle(C₂-C₄), hétéroaryl-halogénoalkyle(C₁-C₄), hétéroaryl-halogénoalcényle(C₂-C₄), hétéroaryl-halogénoalcynyle(C₂-C₄), hétéroaryl-hydroxyalkyle(C₁-C₄), hétéroaryl-hydroxy-alcényle(C₂-C₄), hétéroaryl-hydroxyalcynyle(C₂-C₄), hétéroarylcarbonyl-alkyle(C₁-C₄), hétéroaryl-carbonyloxy-alkyle(C₁-C₄), hétéroaryloxycarbonyl-alkyle(C₁-C₄), hétéroaryloxy-alkyle(C₁-C₄), hétéroarylthio-alkyle(C₁-C₄), hétéroarylsulfinyl-alkyle(C₁-C₄), hétéroarylsulfonyl-alkyle(C₁-C₄),
les radicaux phényle et hétéroaryle nommés précédemment pouvant être partiellement ou totalement halogénés et/ou pouvant porter un à trois radicaux choisis dans l'ensemble constitué par les groupes cyano, nitro, alkyle en C₁-C₆, halogénoalkyle(C₁-C₆), hydroxy, alcoxy en C₁-C₆, halogénoalcoxy(C₁-C₆), hydroxycarbonyle, alcoxy(C₁-C₆) carbonyle, hydroxycarbonyl-alcoxy(C₁-C₆), alcoxy(C₁-C₆)carbonyl-alcoxy(C₁-C₆), amino, alkyl(C₁-C₆)amino, di[alkyl(C₁-C₆)]amino, alkyl(C₁-C₆)sulfonylamino, halogénoalkyl (C₁-C₆) sulfonylamino, (alkyl[C₁-C₆]-amino)carbonylamino, di-(alkyl[C₁-C₆])-aminocarbonyl-amino, aryle et aryl(alkyle[C₁-C₆]) ;
R¹² représente un groupe alkyle en C₁-C₆, halogénoalkyle(C₁-C₆) ou phényle,
le radical phényle pouvant être partiellement ou totalement halogéné et/ou pouvant porter un à trois des groupes suivants : alkyle en C₁-C₆, halogéno-alkyle(C₁-C₆) ou alcoxy en C₁-C₆ ;
ainsi que leurs sels utilisables en agriculture.

2. Sérine-amides substitués par benzoyle de formule I selon la revendication 1, dans lesquels R¹ représente un atome d'halogène ou un groupe halogénoalkyle en C₁-C₆.

3. Sérine-amides substitués par benzoyle de formule I selon la revendication 1 ou 2, dans lesquels R² et R³ représentent indépendamment l'un de l'autre un atome d'hydrogène ou d'halogène, ou un groupe halogénoalkyle en C₁-C₆.

4. Sérine-amides substitués par benzoyle de formule I selon les revendications 1 à 3, dans lesquels R⁴, R⁵, R⁶, R et R¹⁰ représentent un atome d'hydrogène.

5. Sérine-amides substitués par benzoyle de formule I selon les revendications 1 à 4, dans lesquels R¹¹ représente un groupe alkyle en C₁-C₆, alcényle en C₂-C₆, alcynyle en C₂-C₆, halogénoalkyle (C₁-C₆), halogénoalcényle(C₂-C₆), halogénoalcynyle(C₂-C₆), cyanoalkyle(C₁-C₆), hydroxyalkyle(C₁-C₆), hydroxy-alcényle(C₂-C₆), hydroxyalcynyle(C₂-C₆), cycloalkyle en C₃-C₆, cycloalcényle en C₃-C₆, hétérocyclyle à 3-6 chaînons,
les radicaux cycloalkyle, cycloalcényle ou hétérocyclyle à 3-6 chaînons nommés précédemment pouvant être partiellement ou totalement halogénés et/ou pouvant porter un à trois radicaux choisis dans l'ensemble constitué par les radicaux oxo, alkyle en C₁-C₆, halogénoalkyle(C₁-C₆), hydroxycarbonyle et alcoxy(C₁-C₆)carbonyle,
alcoxy(C₁-C₆)-alkyle(C₁-C₄), halogénoalcoxy(C₁-C₆)-alkyleC₁-C₄), alcoxy(C₁-C₆)-alcoxy(C₁-C₄)-alkyle(C₁-C₄), alkyl(C₁-C₆)thio-alkyle(C₁-C₄), alkyl(C₁-C₆)sulfonyl-amino-alkyle(C₁-C₄), hydroxycarbonyle, alcoxy(C₁-C₆)-carbonyle, hydroxycarbonyl-alkyle(C₁-C₄), alcoxy(C₁-C₆)-carbonyl-alkyle(C₁-C₄), halogénoalcoxy(C₁-C₆)carbonyl-alkyle(C₁-C₄), alkyl(C₁-C₆)carbonyloxy-alkyle(C₁-C₄), alkyl(C₁-C₆)carbonylamino-alkyle(C₁-C₄), di[alkyl(C₁-C₆)]carbonylamino-alkyle(C₁-C₄), di(alkyl[C₁-C₆])aminocarbonylamino-alkyle(C₁-C₄), [(alkyl[C₁-C₆])aminocarbonyl]amino-alkyle(C₁-C₄), [di(alkyl[C₁-C₆])amino-carbonyloxy]alkyle(C₁-C₄), formylamino-alkyle(C₁-C₄),
phényl-alkyle(C₁-C₄), phényl-alcényle(C₂-C₄), phényl-alcynyle(C₂-C₄), phényl-halogénoalkyle(C₁-C₄), phényl-halogénoalcényle(C₂-C₄), phényl-hydroxyalkyle(C₁-C₄), phényloxy-alkyle(C₁-C₄), phénylthio-alkyle(C₁-C₄), phénylsulfinyl-alkyle(C₁-C₄), phénylsulfonyl-alkyle(C₁-C₄), hétéroaryl-alkyle(C₁-C₄), hétéroaryl-hydroxy-alkyle(C₁-C₄), hétéroaryloxy-alkyle(C₁-C₄), hétéroarylthio-alkyle(C₁-C₄), hétéroarylsulfinyl-alkyle(C₁-C₄), hétéroarylsulfonyl-alkyle(C₁-C₄),
les radicaux phényle et hétéroaryle nommés précédemment pouvant être partiellement ou totalement halogénés et/ou pouvant porter un à trois radicaux choisis dans l'ensemble constitué par les groupes cyano, nitro, alkyle en C₁-C₆, halogénoalkyle(C₁-C₆), hydroxy, alcoxy en C₁-C₆, halogénoalcoxy(C₁-C₆), hydroxycarbonyle, alcoxy(C₁-C₆)carbonyle, hydroxycarbonyl-alcoxy(C₁-C₆), alkyl(C₁-C₆)sulfonylamino et halogénoalkyl(C₁-C₆)sulfonylamino.

6. Procédé pour la préparation de sérine-amides substitués par benzoyle, de formule I selon la revendication 1, **caractérisé en ce qu'**on fait réagir des dérivés de sérine de formule V 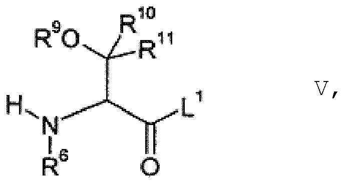 dans laquelle R⁶, R⁹, R¹⁰ et R¹¹ ont les significations données dans la revendication 1 et L¹ représente un groupe hydroxy ou alcoxy en C₁-C₆,
avec un/des dérivé(s) d'acide benzoïque de formule IV 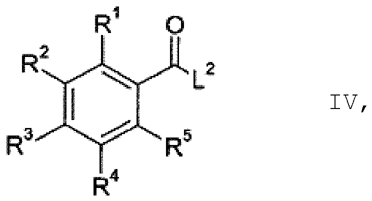 dans laquelle R¹ à R⁵ ont les significations données dans la revendication 1 et L² représente un atome d'halogène ou un groupe hydroxy, alkyl(C₁-C₆)carbonyle, alcoxy(C₁-C₆)carbonyle, alkyl(C₁-C₄)sulfonyle, phosphoryle ou iso-uréyle,
pour aboutir aux dérivés benzoyle correspondants de formule III 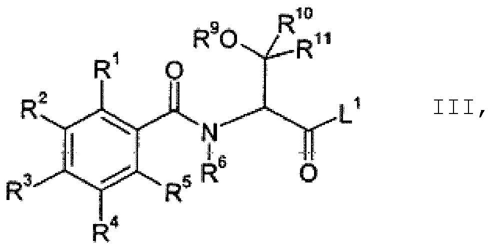 dans laquelle R¹ à R⁶ et R⁹ à R¹¹ ont les significations données dans la revendication 1 et L¹ représente un groupe hydroxy ou alcoxy en C₁-C₆,
et on fait ensuite réagir les dérivés benzoyle de formule III obtenus avec une amine de formule II
HNR⁷R⁸ II,
où R⁷ et R⁸ ont les significations données dans la revendication 1.

7. Procédé pour la préparation de sérine-amides substitués par benzoyle, de formule I selon la revendication 6, dans lesquels R⁹ et R¹⁰ représentent un atome d'hydrogène, **caractérisé en ce qu'**on prépare des dérivés benzoyle de formule III dans lesquels R⁹ et R¹⁰ représentent un atome d'hydrogène, par acylation de composés céto de formule XIII 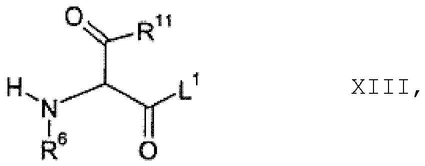 dans laquelle R⁶ et R¹¹ ont les significations données dans la revendication 1 et L¹ représente un groupe hydroxy ou alcoxy en C₁-C₆,
avec un/des dérivé(s) d'acide benzoïque de formule IV, pour aboutir à des composés N-acyl-céto de formule XII 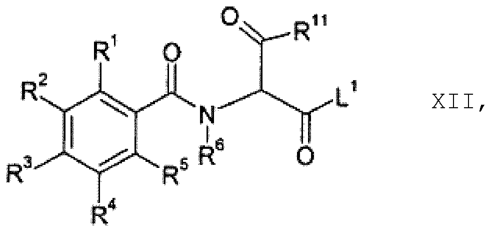 dans laquelle R¹ à R⁶ ainsi que R¹¹ ont les significations données dans la revendication 1 et L¹ représente un groupe hydroxy ou alcoxy en C₁-C₆,
et réduction subséquente du groupe céto.

8. Dérivés benzoyle de formule III 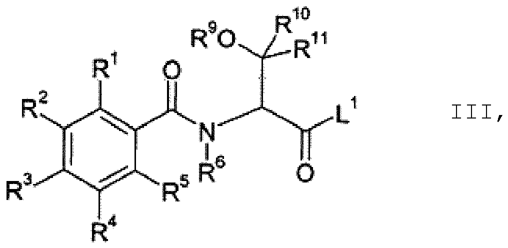 dans laquelle R¹ représente un atome de fluor, de chlore ou CF₃, et R⁵ représente un atome d'hydrogène, et R² à R⁴, R⁶ et R⁹ à R¹¹ ont les significations données dans la revendication 1, et L¹ représente un groupe hydroxy ou alcoxy en C₁-C₆.

9. Compositions contenant une quantité à effet herbicide d'au moins un sérine-amide substitué par benzoyle, de formule I, ou d'un sel de I utilisable en agriculture, selon les revendications 1 à 5, et des adjuvants usuels pour la formulation de produits phytosanitaires.

10. Procédé pour la préparation de compositions selon la revendication 9, **caractérisé en ce qu'**on mélange une quantité à effet herbicide d'au moins un sérine-amide substitué par benzoyle, de formule I, ou d'un sel de I utilisable en agriculture, selon les revendications 1 à 5 et des adjuvants usuels pour la formulation de produits phytosanitaires.

11. Procédé pour la lutte contre une croissance indésirable de plantes, **caractérisé en ce qu'**on fait agir sur des plantes, leur habitat et/ou sur des semences une quantité à effet herbicide d'au moins un sérine-amide substitué par benzoyle, de formule I, ou d'un sel de I utilisable en agriculture, selon les revendications 1 à 5.

12. Utilisation des sérine-amides substitués par benzoyle, de formule I, et de leurs sels utilisables en agriculture, selon les revendications 1 à 5, en tant qu'herbicides.
